# EUROPEAN PATENT APPLICATION

(11) **EP 3 095 787 A1**
(43) Date of publication of application: **23.11.2016**
(21) Application number: 15169052.6
(22) Date of filing: 22.05.2015
(51) Int. Cl.: C07D 498/04, C07D 263/56, A61K 31/423, A61K 31/424, A61P 25/30, A61P 3/04, A61P 25/28

(54) **PHARMACEUTICALLY ACTIVE COMPOUNDS AS DAG-LIPASE INHIBITORS**

(71) Applicant: Universiteit Leiden, 2311 RA Leiden (NL)
(72) Inventor: VAN DER STELT, Mario, 2333 CC Leiden (NL); JANSSEN, Freek, 2333 CC Leiden (NL); BAGGELAAR, Marc, 2333 CC Leiden (NL); HUMMEL, Jessica, 2333 CC Leiden (NL)
(74) Representative: Arth, Hans-Lothar

(57) **Abstract**

The present invention relates to novel compounds which are highly selective inhibitors of diacylglycerol lipase α and β. These compounds are suitable for the treatment or prevention of disorders associated with, accompanied by or caused by increased 2-arachidonoylglycerol levels. Diacylglycerol lipase-α (alternative name: Sn1-specific diacylglycerol hydrolase a; DAGL-α) and -β are enzymes responsible for the biosynthesis of the endocannabinoid 2-arachidonoylglycerol. Selective and reversible inhibitors are required to study the function of DAGLs in neuronal cells in an acute and temporal fashion. The inventive ompounds are in particular suitable for the treatment of neurodegenerative diseases, inflammatory diseases, drug abuse and impaired energy balance, such as obesity.

## Description

The present invention relates to novel compounds which are highly selective inhibitors of diacylglycerol lipase-α and -β. These compounds are suitable for the treatment or prevention of disorders associated with, accompanied by or caused by increased 2-arachidonoylglycerol levels. Diacylglycerol lipase-α (alternative name: Sn1-specific diacylglycerol hydrolase α; DAGL-α or DAGLA) and -β (alternative name: Sn1-specific diacylglycerol hydrolase β; DAGL-β or DAGLB) are enzymes responsible for the biosynthesis of the endocannabinoid 2-arachidonoylglycerol (2-AG). Selective and reversible inhibitors are required to study the function of DAGLs in neuronal cells in an acute and temporal fashion. The inventive ompounds are in particular suitable for the treatment of neurodegenerative diseases, inflammatory diseases, drug abuse and impaired energy balance, such as obesity.

### Background of the invention

Endocannabinoids are endogenous signaling lipids that activate the cannabinoid CB₁ receptor. They play an essential role in human health and disease, regulating processes, such as immunomodulation, energy balance and neurotransmission. There are two main endocannabinoids: anandamide and 2-arachidonoylglycerol. Both endocannabinoids are often found together, but their levels vary between species, tissue, developmental stage and pathological condition. Selective inhibition of the formation of anandamide and 2-AG would be instrumental to target endocannabinoid specific CB₁-mediated physiological effects. However, pathway-selective inhibitors for 2-AG and anandamide biosynthesis are currently lacking.

2-AG is mainly formed by the action of two diacylglycerol lipases (DAGL-α and DAGL-β). DAGLs are intracellular, multi-domain integral membrane proteins. The DAGLs share extensive homology, but differ in size: ∼120 and ∼70 kD for DAGL-α and DAGL-β respectively. DAGLs belong to the class of serine hydrolases that employ the typical Ser-His-Asp catalytic triad to hydrolyze the ester bond of acyl chains from arachidonate-containing diacylglycerols in a *sn-1* specific manner. Studies with DAGL knock-out mice have shown that DAGL-α controls to a large extent the formation of 2-AG in the central nervous system, whereas DAGL-β appears to partake in 2-AG production in the periphery during inflammation. Importantly, also basal anandamide levels were reduced in DAGL-α knock-out mice. Selective inhibitors for DAGLs, which can be used in an acute and temporal fashion and do not modulate anandamide levels, would, therefore, constitute an important counterpart of the DAGL knock-out mice, and allow the examination of acute versus congenital inhibition.

Although, several classes of DAGL inhibitors have been described in the literature, these inhibitors are based on the natural substrates and/or have reactive chemical warheads, and are not selective over other serine hydrolases that modulate endocannabinoid signaling (e.g. ABHD6, ABHD12, monoacylglycerol lipase (MAGL) or fatty acid amide hydrolase (FAAH)). Previously, the α-ketoheterocycle 1-(oxazolo[4,5-b]pyridin-2-yl)-6-phenylhexan-1-one was identified as the first reversible inhibitor for DAGL-α (Baggelaar et al.; Angew. Chem. Int. 2013, 52, 12081 - 12085). 1-(oxazolo[4,5-b]pyridin-2-yl)-6-phenylhexan-1-one was, however, weakly active in a cellular assay and was not selective over FAAH, the enzyme responsible for the metabolism of the other endocannabinoid anandamide. Therefore highly selective DAGL inhibitors are still warranted. In order to apply of inhibitors as chemical tools to study 2-AG signaling, it is important to increase their cellular activity and to have selectivity over FAAH and to assess their activity on endogenous DAGL-α.

It is the object of the present invention to provide compounds and/or pharmaceutically acceptable salts thereof which are highly selective DAGL inhibitors and are suitable as pharmaceutically active agents, especially for treatment of neurodegenerative diseases, inflammation and impaired energy balance, as well as compositions comprising at least one of those compounds and/or pharmaceutically acceptable salts thereof as pharmaceutically active ingredients.

### Description of the invention

The inventors were able to identify highly selective DAGL inhibitors. In comparative and competitive activity-based proteome profiling (ABPP), in which broad-spectrum and tailor-made activity-based probes are combined to report on the inhibition of a protein family in its native environment, the selectivity of the inhibitors has been shown. Competitive ABPP with broad-spectrum fluorophosphonate-based probes and specific β-lactone-based probes led to the discovery of the compounds according to the general formula (I) as potent, highly selective dual DAGL-α/DAGL-β inhibitor. These compounds did not affect other enzymes involved in endocannabinoid metabolism including α/β-hydrolase domain containing ABHD6 and ABHD12, monoacylglycerol lipase and fatty acid amide hydrolase and neither interfered with cannabinoid CB₁ receptor binding. Targeted lipidomics revealed that compounds according to the general formula (I) dose-dependently reduce 2-AG levels, but not anandamide levels, in cells.

It has now surprisingly been discovered that α-ketoheterocycle of the present invention exhibit particularly high levels of inhibition of the activity of diacylglycerol lipase-α and - β. Therefore the present invention refers to compounds defined by the general formula (I): 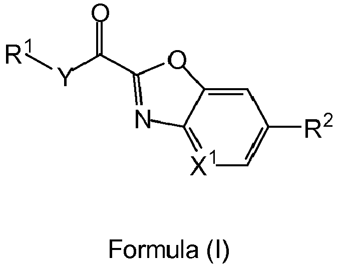 wherein
X¹ is -CH-, -CF- or -N-;
Y represents one of the following moieties:
   - CH₂-, -C₂H₄-, -C₃H₆-, -C₄H₈-, -C₅H₁₀-, -C₆H₁₂-, -C₇H₁₄-, -C₈H₁₆-, -C₉H₁₈-, -C₁₀H₂₀- -CH(CH₃)-, -CH(CH₃)CH₂-, -CH₂CH(CH₃)-, -CH(CH₃)-C₂H₄-, -CH₂-CH(CH₃)CH₂-, -CH₂-CH₂CH(CH₃)-, -CH(CH₃)-C₃H₆-, -CH₂-CH(CH₃)-C₂H₄-, -CH₂-CH₂-CH(CH₃)CH₂-, -CH₂-CH₂-CH₂CH(CH₃)-, -CH(CH₃)-C₄H₈-, -CH₂-CH(CH₃)-C₃H₆-, -CH₂-CH₂-CH(CH₃)-C₂H₄-, -CH₂-CH₂-CH₂-CH(CH₃)-CH₃-, -CH₂-CH₂-CH₂-CH₂-CH(CH₃)-, -CH₂O-, -C₂H₄O-, -C₃H₆O-, -C₄H₈O-, -C₅H₁₀O--C₆H₁₂O- -C₇H₁₄O- -C₈H₁₆O-, -C₉H₁₈O-, -C₁₀H₂₀O-, -CH₂NH-, -C₂H₄NH-, -C₃H₆NH-, -C₄H₈NH- -C₅H₁₀NH- -C₆H₁₂NH-, -C₇H₁₄NH- -C₈H₁₆NH-, -C₉H₁₈NH-, -C₁₀H₂₀NH-, -CH₂OCH₂-, -C₂H₄OCH₂-, -C₃H₆OCH₂-, -C₄H₈OCH₂-, -C₅H₁₀OCH₂-, -C₆H₁₂OCH₂-, -C₇H₁₄OCH₂-, -C₈H₁₆OCH₂-, -C₉H₁₈OCH₂-, -C₁₀H₂₀OCH₂-, -CH₂NHCH₂-, -C₂H₄NHCH₂-, -C₃H₆NHCH₂-, -C₄H₈NHCH₂-, -C₅H₁₀NHCH₂-, -C₆H₁₂NHCH₂-, -C₇H₁₄NHCH₂-, -C₈H₁₆NHCH₂-, -C₉H₁₈NHCH₂-, -C₁₀H₂₀NHCH₂-, -CH=CH-, -CH=CH-CH₂-, -CH₂-CH=CH-, -CH=CH-C₂H₄-, -CH₂-CH=CH-CH₂-, -C₂H₄-CH=CH-, -CH=CH-C₃H₆-, CH₂-CH=CH-C₂H₄-, -C₂H₄-CH=CH-CH₂-, -C₃H₆-CH=CH-, -CH=CH-C₄H₈-, -CH₂-CH=CH-C₃H₆-, -C₂H₄-CH=CH-C₂H₄-, -C₃H₆-CH=CH-CH₂-, -C₄H₈-CH=CH-, -CH=CH-CH=CH-, -CH=CH-CH=CH-CH₂-, -CH₂-CH=CH-CH=CH-, -CH=CH-CH₂-CH=CH-, -C≡C-, -C≡C-CH₂-, -CH₂-C≡C-, -C≡C-C₂H₄-, -CH₂-C≡C-CH₂-, -C₂H₄-C≡C-, -C₂H₄-C≡C-, -C≡C-C₃H₆-, -CH₂-C≡C-C₂H₄-, -C₂H₄-C≡C-CH₂-, -C₃H₆-C≡C-, -C≡C-C₄H₈-, -CH₂-C≡C-C₃H₆-, -C₂H₄-C≡C-C₂H₄-, -C₃H₆-C≡C-CH₂-, -C≡C-C≡C-, -C≡C-C≡C-CH₂-, -C≡C-CH₂-C≡C-, -CH₂-C≡C-C≡C-, -C₂H₄-C≡C-C≡C-, -CH₂-C≡C-CH₂-C≡C-, -C≡C-C₂H₄-C≡C-, -CH₂-C≡C-C≡C-CH₂-, -C≡C-CH₂-C≡C-CH₂-, or -C≡C-C≡C-C₂H₄-;
R¹ represents: -CF₃, -CHF₂, -CH₂F, -CF₂CF₃, -CHFCF₃, -CF₂CHF₂, -CHFCHF₂, -CF₂CH₂F, -CHFCH₂F, -CHFCH₃, -CF₂CH₃, 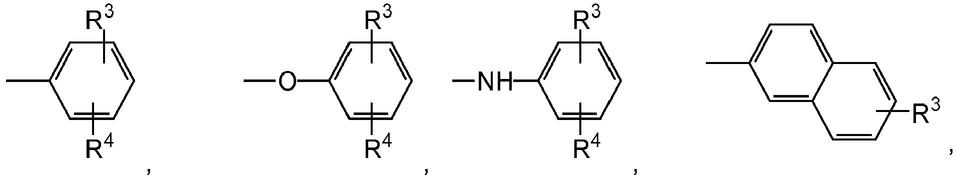 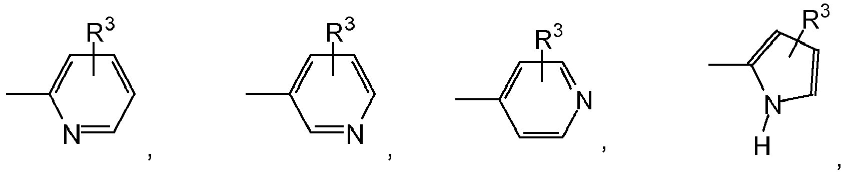 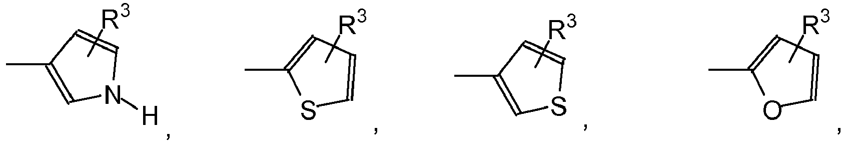 or 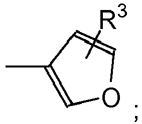
R² represents one of the following moieties: -CH(CH₃)₂, -CH(C₂H₅)₂, -CH₂CH(CH₃)₂, -C(CH₃)₃, -CH₂C(CH₃)₃, -CHPh₂, -CPh₃, -CH₂CPh₃, 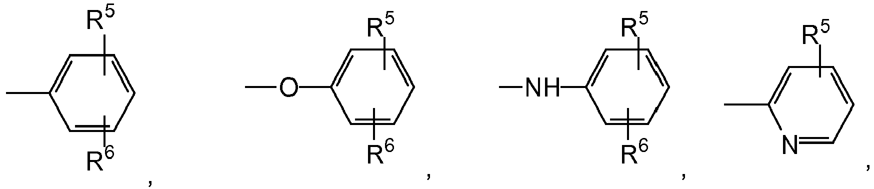 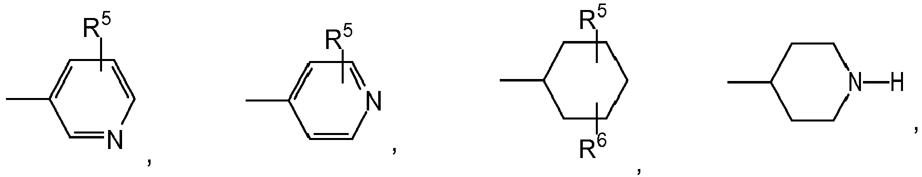 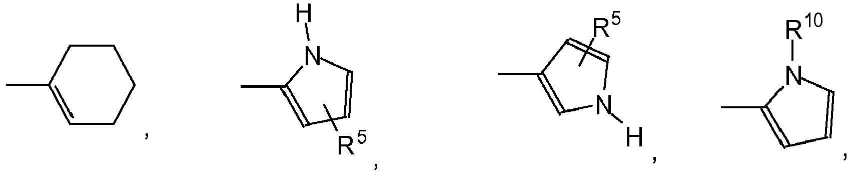 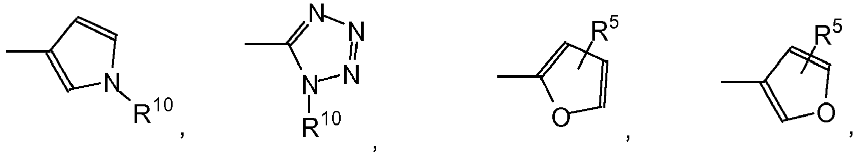 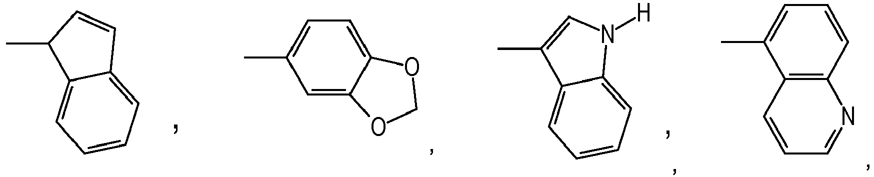 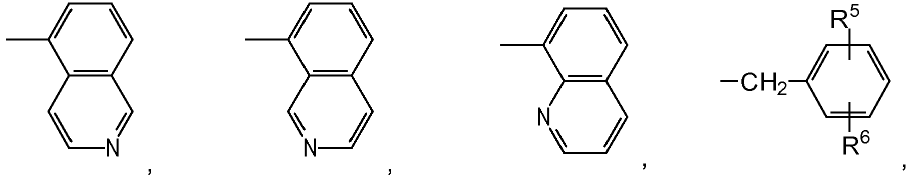 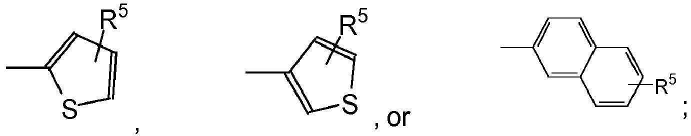
R³ and R⁴ are independently of each other selected from: -R⁸, -R⁹, -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅,-CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SO₂CH₃, -SO₂C₂H₅, -SO₂CH(CH₃)₂, -SO₂C(CH₃)₃, -SO₃H, -SO₃CH₃, -SO₃C₂H₅, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -NH-CO-NH₂, -NH-CO-NHCH₃, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, 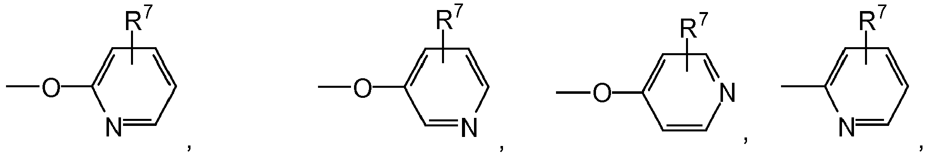 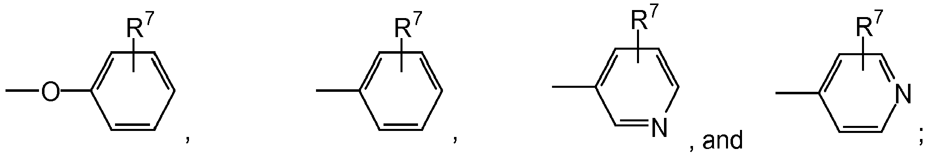
R⁵ and R⁶ are independently of each other selected from:
   -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -N₃, -CN, -COC₂H₅, -COC₃H₇, -COOH, -COOCH₃, -COOC₂H₅, -COOC₃H₇, -CONH₂, -CONHCH₃, -NH₂, -NHCH₃, -NHC₂H₅, -NHC₃H₇, -N(C₂H₅)₂, -SO₂CH₃, -SO₂C₂H₅, -SO₂C₃H₇, -SO₃H, -SO₃CH₃, -CH₂Cl, -CH₂Br, -CH₂I, -CH₂F, -CHF₂, -CF₃, -CF₂CF₃, -CH₂CH₂F, -CH₂CHF₂, -CH₂CF₃, -CF₂CF₂CF₃, -CH₂CH₂CF₃, -CF=CF₂, -CH=CF₂, -CF=CFCF₃, -CH=CHCF₃, -CF₂CF=CF₂, -CH₂CH=CF₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -CH=CH₂, -CH₂-C(CH₃)₂-CH=CH₂, -C(CH₃)₂-CH₂-CH=CH₂, -CH=C(CH₃)-CH(CH₃)₂, -Ph, -CHPh₂, -CPh₃, -CH=CH-Ph, -CH₂-Ph, -C₂H₄-Ph, -OCH₂-Ph, and -OC₂H₄-Ph;
R⁷, R⁸ and R⁹ are independently of each other selected from:
   -H, -F, -Cl, -Br, -I, -CH₂F, -CHF₂, -CF₃, -CF₂CF₃, -CH₂CH₂F, -CH₂CHF₂, -CH₂CF₃, -CF₂CF₂CF₃, -CH₂CH₂CF₃, -CF=CF₂, -CH=CF₂, -CF=CFCF₃, -CH=CHCF₃, -CF₂CF=CF₂, -CH₂CH=CF₂, -OH, -OCH₃, -OC₂H₅, -OC₃H₇, -OC₃H₇, -OC₄H₉, -NH₂, -NHCH₃, -NHC₂H₅, -NHC₃H₇, -NHC₄H₉, -CH₂F, -CHF₂, -CF₃, -CH₂Cl, -CH₂Br, -CH₂I₃ -CH₂-CH₂Cl, -CH₂-CH₂Br, -CH₂-CH₂I, -CH₃, -C₂H₅, -C₃H₇, -C₄H₉, -C₅H₁₁, -C₆H₁₃, -C₇H₁₅, -C₈H₁₇, -C₉H₁₉, -C₁₀H₂₁, -CH(CH₃)₂, -CH(CH₃)-C₂H₅, -CH₂-CH(CH₃)CH₃, -CH(CH₃)-C₃H₇, -CH₂-CH(CH₃)-C₂H₅, -CH₂-CH₂-CH(CH₃)₂, -CH(CH₃)-C₄H₉, -CH₂-CH(CH₃)-C₃H₇, -CH₂-CH₂-CH(CH₃)-C₂H₅, -CH₂-CH₂-CH₂-CH(CH₃)-CH₃, -CH₂-CH₂-CH₂-CH₂-CH(CH₃)₂, -CH=CH₂, -CH=CH-CH₃, -CH₂-CH=CH₂, -CH=CH-C₂H₅, -CH₂-CH=CH-CH₃, -C₂H₄-CH=CH₂, -CH=CH-C₃H₇, -CH₂-CH=CH-C₂H₅, -C₂H₄-CH=CH-CH₃, -C₂H₄-CH₂-CH=CH₂, -CH=CH-C₄H₉, -CH₂-CH=CH-C₃H₇, -C₂H₄-CH=CH-C₂H₅, -C₃H₆-CH=CH-CH₃, -C₄H₈-CH=CH₂, -CH=CH-CH=CH₂, -CH=CH-CH=CH-CH₃, -CH₂-CH=CH-CH=CH₂, -CH=CH-CH₂-CH=CH₂, -C≡C-CH₃, -CH₂-C≡CH, -C≡C-C₂H₅, -CH₂-C≡C-CH₃, -C₂H₄-C≡CH, -C₂H₄-C≡CH, -C≡C-C₃H₇, -CH₂-C≡C-C₂H₅, -C₂H₄-C≡C-CH₃, -C₃H₆-C≡CH, -C≡C-C₄H₉, -CH₂-C≡C-C₃H₇, -C₂H₄-C≡C-C₂H₅, -C₃H₆-C≡C-CH₃, -C≡C-C≡CH, -C≡C-C≡C-CH₃, -C≡C-CH₂-C≡CH, -CH₂-C≡C-C≡CH, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -Ph, -CH₂-Ph, -CH₂-CH₂-Ph, -CH=CH-Ph, -CPh₃, -OCH₂-Ph, and -OC₂H₄-Ph;
R¹⁰ represents one of the following groups: -H, -CH₃, -CH₂CH₃, -CH(CH₃)₂, -CH₂CH₂CH₃, or -CH₂CH₂CH₂CH₃;
and enantiomers, stereoisomeric forms, mixtures of enantiomers, diastereomers, mixtures of diastereomers, prodrugs, hydrates, solvates, acid salt forms, tautomers, and racemates of the above mentioned compounds and pharmaceutically acceptable salts thereof.

The expression prodrug is defined as a substance, which is applied in an inactive or significantly less active form. Once applied and incorporated, the prodrug is metabolized in the body in vivo into the active compound. Conventional procedures for the selection and preparation of suitable prodrug derivatives are described, for example in "Design of Prodrugs", ed. H. B. Bundgaard, Elsevier, 1985.

The expression tautomer is defined as an organic compound that is interconvertible by a chemical reaction called tautomerization. Tautomerization can be catalyzed preferably by bases or acids or other suitable compounds.

The compounds of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, D-o-tolyltartaric acid, tartronic acid, (o, m, p)-toluic acid, naphthylamine sulfonic acid, trifluoroacetic acid, and other mineral or carboxylic acids well known to those skilled in the art. The salts are prepared by contacting the free base form of the compounds of formula (I) with a sufficient amount of the desired acid to produce a salt in the conventional manner well known to those skilled in the art. The inventive compounds may exist in a number of different polymorphic forms.

In the case the inventive compounds bear acidic groups, salts could also be formed with inorganic or organic bases. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

Preferred are compounds of general formula (I), wherein X¹ represents -N- or -CF-. Thus, preferred are compounds of general formula (Ia), or (Ib) 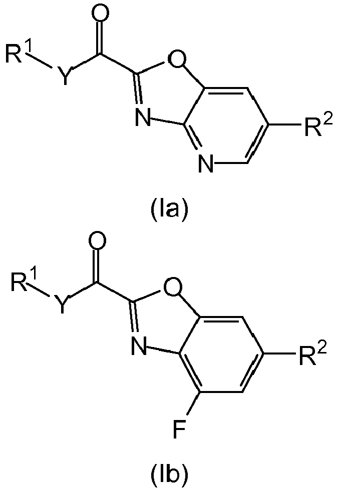 wherein the substituents R¹, and R², and the linker Y have the meanings as disclosed herein.

Also preferred are compounds of general formula (I), wherein the linker Y represents -(CH₂)ₙ-, and wherein n is an integer number from: 0, 1, 2, 3, 4, 5, 6, 7, 8, 9, and 10, 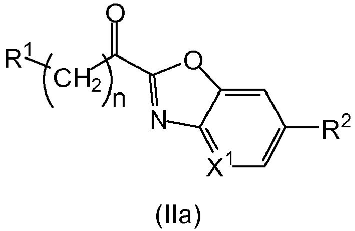 wherein the substituents R¹, and R², and X¹, have the meanings as disclosed herein.

Particularly preferred are compounds of general formula (I), wherein X¹ represents -N-or -CF-. Thus, preferred are compounds of one of the general formula (IIb), or (IIc) 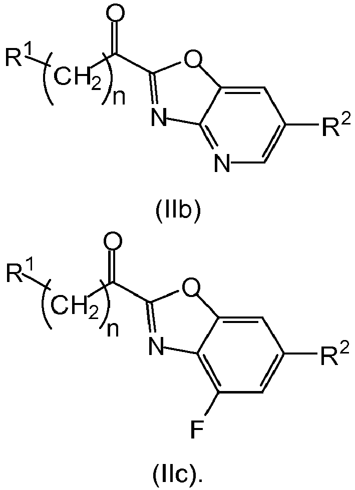 wherein the substituents R¹, and R², and the integer number n have the meanings as disclosed herein.

Also preferred are compounds of the general formula (IIIa) 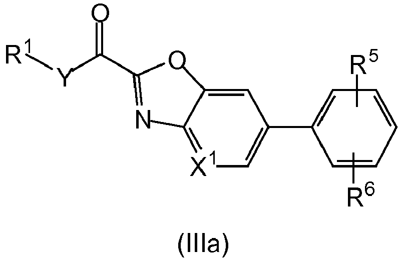 wherein the substituents R¹, R⁵, R⁶, X¹, and the linker Y have the meanings as defined herein. More preferred are compounds of one of the general formula (IIIb), or (IIIc) 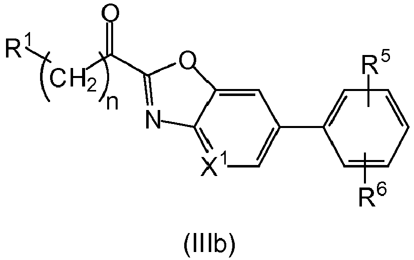 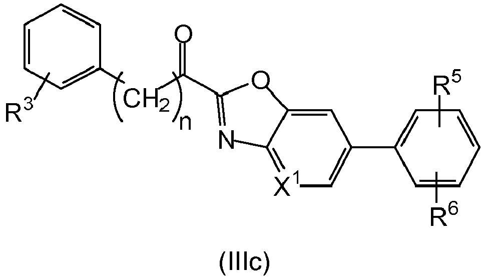 wherein the substituents R¹, R³, R⁵, R⁶, X¹, and the integer number n have the meanings as defined herein.

Another invention regards compounds of the general formula (IVa) 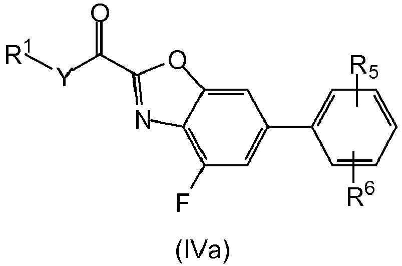 wherein the substituents R¹, R⁵, R⁶, and the linker Y have the meanings as defined herein. More preferred are compounds of one of the general formula (IVb), or (IVc) 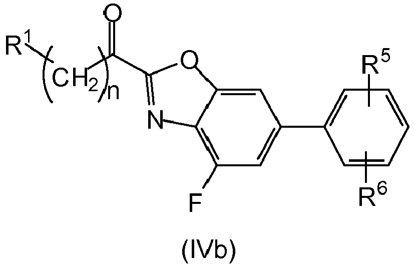 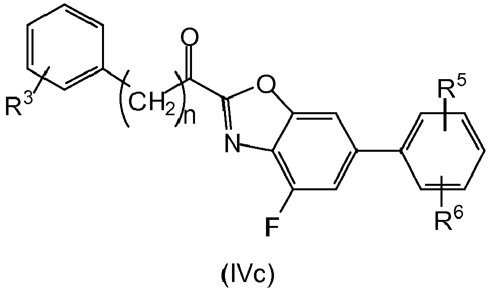 wherein the substituents R¹, R³, R⁵, R⁶, and the integer number n have the meanings as defined herein.

Another embodiment of the invention relates to compounds of the general formula (Va) 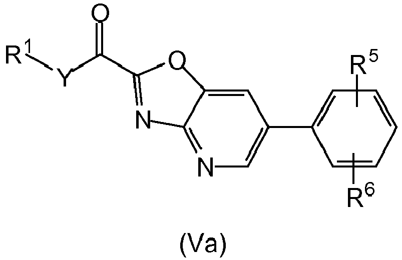 wherein the substituents R¹, R⁵, R⁶, and the linker Y have the meanings as defined herein. More preferred are compounds of one of the general formula (Vb), or (Vc) 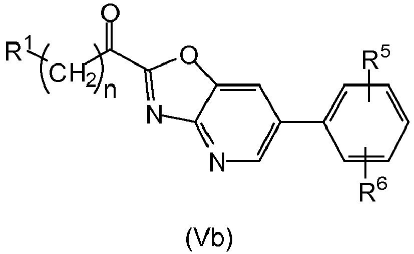 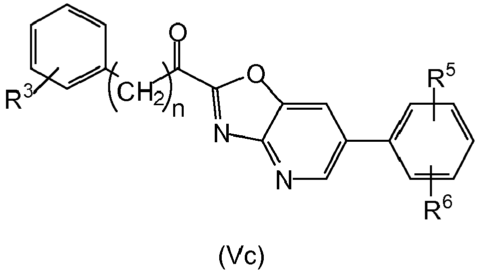 wherein the substituents R¹, R³, R⁵, R⁶, and the integer number n have the meanings as defined herein.

Further preferred are compounds of general formula (I), wherein the residue R⁶ represents H. Thus, preferred are compounds of the general formula (VIa) 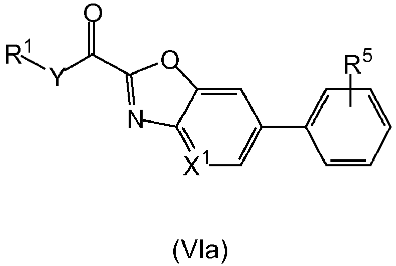 wherein the substituents R¹, R⁵, X¹, and the linker Y have the meanings as defined herein. Particularly preferred are compounds of one of the general formula (VIb), or (VIc) 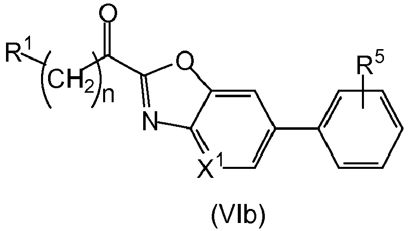 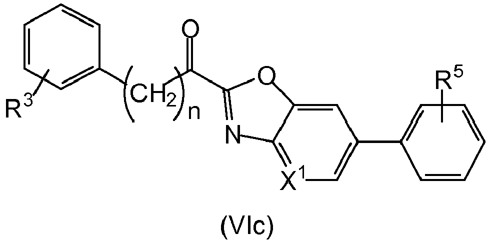 wherein the substituents R¹, R³, R⁵, X¹, and the integer number n have the meanings as defined herein.

Moreover, the invention relates to compounds of the general formula (VIIa) 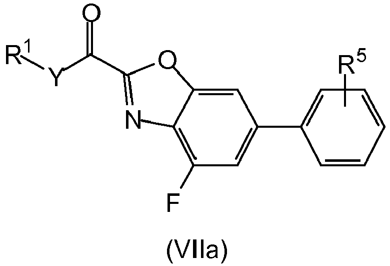 wherein the substituents R¹, R⁵, and the linker Y have the meanings as defined herein. Particularly preferred are compounds of one of the general formula (VIIb), or (VIIc) 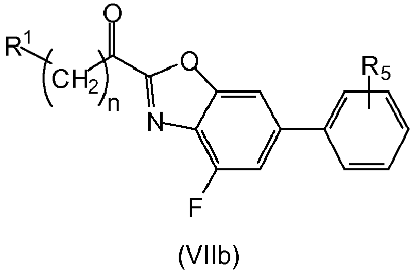 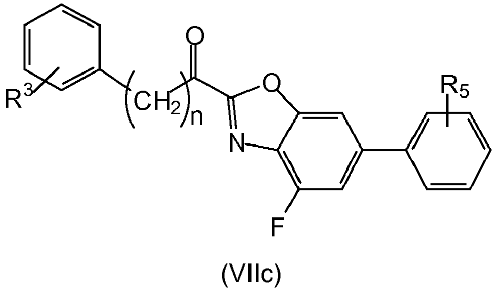 wherein the substituents R¹, R³, R⁵, and the integer number n have the meanings as defined herein.

In another embodiment the invention refers to compounds of the general formula (VIIIa) 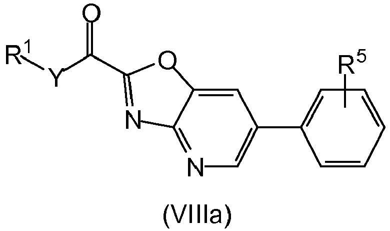 wherein the substituents R¹, R⁵, and the linker Y have the meanings as defined herein. More preferred are compounds of one of the general formula (VIIIb), or (VIIIc) 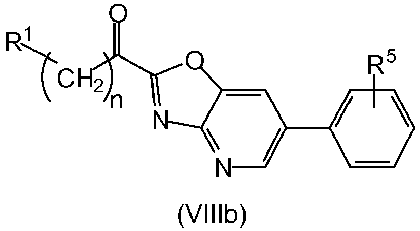 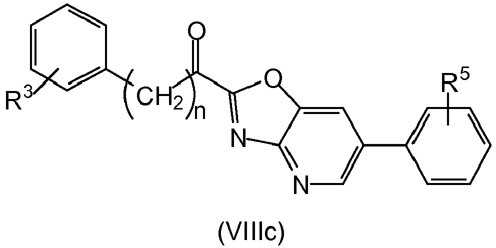 wherein the substituents R¹, R³, R⁵, and the integer number n have the meanings as defined herein.

Preferred are compounds of general formula (I), (Ia), (Ib), (IIIb), (IIIc), (IVb), (IVc), (Vb), (Vc), (VIb), (VIc), (VIIb), (VIIc), (VIIIIb), (VIIIc), wherein Y is selected from: -CH₂-, -C₂H₄-, -C₃H₆-, -C₄H₈-, -C₅H₁₀-, -C₆H₁₂-, -C₇H₁₄-, -C₈H₁₆-, -C₉H₁₈-, -C₁₀H₂₀-, -CH(CH₃)-, -CH(CH₃)CH₂-, -CH₂CH(CH₃)-, -CH(CH₃)-C₂H₄-, -CH₂-CH(CH₃)CH₂, -CH₂-CH₂CH(CH₃)-, -CH(CH₃)-C₃H₆-, -CH₂-CH(CH₃)-C₂H₄-, -CH₂-CH₂-CH(CH₃)CH₂-, -CH₂-CH₂-CH₂CH(CH₃)-, -CH(CH₃)-C₄H₈-, -CH₂-CH(CH₃)-C₃H₆-, -CH₂-CH₂-CH(CH₃)-C₂H₄-, -CH₂-CH₂-CH₂-CH(CH₃)-CH₃-, -CH₂-CH₂-CH₂-CH₂-CH(CH₃)-, -C₂H₄OCH₂-, -C₃H₆OCH₂-, -C₄H₈OCH₂-, -C₅H₁₀OCH₂-, -C₆H₁₂OCH₂-, -C₇H₁₄OCH₂-, -C₈H₁₆OCH₂-, -C₈H₁₈OCH₂-, -C₁₀H₂₀O CH₂-, -CH₂NHCH₂-, -C₂H₄NHCH₂-, -C₃H₆NHCH₂-, -C₄H₈NHCH₂-, -C₅H₁₀NHCH₂-, -C₆H₁₂NHCH₂-, -C₇H₁₄NHCH₂-, -C₈H₁₆NHCH₂-, -C₉H₁₈NHCH₂-, -C₁₀H₂₀NHCH₂-, -CH=CH-, -CH=CH-CH₂-, -CH₂-CH=CH-, -CH=CH-C₂H₄-, -CH₂-CH=CH-CH₂-, -C₂H₄-CH=CH-, -CH=CH-C₃H₆-, CH₂-CH=CH-C₂H₄-, -C₂H₄-CH=CH-CH₂-, C₂H₄-CH₂-CH=CH-, -CH=CH-C₄H₈-, -CH₂-CH=CH-C₃H₆-, -C₂H₄-CH=CH-C₂H₄-, -C₃H₆-CH=CH-CH₂-, -C₄H₈-CH=CH- -CH=CH-CH=CH-, -CH=CH-CH=CH-CH₂-, -CH₂-CH=CH-CH=CH-, -CH=CH-CH₂-CH=CH-, -C≡C-, -C=C-CH₂-, -CH₂-C≡C-, -C≡C-C₂H₄-, -CH₂-C≡C-CH₂-, -C₂H₄-C≡C-, -C₂H₄-C≡C-, -C≡C-C₃H₆-, -CH₂-C≡C-C₂H₄-, -C₂H₄-C≡C-CH₂-, -C₃H₆-C≡C-, -C≡C-C₄H₈-, -CH₂-C≡C-C₃H₆-, -C₂H₄-C≡C-C₂H₄-, -C₃H₆-C≡C-CH₂-, -C≡C-C≡C-, -C≡C-C=C-CH₂-, -C≡C-CH₂-C≡C-, -CH₂-C≡C-C≡C-, -C₂H₄-C≡C-C≡C-, -CH₂-C≡C-CH₂-C≡C-, -C≡C-C₂H₄-C≡C-, -CH₂-C≡C-C≡C-CH₂-, -C≡C-CH₂-C≡C-CH₂-, and -C≡C-C≡C-C₂H₄-.

Particularly preferred are compounds according to general formula (I), (Ia), (Ib), (IIIb), (IIIc), (IVb), (IVc), (Vb), (Vc), (VIb), (VIc), (VIIb), (VIIc), (VIIIIb), (VIIIc), wherein Y is selected from: -CH₂-, -C₂H₄-, -C₃H₆-, -C₄H₈-, -C₅H₁₀-, -C₆H₁₂-, -C₇H₁₄-, -C₈H₁₆-, -C₉H₁₈- -C₁₀H₂₀-, -CH(CH₃)-, -CH(CH₃)CH₂-, -CH₂CH(CH₃)-, -CH(CH₃)-C₂H₄-, -CH₂-CH(CH₃)CH₂, -CH₂-CH₂CH(CH₃)-, -CH(CH₃)-C₃H₆-, -CH₂-CH(CH₃)-C₂H₄-, -CH₂-CH₂-CH(CH₃)CH₂-, -CH₂-CH₂-CH₂CH(CH₃)-, -CH(CH₃)-C₄H₈-, -CH₂-CH(CH₃)-C₃H₆-, -CH₂-CH₂-CH(CH₃)-C₂H₄-, -CH₂-CH₂-CH₂-CH(CH₃)-CH₃-, -CH₂-CH₂-CH₂-CH₂-CH(CH₃)-, -CH=CH-, -CH=CH-CH₂-, -CH₂-CH=CH-, -CH=CH-C₂H₄-, -CH₂-CH=CH-CH₂-, -C₂H₄-CH=CH-, -CH=CH-C₃H₆-, -CH₂-CH=CH-C₂H₄-, -C₂H₄-CH=CH-CH₂-, -C₂H₄-CH₂-CH=CH-, -CH=CH-C₄H₈-, -CH₂-CH=CH-C₃H₆-, -C₂H₄-CH=CH-C₂H₄-, -C₃H₆-CH=CH-CH₂-, -C₄H₈-CH=CH-, -CH=CH-CH=CH-, -CH=CH-CH=CH-CH₂-, -CH₂-CH=CH-CH=CH-, -CH=CH-CH₂-CH=CH-, -C≡C-, -C≡C-CH₂-, -CH₂-C≡C-, -C≡C-C₂H₄-, -CH₂-C≡C-CH₂-, -C₂H₄-C≡C-, -C₂H₄-C≡C-, -C≡C-C₃H₆-, -CH₂-C≡C-C₂H₄-, -C₂H₄-C≡C-CH₂-, -C₃H₆-C≡C-,-C≡C-C₄H₈-, -CH₂-C≡C-C₃H₆-, -C₂H₄-C≡C-C₂H₄-, -C₃H₆-C≡-C-CH₂-, -C=C-C=C-, -C≡C-C=C-CH₂-, -C≡C-CH₂-C≡C-, -CH₂-C≡C-C≡C-, -C₂H₄-C≡C-C≡C-, -CH₂-C≡C-CH₂-C≡C-, -C≡C-C₂H₄-C≡C-, -CH₂-C≡C-C≡C-CH₂-, -C≡C-CH₂-C≡C-CH₂-, and -C=C-C=C-C₂H₄-.

Preferred are compounds of general formula (I), (Ia), (Ib), (IIa) - (IIc), (IIIa) - (IIIc), (IVa) - (IVc), (Va) - (Vc), (VIa) - (VIc), (VIIa) - (VIIc), (VIIIIa) - (Vlllc), wherein R¹ is selected from: R¹ represents: -CF₃, -CHF₂, -CH₂F, -CF₂CF₃, -CHFCF₃, -CF₂CHF₂, -CHFCHF₂, -CF₂CH₂F, -CHFCH₂F, -CHFCH₃, -CF₂CH₃, 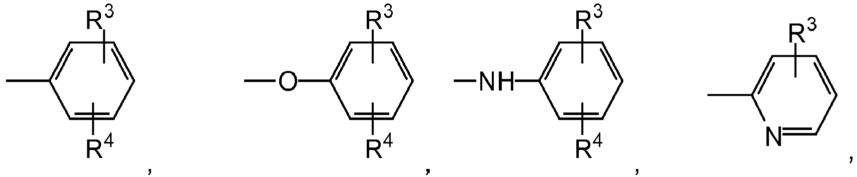 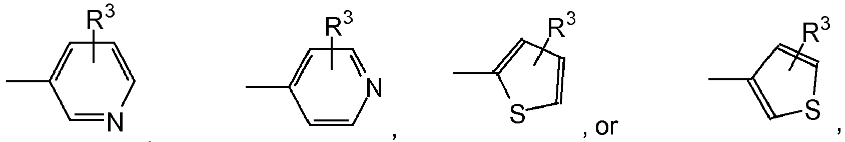
R³ and R⁴ are independently of each other selected from: -R⁸, -R⁹,
   -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SO₂CH₃, -SO₂C₂H₅, -SO₂CH(CH₃)₂, -SO₂C(CH₃)₃, -SO₃H, -SO₃CH₃, -SO₃C₂H₅, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -NH-CO-NH₂, -NH-CO-NHCH₃, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, 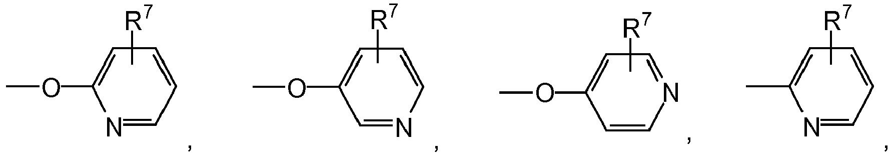 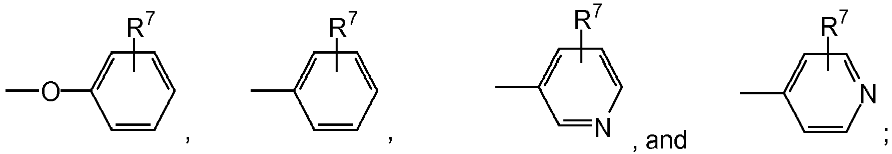
R⁷ has the meaning as described herein.

Further preferred are compounds of general formula (I), (Ia), (Ib), (IIa) - (IIc), (IIIa) - (IIIc), (IVa) - (IVc), (Va) - (Vc), (VIa) - (VIc), (VIIa) - (VIIc), (VIIIIa) - (VIIIc), wherein R¹ is selected from: -CF₃, -CHF₂, -CH₂F, -CF₂CF₃, -CHFCF₃, -CF₂CHF₂, -CHFCHF₂, -CF₂CH₂F, -CHFCH₂F, -CHFCH₃, -CF₂CH₃, 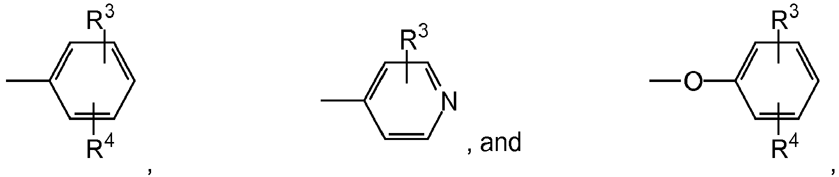 R³ and R⁴ are independently of each other selected from: -H, 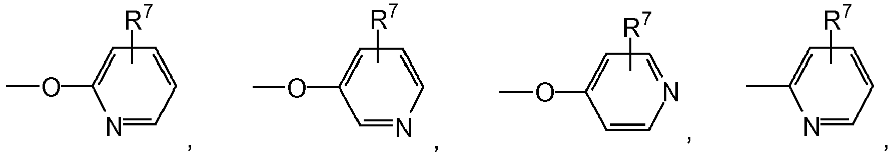 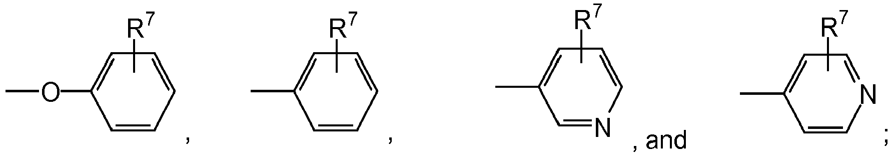 R⁷ has the meaning as described herein.

Particularly preferred are compounds of general formula (I), (Ia), (Ib), (IIa) - (IIc), (IIIa) - (IIIc), (IVa) - (IVc), (Va) - (Vc), (VIa) - (VIc), (VIIa) - (VIIc), (VIIIIa) - (VIIIc), wherein R³ and R⁴ are independently of each other selected from: -H, -F, -CH₂F, 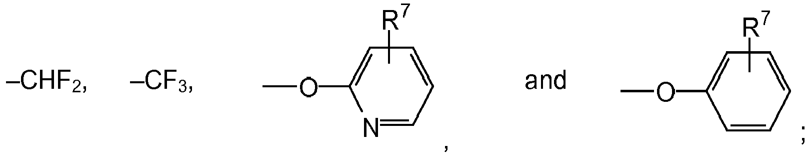 R⁷ has the meaning as described herein.

Particularly preferred are compounds of general formula (I), (Ia), (Ib), (IIa) - (IIc), (IIIa) - (IIIc), (IVa) - (IVc), (Va) - (Vc), (VIa) - (VIc), (VIIa) - (Vllc), (Vlllla) - (Vlllc), wherein R⁷ is selected from: -H, -F, -Cl, -Br, -I, -CH₂F, -CHF₂, -CF₃, -CF₂CF₃, -CH₂CH₂F,-CH₂CHF₂, -CH₂CF₃, -CF₂CF₂CF₃, -CH₂CH₂CF₃, -CF=CF₂, -CH=CF₂, -CF=CFCF₃, -CH=CHCF₃, -CF₂CF=CF₂, -CH₂CH=CF₂, -OH, -OCH₃, -OC₂H₅, -OC₃H₇, -OC₃H₇, -OC₄H₉, -NH₂, -NHCH₃, -NHC₂H₅, -NHC₃H₇, -NHC₄H₉, -CH₃, -C₂H₅, -C₃H₇, -C₄H₉, -C₅H₁₁, -CH=CH₂, -CH=CH-CH₃, -CH₂-CH=CH₂, -CH=CH-C₂H₅, -CH₂-CH=CH-CH₃, -C₂H₄-CH=CH₂, -CH=CH-C₃H₇, -CH₂-CH=CH-C₂H₅, -C₂H₄-CH=CH-CH₃, C₂H₄-CH₂-CH=CH₂, -CH=CH-C₄H₉, -CH₂-CH=CH-C₃H₇, -C₂H₄-CH=CH-C₂H₅, -C₃H₆-CH=CH-CH₃, -C₄H₈-CH=CH₂, -CH=CH-CH=CH₂, -CH=CH-CH=CH-CH₃, -CH₂-CH=CH-CH=CH₂, -CH=CH-CH₂-CH=CH₂, -C≡C-CH₃, -CH₂-C≡CH, -C≡C-C₂H₅, -CH₂-C≡C-CH₃, -C₂H₄-C≡CH, -C₂H₄-C≡CH, -C≡C-C₃H₇, -CH₂-C≡C-C₂H₅, -C₂H₄-C≡C-CH₃, -C₃H₆-C≡CH, -C≡C-C₄H₉, -CH₂-C≡C-C₃H₇, -C₂H₄-C≡C-C₂H₅, -C₃H₆-C≡C-CH₃, -Ph, -CH₂-Ph, -CH₂-CH₂-Ph, -CH=CH-Ph, -CPh₃, -OCH₂-Ph, and -OC₂H₄-Ph.

Preferred are compounds of general formula (I), (Ia), (Ib), (IIa) - (IIc), (IIIa) - (IIIc), (IVa) - (IVc), (Va) - (Vc), (VIa) - (VIc), (VIIa) - (Vllc), (Vlllla) - (Vlllc), wherein R² represents one of the following moieties: -CH(CH₃)₂, -CH(C₂H₅)₂, -CH₂CH(CH₃)₂, -C(CH₃)₃, -CH₂C(CH₃)₃, -CHPh₂, -CPh₃, -CH₂CPh₃,

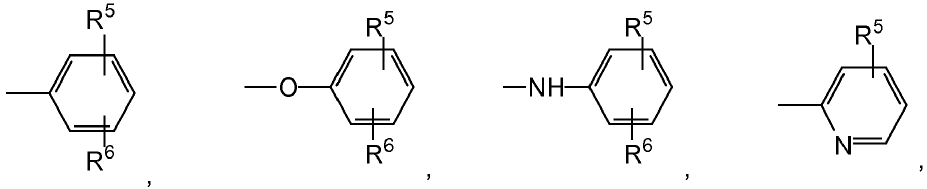

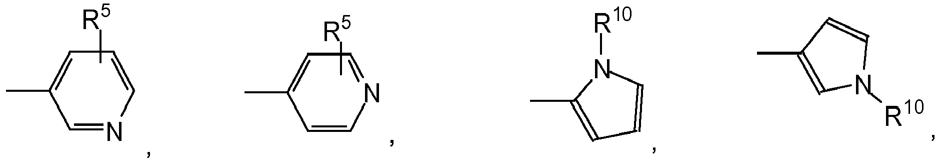

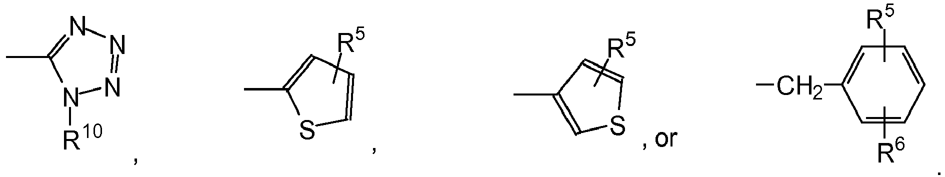

Even more preferred are compounds of general formula (I), (Ia), (Ib), (IIa) - (IIc), (IIIa) - (IIIc), (IVa) - (IVc), (Va) - (Vc), (VIa) - (VIc), (VIIa) - (VIIc), (VIIIIa) - (VIIIc), wherein R² is selected from: 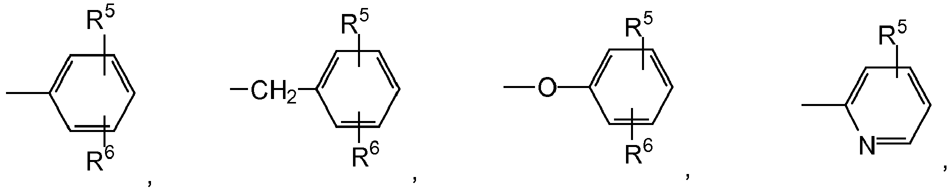 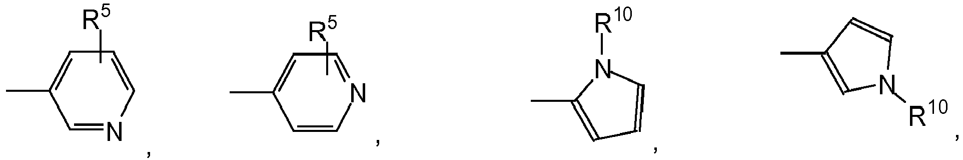 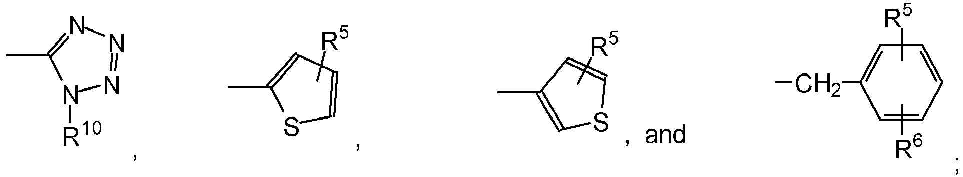 R⁵ and R⁶ are independently of each other selected from:
-H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -NO₂, -F, -CI, -Br, -I, -N₃, -CN, -COC₂H₅, -COC₃H₇, -COOH, -COOCH₃, -COOC₂H₅, -COOC₃H₇, -CONH₂, -CONHCH₃, -NH₂, -NHCH₃, -NHC₂H₅, -NHC₃H₇, -N(C₂H₅)₂, -CH₂F, -CHF₂, -CF₃, -CF₂CF₃, -CH₂CH₂F, -CH₂CHF₂, -CH₂CF₃, -CF₂CF₂CF₃, -CH₂CH₂CF₃, -CF=CF₂, -CH=CF₂, -CF=CFCF₃, -CH=CHCF₃, -CF₂CF=CF₂, -CH₂CH=CF₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -CH=CH₂, -CH₂-C(CH₃)₂-CH=CH₂, -C(CH₃)₂-CH₂-CH=CH₂, -CH=C(CH₃)-CH(CH₃)₂, -Ph, -CHPh₂, -CPh₃, -CH=CH-Ph, -CH₂-Ph, -C₂H₄-Ph, -OCH₂-Ph, and -OC₂H₄-Ph.

Particularly preferred are compounds of general formula (I), (Ia), (Ib), (IIa) - (IIc), (IIIa) - (IIIc), (IVa) - (IVc), (Va) - (Vc), (VIa) - (VIc), (VIIa) - (VIIc), (VIIIIa) - (VIIIc), wherein R₅ and R₆ independently of each other selected from: -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -NO₂, -F, -CI, -Br, -I, -N₃, -CN, -CH₂F, -CHF₂, -CF₃, -CF₂CF₃, -CH₂CH₂F, -CH₂CHF₂, -CH₂CF₃, -CF₂CF₂CF₃, -CH₂CH₂CF₃, -CF=CF₂, -CH=CF₂, -CF=CFCF₃, -CH=CHCF₃, -CF₂CF=CF₂, -CH₂CH=CF₂, -CH₃, -C₂H₅, -C₃H₇, -CH(CH₃)₂, -CH₂-CH(CH₃)₂, -C(CH₃)₃, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -CH=CH₂, -Ph, -CHPh₂, -CPh₃, -CH=CH-Ph, -CH₂-Ph, -C₂H₄-Ph, -OCH₂-Ph, and -OC₂H₄-Ph.

Further preferred are compound of general formula (I), (Ia), or (Ib), wherein the residue R² represents 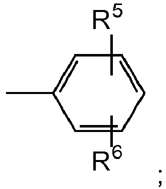 R⁵ and R⁶ independently of each other selected from:
-H, -CH₂F, -CHF₂, -CF₃, -CF₂CF₃, -CH₂CH₂F, -CH₂CHF₂, -CH₂CF₃, -CF₂CF₂CF₃, -CH₂CH₂CF₃, -CF=CF₂, -CH=CF₂, -CF=CFCF₃, -CH=CHCF₃, -CF₂CF=CF₂, -CH₂CH=CF₂, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -CH=CH₂, -CH₂-C(CH₃)₂-CH=CH₂, -C(CH₃)₂-CH₂-CH=CH₂, -CH=C(CH₃)-CH(CH₃)₂, -Ph, -CH=CH-Ph, -CH₂-Ph, -C₂H₄-Ph, -OCH₂-Ph, and -OC₂H₄-Ph.

Further preferred embodiments are represented by one of the following general formulas wherein Y, X¹, R², R³, R⁵, and R⁶ are defined as written above.

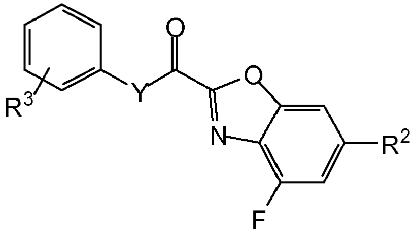

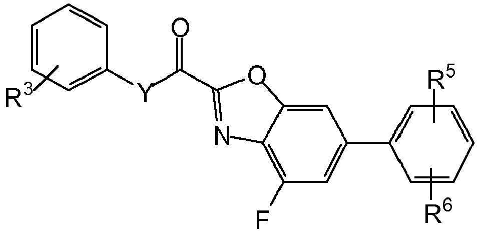

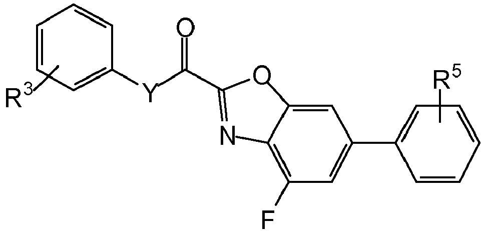

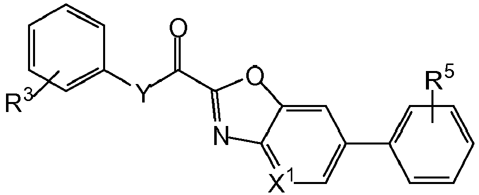

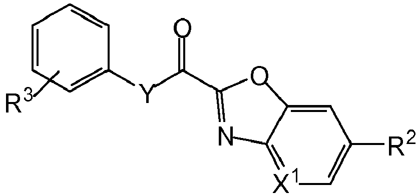

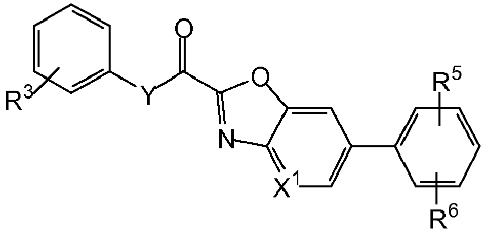

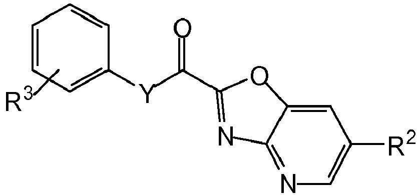

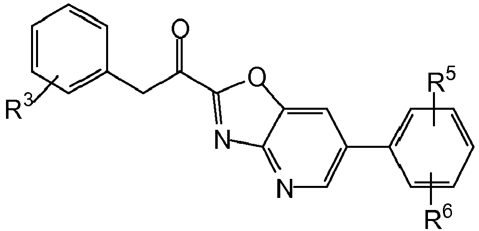

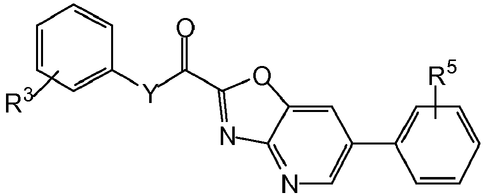

A further embodiment of the present invention relates to compounds of the general formula (I) 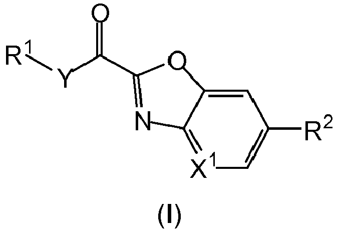 wherein
X¹ is -CF- or -N-;
Y represents -CH₂-, -C₂H₄-, -C₃H₆-, -C₄H₈-, or -C₅H₁₀-;
R¹ represents: -CF₃, 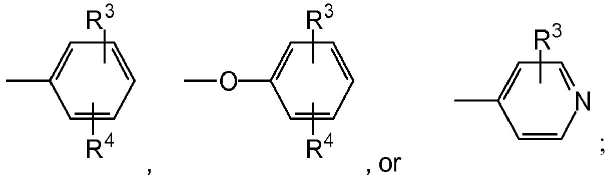
R² represents: 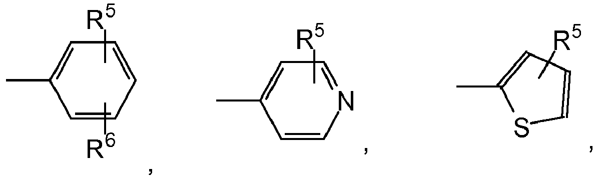 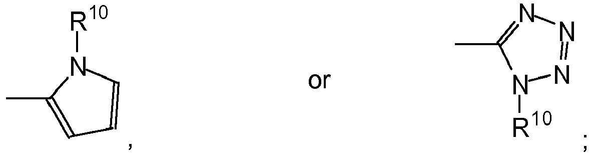
R³ represents -H;
R⁴ represents -H, F, 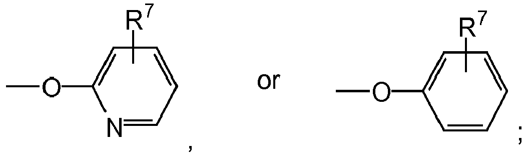
R⁵ represents -H, or -CH₃;
R⁶ represents -F, -CI, -CH₃, -OCH₃, or -CN;
R⁷ represents -H or -CF₃;
R¹⁰ represents: -H or -CH₃;
and enantiomers, stereoisomeric forms, mixtures of enantiomers, diastereomers, mixtures of diastereomers, prodrugs, hydrates, solvates, acid salt forms, tautomers, and racemates of the above mentioned compounds and pharmaceutically acceptable salts thereof.

A particularly preferred compound of the present invention is selected from the group comprising or consisting of:
1-(6-(*p*-tolyl)oxazolo[4,5-*b*]pyridin-2-yl)-6-phenylhexan-1-one
3-(2-phenoxyphenyl)-1-(6-(*p*-tolyl)oxazolo[4,5-b]pyridin-2-yl)propan-1-one,
3-(3-Phenoxyphenyl)-1-(6-(*p*-tolyl)oxazolo[4,5-*b*]pyridin-2-yl)propan-1-one,
3-(4-phenoxyphenyl)-1-(6-(*p*-tolyl)oxazolo[4,5-*b*]pyridin-2-yl)propan-1-one,
1-(6-(p-Tolyl)oxazolo[4,5-*b*]pyridin-2-yl)-3-(4-((5-(trifluoromethyl)pyridin-2-yl)oxy)phenyl)propan-1-one,
1-(4-fluoro-6-(4-fluorophenyl)benzo[d]oxazol-2-yl)-6-phenylhexan-1-one,
1-(4-fluoro-6-(2-fluorophenyl)benzo[d]oxazol-2-yl)-6-phenylhexan-1-one,
1-(4-fluoro-6-(*p*-tolyl)benzo[d]oxazol-2-yl)-6-phenylhexan-1-one, and
1-(4-fluoro-6-(o-tolyl)benzo[d]oxazol-2-yl)-6-phenyl hexan-1-one,
2-phenyl-1-(6-(p-tolyl)oxazolo[4,5-*b*]pyridin-2-yl)ethan-1-one,
3-phenyl-1-(6-(*p*-tolyl)oxazolo[4,5-*b*]pyridin-2-yl)propan-1-one,
4-phenyl-1-(6-(*p*-tolyl)oxazolo[4,5-*b*]pyridin-2-yl)butan-1-one,
6-phenyl-1-(6-(pyridin-4-yl)oxazolo[4,5-*b*]pyridin-2-yl)hexan-1-one,
1-(6-(2-chlorophenyl)oxazolo[4,5-*b*]pyridin-2-yl)-6-phenylhexan-1-one,
1-(6-(3-chlorophenyl)oxazolo[4,5-*b*]pyridin-2-yl)-6-phenylhexan-1-one,
1-(6-(4-chlorophenyl)oxazolo[4,5-*b*]pyridin-2-yl)-6-phenylhexan-1-one,
1-(6-(4-methoxyphenyl)oxazolo[4,5-*b*]pyridin-2-yl)-6-phenylhexan-1-one,
4,4,4-trifluoro-1-(6-(*p*-tolyl)oxazolo[4,5-*b*]pyridin-2-yl)butan-1-one,
6,6,6-trifluoro-1-(6-(*p*-tolyl)oxazolo[4,5-*b*]pyridin-2-yl)hexan-1-one,
6-phenyl-1-(6-(thiophen-2-yl)oxazolo[4,5-*b*]pyridin-2-yl)hexan-1-one,
1-(6-(1*H*-tetrazol-5-yl)oxazolo[4,5-*b*]pyridin-2-yl)-6-phenylhexan-1-one,
1-(6-(1-methyl-1*H*-pyrrol-2-yl)oxazolo[4,5-*b*]pyridin-2-yl)-6-phenylhexan-1-one,
6-(4-fluorophenyl)-1-(6-(*o*-tolyl)oxazolo[4,5-*b*]pyridin-2-yl)hexan-1-one,
1-(6-(2-fluorophenyl)oxazolo[4,5-*b*]pyridin-2-yl)-6-(pyridin-4-yl)hexan-1-one,
5-(4-fluorophenoxy)-1-(6-(o-tolyl)oxazolo[4,5-*b*]pyridin-2-yl)pentan-1-one,
1-(6-(2,6-dimethylphenyl)oxazolo[4,5-*b*]pyridin-2-yl)-6-phenylhexan-1-one,
4-(2-(6-phenylhexanoyl)oxazolo[4,5-*b*]pyridin-6-yl)benzonitrile,
1-(6-(3-fluorophenyl)oxazolo[4,5-*b*]pyridin-2-yl)-6-phenylhexan-1-one,
6-phenyl-1-(6-(pyridin-2-yl)oxazolo[4,5-*b*]pyridin-2-yl)hexan-1-one,
6-phenyl-1-(6-(*o*-tolyl)oxazolo[4,5-*b*]pyridin-2-yl)hexan-1-one,
6-phenyl-1-(6-(*m*-tolyl)oxazolo[4,5-*b*]pyridin-2-yl)hexan-1-one,
6-phenyl-1-(6-(pyridin-4-yl)oxazolo[4,5-*b*]pyridin-2-yl)hexan-1-one,
1-(6-(2-fluorophenyl)oxazolo[4,5-*b*]pyridin-2-yl)-6-phenylhexan-1-one,
1-(6-(4-fluorophenyl)oxazolo[4,5-*b*]pyridin-2-yl)-6-phenylhexan-1-one.

One aspect of the invention are the compounds as defined by general formula (I) being selective inhibitors of diacylglycerol lipase α and/or diacylglycerol lipase β. The compounds of the present invention are efficient and highly selective inhibitors of DAGL and thus, are suitable for the treatment of disorders associated with, accompanied by and/or caused by increased 2-arachidonoylglycerol levels, and thereby having an effect on endocannabinoid signaling pathway (preferred on 2-AG signalling pathway) and on prostaglandin E₂ glycerol ester formation. Thereby the series of molecular signals generated as a consequence of an endocannabinoid binding to a cell surface receptor is defined as endocannabinoid signaling pathway and the series of molecular signals generated as a consequence of 2-AG binding to a cell surface receptor is defined as 2-AG signaling pathway. The pathway proceeds with the receptor transmitting the signal to a heterotrimeric G-protein complex and ends with regulation of a downstream cellular process, e.g. transcription.

Therefore one embodiment of the present invention refers to a compound of general formula (I) for use in the treatment and/or prevention of neurodegenerative diseases, inflammatory disease and impaired energy balance, wherein the compound prevents the formation of prostaglandin ester formation, and in particular prevents formation of prostaglandin E₂ glycerol ester.

DAGL-dependent endocannabinoid signalling regulates axonal growth and guidance during development, and is required for the generation and migration of new neurons in the adult brain. Adult neurogenesis constitutes a form of cellular plasticity in the developed brain that impacts on memory, depression and neurodegenerative diseases. A role for the CB₁ receptors in adult neurogenesis is supported by studies that show that antagonists of these receptors, or genetic deletion of them, reduce the number of proliferating stem cells in the hippocampus, whereas agonists can increase the number of said cells.

Additionally, diacylglycerol lipase α and diacylglycerol lipase β can also function independendly of cannabinoid receptors. The product of DAGL, 2-AG, is a precursor for synthesis of arachidonic acid and its metabolites (such as prostaglandins). For example, 2-AG is the precursor of arachidonic acid (AA) in a pathway that drives the cyclooxygenase-dependent generation of inflammatory prostaglandins in the brain, which has recently been implicated in the degeneration of dopaminergic neurons in Parkinson's disease.

In summary, the DAGLs regulate a wide range of biological responses via the generation of a cannabinoid receptor signalling pool of 2-AG, but also serve as 'hub' enzymes in pathways that generate and/or maintain signalling pools of AA and various prostanoids. DAGLs are also key enzymes with regulatory roles in pathological processes, including driving inflammatory responses and neurodegeneration.

The compounds of the present invention are efficient inhibitors of DAGLs. The inventive compounds are suitable for the use as a pharmaceutically active agent. The inventive compounds are suitable for the treatment and/or prevention of DAGL activity induced disorders. Thus, one aspect of the present invention refers to the inventive compounds for use in the treatment and/or prevention of neurodegenerative diseases, inflammatory disease and impaired energy balance.

The inventive compounds are used in the manufacture of a medicament or of a pharmaceutical composition for the treatment of disorders associated with, accompanied by and/or caused by pathologically 2-arachidonoylglycerol levels. The inventive compounds are further used in the manufacture of a medicament or of a pharmaceutical composition for the treatment and/or prevention of neurodegenerative diseases, inflammatory disease and impaired energy balance.

The compounds of the invention are capable of inhibiting neurodegeneration and thus, are suitable for the treatment and/or prevention of neurodegenerative diseases, particularly selected from the group comprising or consisting of: amyotrophic lateral sclerosis (ALS), Parkinson's disease, Huntington's disease, spinal muscular-atrophy, progressive supranuclear palsy (Steele-Richardson-Olszewski syndrome), Wilson's disease, multi-system atrophy (MSA-P, MSA-C), Shy-Drager syndrome, Alzheimer's disease, spinocerebellar ataxia (SCA), glaucoma, Pick's disease, Lewy-body disease, Hallervorden-Spatz disease, Creutzfeld-Jakob-disease, Machado-Joseph disease, Friedreich ataxia, non-Friedreich ataxias, Gilles de la Tourette syndrome, familial tremors, olivopontocerebellar degenerations, paraneoplasticcerebral syndromes, hereditary spastic paraplegias, hereditary optic neuropathy (Leber), degenerative retinal diseases, retinitis pigmentosa, dry and humid macular degeneration, Stargardt disease, and Keams- Sayre syndrome. Of the enurodegenartive disease Parkinson's disease is preferred.

Prostaglandins or eicosanoids are lipid autacoids derived from arachidonic acid. They sustain homeostatic functions and mediate pathogenic mechanisms, including the inflammatory response. They are generated by the action of cyclooxygenase (COX) isoenzymes. Inflammation is the immune system's response to infection and injury and has been implicated in the pathogeneses of several disorders such as in neurodegenerative disease. Inflammation is an intrinsically beneficial event that leads to removal of offending factors and restoration of tissue structure and physiological function. Prostaglandins play a key role in the process of inflammation. Their biosynthesis is significantly increased in inflamed tissue and they contribute to the development of the cardinal signs of acute inflammation.

The compounds of the invention are capable of inhibiting synthesis of 2-AG being a precursor for arachidonic acid and its metabolites, in particular prostaglandin, synthesis and thus, are suitable for the treatment and/or prevention of inflammatory disease in particular of inflammatory disease mediated by arachidonic acid metabolites (such as prostaglandins) or prostaglandin E₂-glycerol.

One preferred embodiment of the present invention refers to the inventive compounds according to general formula (I) for use in the treatment of inflammatory disease caused, induced, initiated and/or enhanced by bacteria, viruses, prions, parasites, fungi, and/or caused by irritative, traumatic, metabolic, allergic, autoimmune, or idiopathic reasons. Thereby the viruses are selected from the group comprising or consisting of human immunodeficiency virus-I, herpes viruses, herpes simplex virus, herpes zoster virus, humam papiloma virus, influenza virus, rota virus and cytomegalovirus; and the bacteria are selected from the group comprising or consisting of mycoplasma pulmonis, ureaplasma urealyticum, mycoplasma pneumoniae, chlamydia pneumoniae, C. pneumoniae, Escherichia coli, Salmonella, Neisseria meningitidis Staphylococcus aureus, Streptococcal bacteria, Helicobacter pylori, and propriono-bacterium.

Another preferred embodiment of the present invention refers to the inventive compounds according to general formula (I) for use in the treatment of inflammatory disease, wherein the inflammatory disease is selected from the group comprising or consisting of inflammatory diseases of the central nervous system (CNS), inflammatory rheumatic diseases, inflammatory diseases of blood vessels, inflammatory diseases of the middle ear, inflammatory bowel diseases, inflammatory diseases of the skin, inflammatory disease uveitis, and inflammatory diseases of the larynx.

In addition, prostaglandins play a role in pain. High concentrations of prostaglandins cause pain by direct action upon nerve endings and at low concentrations, they markedly increase sensitivity to pain. Prostaglandins are also incriminated in pain perception within the nervous system. They are produced within the central nervous system and sensitize it to painful substances. Pain is thus induced in two ways (local and central). Therefore one aspect of the present invention refers to the inventive compounds used as analgesic agents.

The compounds of the present invention are efficient and highly selective inhibitors of DAGL and have been shown to have an effect on endocannabinoid signaling pathway. Activation of cannabinoid CB₁ receptors has been implicated in increased food uptake and drug addiction. As a consequence the inventive compounds according to general formula (I) are useful for therapeutic intervention in impaired energy balance and in particular of obesity.

Diseases related to impaired energy balance refer to diseases and conditions characterized by pathological disorders of the metabolism mainly caused by deregulated amount of energy eaten and/or metabolized. Energy balance is the relationship between energy intake (food calories taken into the body by food and drink) and energy consumption (calories being used in the body). Energy intake refers to the sum of calories consumed as food and energy expenditure is mainly a sum of internal heat produced and external work. Diseases related to impaired energy balance used herein are particularly, but are not limited to, obesity and diabetes type 2.

Obesity is defined as abnormal or excessive fat accumulation that may impair health. The body mass index (BMI; weight in kg divided by height in meters squared) may be used to classify overweight and obesity. BMI are sex and age dependent. In general overweight is defined as having a BMI ≥ 25 kg/m² and obesity is defined as having a BMI ≥ 30 kg/m².

Another embodiment of the present invention refers to the compounds according to general formula (I) for use in the treatment of drug abuse or drug addiction. It is preferred that the drug is selected from the group comprising or consisting of Δ⁹- tetrahydrocannabinol, ethanol, nicotine, cocaine and opiates.

Still another aspect of the present invention deals with pharmaceutical compositions comprising at least one compound according to general formula (I) as an active ingredient, together with at least one pharmaceutically acceptable carrier, excipient and/or diluents. The pharmaceutical compositions of the present invention can be prepared in a conventional solid or liquid carrier or diluents and a conventional pharmaceutically-made adjuvant at suitable dosage level in a known way. The preferred preparations are adapted for oral application or parenteral application via injection. These administration forms include, for example, pills, tablets, film tablets, coated tablets, capsules, liposomal formulations, micro- and nano-formulations, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention, especially one pure optical isomer, and/or a pharmaceutically acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, aerosol preparations consistent with conventional pharmaceutical practices. Other suitable formulations are gels, elixirs, dispersable granules, syrups, suspensions, creams, lotions, solutions, emulsions, suspensions, dispersions, and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

As pharmaceutically acceptable carrier, excipient and/or diluents can be used carriers such as preferably with an inert carrier like lactose, starch, sucrose, cellulose, magnesium stearate, dicalcium phosphate, calcium sulfate, talc, mannitol, ethyl alcohol (liquid filled capsules). Suitable binders include starch, gelatin, natural sugars, corn sweeteners, natural and synthetic gums such as acacia, sodium alginate, carboxymethylcellulose, polyethylene glycol and waxes, sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. Lubricants are selected from the group comprising of comsiting of boric acid, sodium benzoate, sodium acetate, sodium chloride, magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Suitable disintegrating agents (disintegrates) are starch, methylcellulose, guar gum, modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. Further components of the pharmaceutical compositions may be coloring agents, sweetening agents, flavoring agents, preservatives; glidents such as silicon dioxide and talc and suitable adsorbent such as clay, aluminum oxide. Suitable diluents are water or water/propylene glycol solutions for parenteral injections, juice, sugars such as lactose, sucrose, mannitol, and sorbitol, starches derived from wheat, corn rice, and potato, and celluloses such as microcrystalline cellulose.

### Description of the Figures

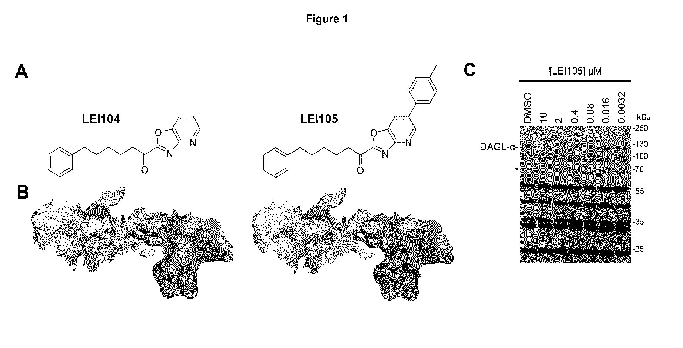

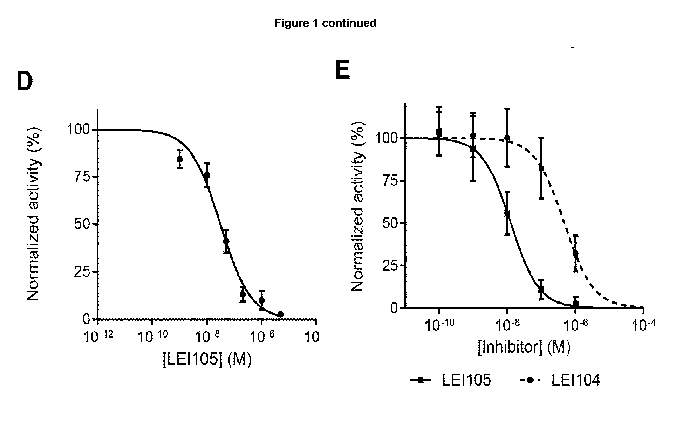

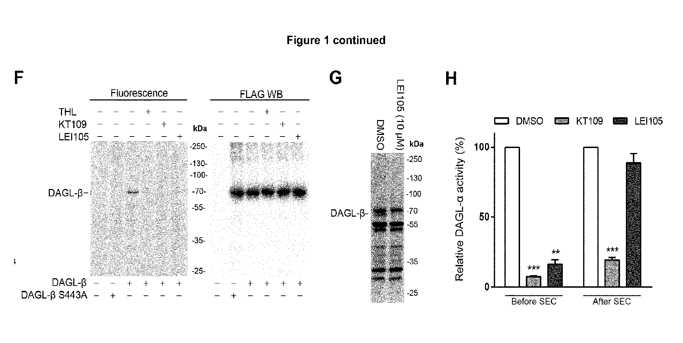

**Figure 1****:** shows (a) a representative fluorescent ABPP gel showing dose dependent inhibition of endogenous DAGL-α labeling by MB064 (250 nM) by inventive compound 25 (LEI105) in the mouse brain membrane proteome. (b) Dose response curve of DAGL-α inhibition as determined with competitive ABPP (pIC₅₀ 7.5 ± 0.07; *n* = 3). (c) Dose response curve of DAGL-α inhibition by comparative compound 1 (pIC₅ₒ 6.3 ± 0.1; *n* = 4) and inventive compound 25 (pIC₅₀ 7.9 ± 0.08 nM; *n* = 4) as determined with a glycerol based natural substrate assay. (d) ABPP using MB064 (1 µM) with different hDAGL-β constructs and anti-FLAG western blot of the same gel. (e) Competitive ABPP in the mouse spleen membrane proteome using MB064 (1.0 µM) in competition with 10 µM of inventive compound 25 (LEI105), which can block labeling of endogenously expressed DAGL-β in the mouse spleen membrane proteome. (f) Schematic representation of the size exclusion chromatography (SEC) experiment that shows reversibility of inventive compound 25 (n = 3). Statistical analysis: 2-way ANOVA (*** = p < 0.001; ** = p < 0.01 vs vehicle)

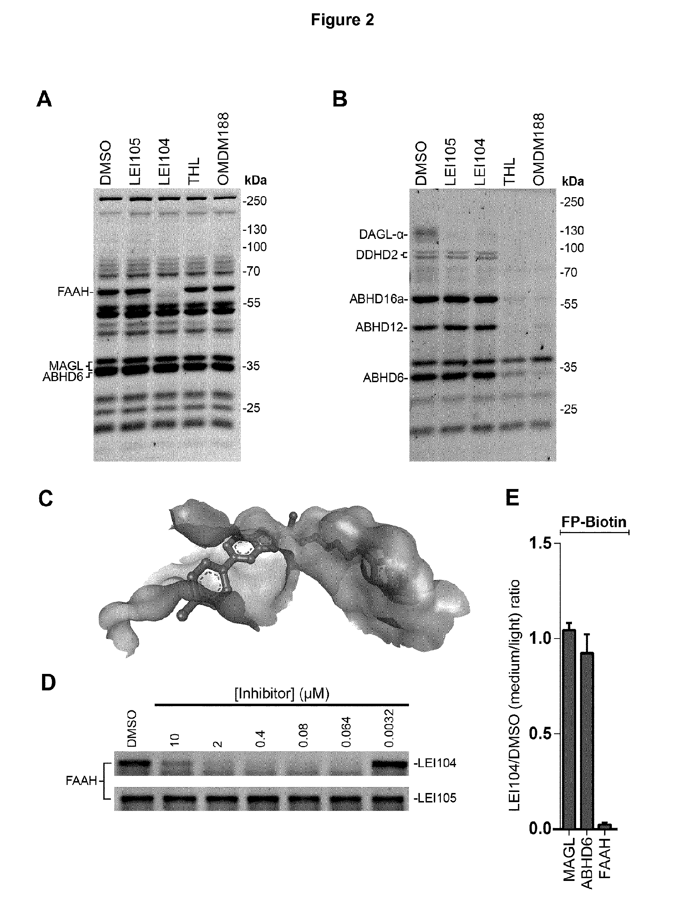

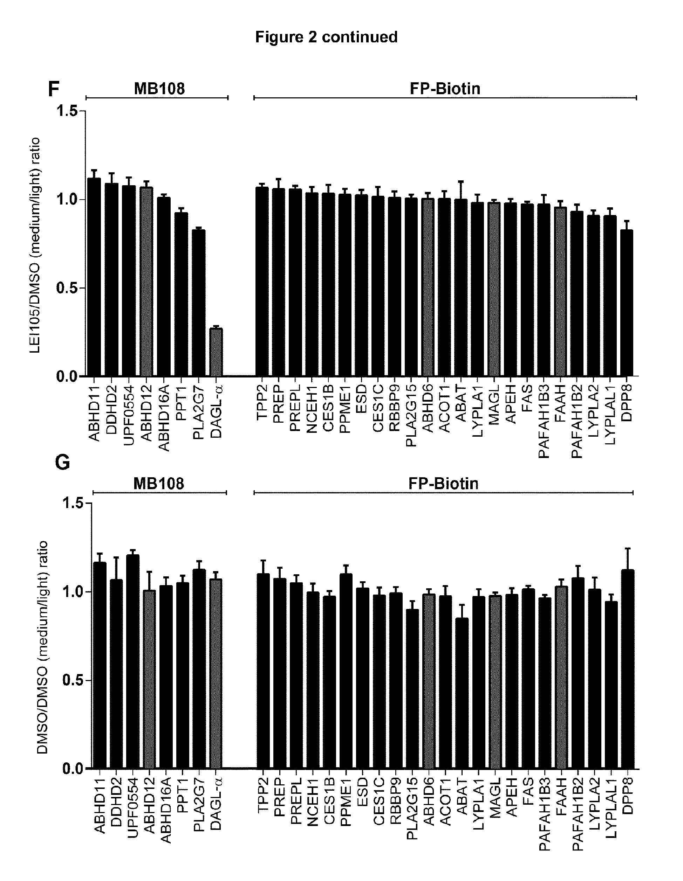

**Figure 2****:** shows selectivity of inventive compound 25 (LEI105). (a) Competitive ABPP in the mouse brain membrane proteome with comparative compound 1 (10 µM), inventive compound 25 (LEI105) (10 µM), OMDM188 (1 µM) and THL (1 µM) using TAMRA-FP (500 nM). (b) Same experiment as in (a) with THL based ABP MB064 (250 nM) showing excellent selectivity of inventive compound 25 at 10 µM compared to THL and OMDM188. (c) FAAH inhibitor OL-135 with the α-ketoheterocycle substituted by the α-ketoheterocycle of inventive compound 25 (LEI105) in the crystal structure of FAAH, showing a steric clash between the toluoyl group and FAAH. (d) Activity of comparative compound 1 and inventive compound 25 (LEI105) against FAAH in a dose response experiment in the mouse brain membrane proteome using TAMRA-FP (500 nM). (e) Chemoproteomic competition between comparative compound 1 (10 µM) and FP-biotin (5 µm) for FAAH and MgII, showing that the chemproteomic settings using FP-biotin are compatible with the reversible α-ketoheterocycles. (f) THL-Biotin (2.5 µM) and FP-biotin based chemoproteomic analysis of serine hydrolase activities in the mouse brain membrane proteome treated with inventive compound 25 (LEI105) (10 µM). DAGL-α is inhibited to over 70% and no other targets were detected. (g) Chemoproteomic control experiment shows equal isotopic labeling and detection of peptides from THL-biotin and FP-biotin labeled proteins. Error bars represent ± s.e.m. of medium over light ratios of quantified peptides (minimum of two unique peptides per enzyme).

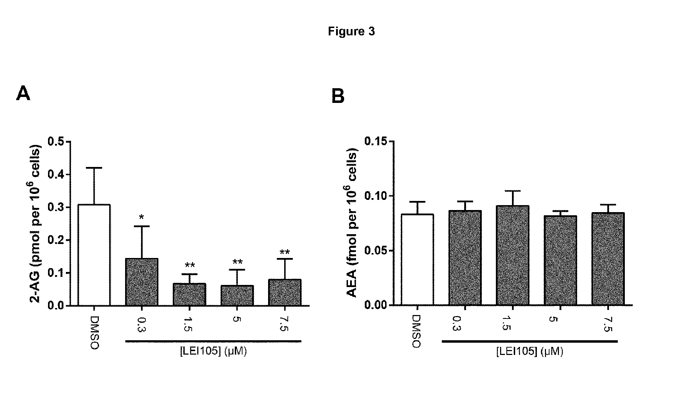

**Figure 3****:** shows cellular activity of the inventive compound 25 (LEI105). (a) *In situ* treatment of Neuro2A cels (1h, 37 °C) dose dependently decreased basal 2-AG levels while keeping anandamide levels (b) constant (mean ± SEM; *n* =4). Statistical analysis: 2-way ANOVA (*** = p < 0.001; ** = p < 0.01; * = p < 0.05 vs vehicle)

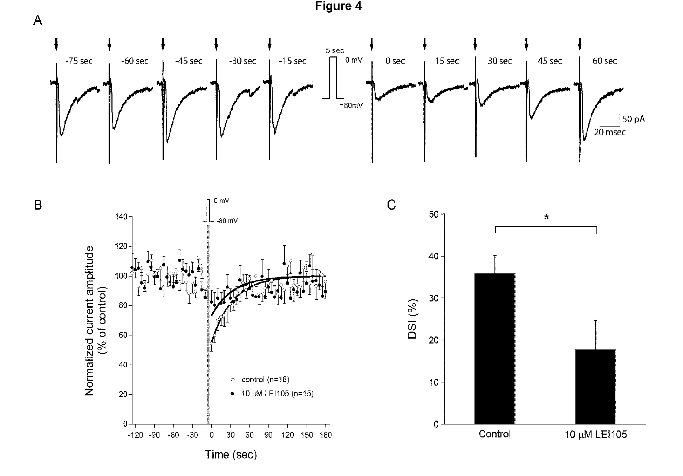

**Figure 4****:** DSI is reduced in hippocampal slices treated with 10 µM of inventive compound 25 (LEI015). (a) Example of a typical whole-cell voltage-clamp recording of evoked inhibitory post-synaptic currents (IPSCs) in a CA1 pyramidal neuron under control conditions. IPSCs are evoked every 5 seconds, but for clarity, IPSCs shown here are representative traces recorded every 15 seconds. DSI is induced with a 5-sec duration depolarization from -80 mV to 0 mV and is observed as a marked and brief reduction of IPSC amplitude following the depolarization. (b) Averaged IPSC amplitude recorded every 5 seconds in CA1 neurons from vehicle-treated slices (DMSO) and from slices pre-incubated at least 30 minutes and in the continuous presence of inventive compound 25 (LEI 105) (10 µM). The dark lines represent the single exponential fit of the recovery phase following the depolarizing step. (c) Initial DSI amplitude under control conditions (DMSO) and in the presence of LE1105 (10 µM). (*P<0.05)

### Examples

### Abbreviations

- 2-AG: 2-Arachidonoylglycerol
- aACSF: Adapted artificial cerebrospinal fluid
- ACSF:: Artificial cerebrospinal fluid
- ABP:: Activity based probe
- ABPP:: Activity based protein profiling
- ACN:: Acetonitrile
- AP5:: (2R)-Amino-5-phosphonovaleric acid
- APT:: Attached proton test
- ATP:: Adenosine triphosphate
- BnBr:: Benzylbromide
- BSA: Bovine serum albumin
- BODIPY:: Boron-dipyrromethene
- CA1:: *Cornu Ammonis 1*
- CB2:: Cannabinoid receptor 2
- CDI:: Carbodiimidazole
- CHOK1hCB₁_bgal:: CHOK1 cell line expressing human cannabinoid receptor 1
- CNQX:: 6-cyano-7-nitroquinoxaline-2,3-dione
- DME:: 1,2-Dimethoxyethane
- DAGL-α: Sn1 specific diacylglycerol lipase-α
- DAGL-β: Sn1 specific diacylglycerol lipase-β
- DCM:: Dichloromethane
- DMF:: Dimethylformamide
- DPBS:: Dulbecco's phosphate-buffered saline
- DSI:: Depolarization-induced suppression of inhibition
- DTT:: Dithiothreitol
- DMP:: Dess-Martin Periodinane
- DMSO:: Dimethylsulfoxide
- EDTA:: Ethylenediamine tetraacetic acid
- EGTA:: Ethylene glycol tetraacetic acid
- ESI:: Electrospray ionization
- Et₂O:: Diethylether
- EtOH:: Ethanol
- FA:: Formic acid
- FAAH:: Fatty acid amide hydrolase
- FP:: Fluorophosphonate
- GK:: Glycerol kinase
- GPO:: Glycerol-3-phosphate oxidase
- hDAGL-α:: Human Sn1 specific diacylglycerol lipase-α
- hDAGL-β:: Human Sn1 specific diacylglycerol lipase-β
- HEK293T:: Human embryonic kidney 293 SV40 large T-antigen
- HEMNB:: HEPES, EDTA, MgCl₂, NaCl, BSA
- HEPES:: 4-(2-Hydroxyethyl)-1-piperazineethanesulfonic acid
- HPLC: High performance liquid chromatography
- HRMS:: High resolution mass spectrometry
- HRP:: Horseradish peroxidase
- IPSC:: Inhibitory postsynaptic currents
- LAH:: Lithium aluminium hydride
- LCMS:: Liquid chromatography mass spectrometry
- MAGL:: Monoacylglycerol lipase
- MeOH:: Methanol
- m/z:: Mass over charge ratio
- n-BuLi: n-Butyllithium
- Na-GTP:: Guanosine 5'-triphosphate sodium salt hydrate
- NBS:: *N*-bromosuccinimide
- NMR:: Nuclear magnetic resonanc
- ON:: Over night
- PBS:: Phosphate-buffered saline
- PC:: Prostate cancer
- PNP-butyrate:: Paranitrophenylbutyrate
- ppm: Parts per million
- rt:: Room temperature
- SAG:: 1-Stearoyl-2-arachidonoyl-sn-glycerol
- SD:: Standard deviation
- SDS:: Sodium dodecyl sulfate
- SEM:: Standard error of mean
- TAMRA:: Carboxytetramethylrhodamine
- TFA:: Trifluoroacetic acid
- TFE:: Tetrafluoroethylene
- THF:: Tetrahydrofuran
- THL:: Tetrahydrolipstatin
- TLC:: Thin layer chromatography.

### Example 1: Biochemical hDAGL-α activity assay (96 well plate)

The biochemical hDAGL-α activity assay is based on the hydrolysis of para-nitrophenylbutyrate (PNP-butyrate) by membrane preparations from HEK293T cells transiently transfected with hDAGL-α. 200 µL reactions were performed in flat bottom Greiner 96-wells plates in a 50 mM pH 7.2 Hepes buffer. Membrane protein fractions from HEK293T cells transiently transfected with hDAGL-α (0.05 µg/µL final concentration) were used as hDAGL-α source. Compounds to be tested were introduced in 5 µL DMSO. The mixtures were incubated for 30 minutes before 5.0 µL 12 mM PNP-butyrate (final concentration 0.3 mM) in DMSO was added (final DMSO concentration 5.0%). Kinetics were followed immediately after addition of PNP-butyrate on a plate reader (TECAN GENios^{®} microplate reader), by measuring the OD420 every 60 seconds, for 20 minutes at 37°C. The slope of the linear region from 5-15 minutes was determined, and all experiments were performed at N = 2, n = 2 for experimental measurements and N = 2, n = 4 for controls.

Data analysis: Z'-factor of each plate was determined for the validation of each experiment, using the following formula Z' = 1-3(σ_{pc} + σ_{nc})/(µ_{pc} - µ_{nc}). The slope from 5-15 minutes of the positive control (pc: hDAGL-α, DMSO), and the negative control (nc: Mock, DMSO) was used. Plates were accepted for further analysis when Z' > 0.6. Kinetic measurements were corrected for the average absorption of the negative control (mock, DMSO). The slope of the linear region from 5-15 minutes was determined. The average, standard deviation (SD) and standard error of mean (SEM) were calculated and normalized to the corrected positive control. Data was exported to Graphpad Prism 5.0 for the calculation of the pIC₅₀ using a nonlinear dose-response analysis. The inventive compounds proved to be a potent inhibitor some 10 fold more potent than compound 1 as closest prior art. Results are shown in table 1.

The activity of the inventive compounds on human DAGL-β using the biochemical assay with *para*-nitrophenylbutyrate as a surrogate substrate was also tested (compound 25 showed a pIC₅₀ of 8.1 ± 0.07, n=4).

### Example 2: Activity based protein profiling (ABPP) selectivity assay

Activity-based protein profiling (ABPP) has emerged as a powerful technique for discovering selective enzyme inhibitors acting in their native physiological context. ABPP hinges on the use of activity-based probes (ABPs) to report on enzyme activity in cells, tissue or animals. An ABP normally consists of a covalent, irreversible enzyme inhibitor featuring a reporter entity (fluorophore, biotin, bioorthogonal tag) to label the active site of the enzyme or enzyme family at hand. ABPP is unique in its ability to rapidly identify inhibitor activity and selectivity within large enzyme families in complex proteome samples.

For gel based ABPP experiments mouse brain proteome (2.0 mg/mL, 20 µL) was preincubated for 30 min with vehicle (DMSO) or compound to be tested in 0.5 µL DMSO at rt. And subsequently treated with 500 nM (final concentration) TAMRA-FP (TAMRA-FP was bought at Thermo Fischer) for 15 minutes at rt. The reactions were quenched with 10 µL standard 3 x SDS page sample buffer. The samples were directly loaded and resolved on SDS page gel (10 % acrylamide). The gels were scanned with a ChemiDoc MP sytem (Biorad, Cy 3 settings, 605/50 filter) and analyzed using Image Lab 4.1.

The percentage effect on FAAH was determined by measuring the integrated optical intensity of the band corresponding to FAAH (∼ 63 kD for mouse) using Image lab software. This activity was corrected for the total protein loading per lane as determined by coomassie staining and imaging with a ChemiDoc™ MP sytem (Biorad) followed by determination of the integrated optical intensity per lane by using Image Lab 4.1 software. The intensity of the protein bands from the the samples treated with vehicle was set to 100%.

**Table 1. shows results of examples 1 and 2. The compound is defined to be active if pIC₅₀ > 5.0 for hDAGL-α. In additiuon a compound is selective over FAAH if % Effect (10 µM) on FAAH < 50%. N/A means less than 50% probe displacement at 10 µM inhibitor concentration.**

| **Structure** | | **DAGL-α Activity assay** | | **FAAH ABPP (10 µm)** | | **FAAH Activity assay reported** | |
|---|---|---|---|---|---|---|---|
| | | **pIC₅₀** | **± SEM** | **% Eff.** | **± SEM** | **pKᵢ** | **± SE M** |
| 1 | 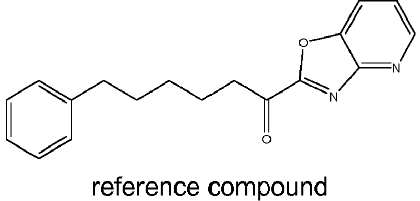 | 7.43 | 0.05 | 99 | 3 | 9.70 | 0.1 |
| 25 | 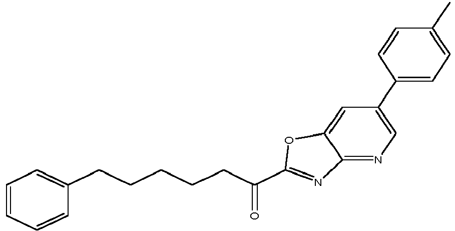 | 8.52 | 0.06 | N/A | - | - | - |
| 26 | 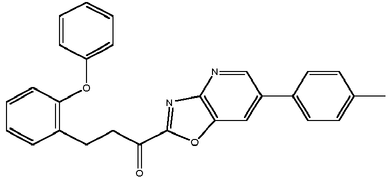 | 8.08 | 0.08 | N/A | - | - | - |
| 27 | 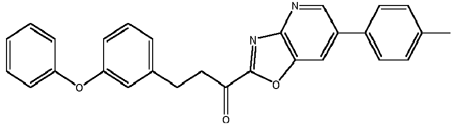 | 8.43 | 0.08 | N/A | - | - | - |
| 28 | 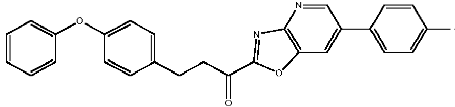 | 8.29 | 0.08 | N/A | - | - | - |
| 29 | 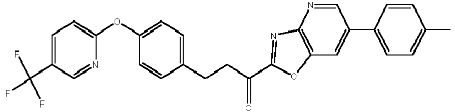 | 9.07 | 0.06 | N/A | - | - | - |
| 30 | 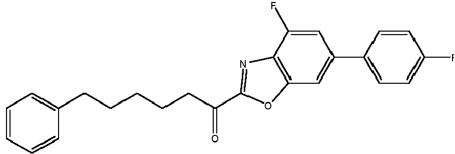 | 8.30 | 0.08 | N/A | - | - | - |
| 31 | 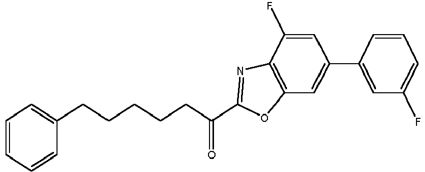 | 8.6 | 0.3 | N/A | - | - | - |
| 32 | 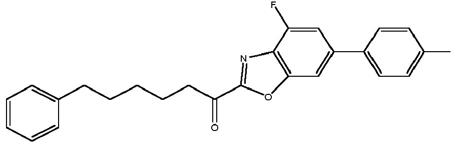 | 8.85 | 0.05 | N/A | - | - | - |
| 33 | 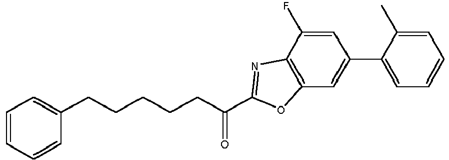 | 8.88 | 0.07 | N/A | - | - | - |
| 34 | 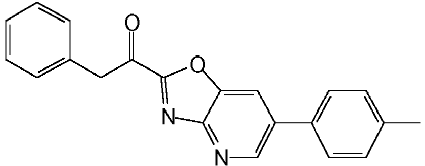 | - | - | N/A | - | - | - |
| 35 | 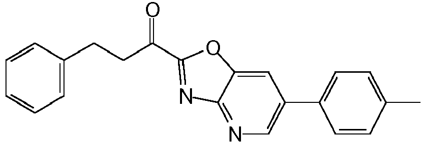 | - | - | N/A | - | - | - |
| 36 | 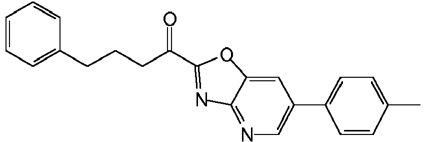 | - | - | N/A | - | - | - |
| 37 | 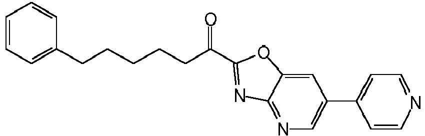 | - | - | N/A | - | - | - |
| 38 | 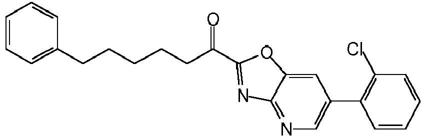 | - | - | N/A | - | - | - |
| 39 | 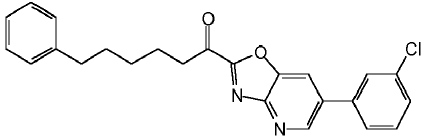 | - | - | N/A | - | - | - |
| 40 | 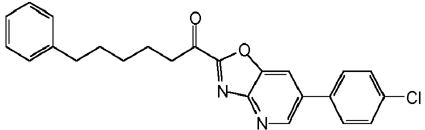 | - | - | N/A | - | - | - |
| 41 | 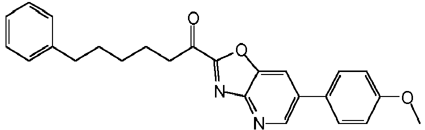 | - | - | N/A | - | - | - |
| 42 | 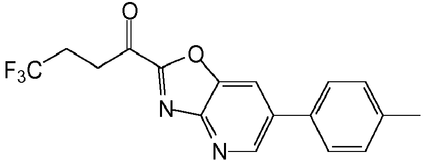 | - | - | N/A | - | - | - |
| 43 | 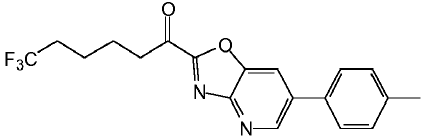 | - | - | N/A | - | - | - |
| 44 | 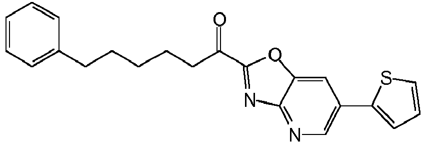 | - | - | N/A | - | - | - |
| 45 | 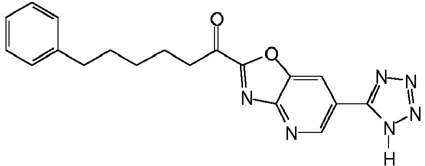 | - | - | N/A | - | - | - |
| 46 | 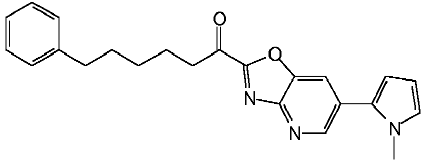 | - | - | N/A | - | - | - |
| 47 | 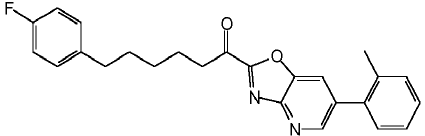 | - | - | N/A | - | - | - |
| 48 | 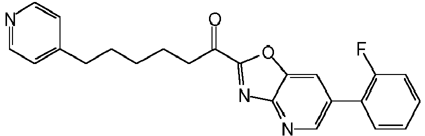 | - | - | N/A | - | - | - |
| 49 | 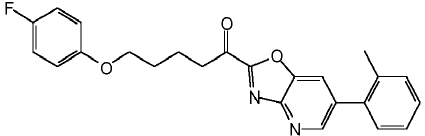 | - | - | N/A | - | - | - |
| 50 | 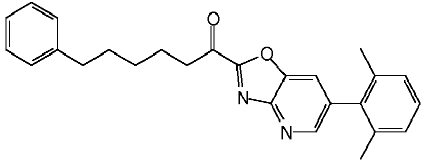 | - | - | N/A | - | - | - |
| 51 | 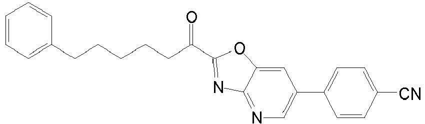 | - | - | N/A | - | - | - |
| 52 | 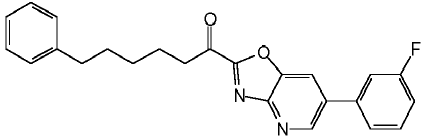 | - | - | N/A | - | - | - |
| 53 | 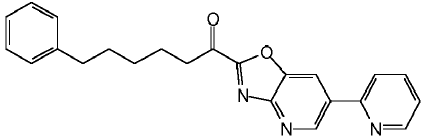 | - | - | N/A | - | - | - |
| 54 | 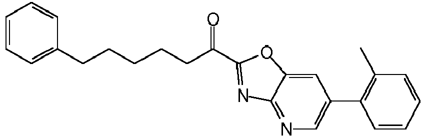 | - | - | N/A | - | - | - |
| 55 | 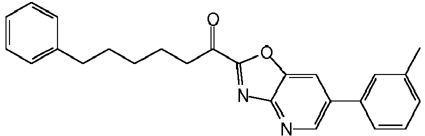 | - | - | N/A | - | - | - |
| 56 | 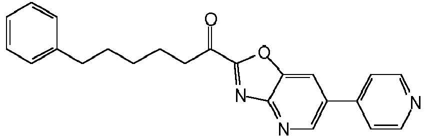 | - | - | N/A | - | - | - |
| 57 | 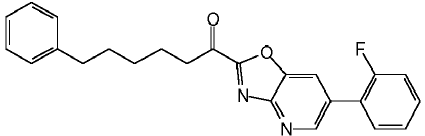 | - | - | N/A | - | - | - |
| 58 | 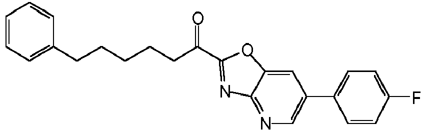 | - | - | N/A | - | - | - |

### Example 3: Further assays to confirm inhibitory action

To confirm that compound 25 was also able to block conversion of the natural substrate 1-stearoyl-2-arachidonoyl-sn-glycerol of DAGL-α to the endocannabinoid 2-AG, the inventors employed a real-time, fluorescence-based assay. The DAGL-α natural substrate assay is based on the production of 2-AG from 1-stearoyl-2-arachidonoyl-sn-glycerol (SAG) hydrolysis by DAGL-α-overexpressing membrane preparations from transiently transfected HEK293T cells. The 2-AG production is coupled to the oxidation of commercially available Amplifu™Red via a multi-enzyme cascade, resulting in a fluorescent signal from the dye resorufin.

Standard assays were performed in HEMNB buffer (50 mM HEPES pH 7.4, 1 mM EDTA, 5 mM MgCl₂, 100 mM NaCl, 0.5% (w/v) BSA) in black, flat clear-bottom 96-wells plates (Greiner). Final protein concentration of membrane preparations from DAGL-α-overexpressing HEK293T cells was 50 µg/mL (10 µg per well). Inhibitors were added from 40x concentrated DMSO stocks. A substrate solution of 1-stearoyl-2-arachidonoyl-sn-glycerol was prepared just prior to use. The SAG stock solution (10 mg/mL in methyl acetate) was dried under argon and subsequently dissolved in 50 mM HEPES buffer (pH 7.0) containing 0.75% (w/v) Triton X-100. The substrate solution was mixed to form an emulsion and stored on ice until use.

DAGL-α-overexpressing membranes were incubated with inhibitor for 20 min. Subsequently, assay mix containing glycerol kinase (GK), glycerol-3-phosphate oxidase (GPO), horseradish peroxidase (HRP), adenosine triphosphate (ATP), monoacylglycerol lipase (MAGL), Amplifu™Red and 1-stearoyl-2-arachidonoyl-sn-glycerol (SAG) was added and fluorescence was measured in 5 min intervals for 60 min on a GENios microplate reader (Tecan, Giessen, The Netherlands).

Standard assay concentrations: 0.2 U/mL GK, GPO and HRP, 0.125 mM ATP, 5 µg/mL MAGL-overexpressing membranes, 10 µM Amplifu™Red, 100 or 150 µM SAG, 5% DMSO, 0.0075% (w/v) Triton X-100 in a total volume of 200 µL.

In this assay compound 25 inhibited recombinant human DAGL-α with a pIC₅₀ of 7.9 ± 0.08 (n=4) (Figure 1 E), which is a 40-fold increase compared to compound 1 (pIC₅₀ 6.3 ± 0.1 (n = 4)).

### Example 4: Determining endogenous DAGL activity using MB064 as ABP

To test its activity on endogenously expressed DAGL-α in mouse membrane proteome, an ABPP assay with MB064, a specific β-lactone-containing probe, which was tailor-made for the detection of DAGL-α activity, was used. An alkyne group was introduced in the prototypical DAGL-inhibitor tetrahydrolipstatin (THL) and conjugated this covalent, irreversible inhibitor via copper-catalyzed click chemistry to a BODIPY-acid as a fluorencent reporter group to yield tailor-made probe MB064. Compound 25 prevented DAGL-α labeling in the mouse brain membrane proteome by MB064 with a pIC₅₀ of 7.5 ± 0.07 (n=3) (Figure 1C, D).

In view of the high homology between DAGL-α and DAGL-β the inventors assesed the activity of compound 25 also on native DAGL-β. To this end, it was tested whether the ABP probe MB064 was also able to label DAGL-β. MB064 was incubated with membranes from mock and hDAGL-β transfected HEK293 cells. MB064 was able to label a protein at the expected molecular weight of DAGL-β, which was not present in the control membranes, or in S443A-hDAGL-β transfected cells, in which the catalytic serine is replaced by alanine using site-directed mutagenesis (Figure 1 F). Thus, the tailor-made probe can also detect DAGL-β. Labeling of hDAGL-β was inhibited by compound 25 with a pIC₅₀ of 7.4 ± 0.07 (n=3). Native DAGL-β activity in the spleen membrane proteome could also be inhibited by compound 25 (10 µM) (Figure 1 G).

### Example 5: Determining mode of action

To determine the mode of action (reversible versus irreversible) of the inventive compounds, an experiment in which human DAGL-α membranes were pre-incubated at the IC₈₀ concentration of compound 25 or the irreversible DAGL inhibitor KT-109 was performed. The protein was separated from small molecule inhibitors via size exclusion column chromatography and remaining enzyme activity was visualized with MB064. No recovery of DAGL-α activity was found for KT-109, whereas MB064 labeled DAGL-α exposed to compound 25 with similar intensity to DMSO treated DAGL-α (Figure 1 H). This indicates that the inventive compounds are reversible inhibitor for DAGL-α.

### Example 6: Determining proteome-wide selectivity of compound 25 using comparative and competitive chemoproteomics

To investigate the selectivity of compound 25 in the mouse brain proteome comparative and competitive ABPP was used with a broad-spectrum FP-based probe (TAMRA-FP) and the tailored DAGL-α ABP MB064. As a reference the widely used β-lactone-based DAGL-inhibitors THL and OMDM-188, two broad-spectrum covalent and irreversible serine hydrolase inhibitors, were firstly tested. In this experimental setup, both compounds blocked labeling of at least 4 serine hydrolases at concentrations as low as 1 µM, including ABHD6 and ABHD12, enzymes involved in 2-AG metabolism in the brain (Figure 2A,B).

Fatty acid amide hydrolase (FAAH) was identified in a previous study as a major off-target for comparative compound 1. In stark contrast to comparative compound 1, which inhibited FAAH labeling with a pIC₅₀ of 7.8 ± 0.04 (n=3) (Figure 2D), the inventive compound 25 did not block FAAH labeling up to a concentration of 10 µM (n=3) (Figure 2A, D). Of note, compound 25 did not inhibit labeling of any other band as well, and the inventors conclude that it is highly selective at least within the panel of serine hydrolases targeted by the activity-based probes. To explain this remarkable selectivity the inventors replaced the α-ketoheterocyclec part of the α-ketoheterocycle based FAAH inhibitor OL-135 in a previously published co-crystal structure of FAAH (pdb: 2WJ1 and 2WJ2) with the α-ketoheterocyclic part of compound 25. This revealed a steric clash of the R² moiety of the inventive compound with the substrate channel of FAAH, thereby possibly explaining the high selectivity (Figure 2C).

Since, the human serine hydrolase family contains approximately 200 family members, the selectivity in brain proteome was examined in a broader and more detailed manner. To this end a semi-quantitative chemoproteomics protocol was adapted, which was previously applied to determine the selectivity profile of the irreversible inhibitor KT-109, using a biotinylated version of MB064 and the FP-probe.

Mouse brain proteome (500 µL, 2.0 mg/mL) membrane or soluble fraction was incubated with vehicle (DMSO) or inhibitor (10 µM) in DMSO for 30 minutes at rt. The proteome was labeled with MB108 (2.5 µM, 30 minutes, rt) or FP-Biotin (5 µM, 30 minutes, 37 °C). Subsequently the labeling reaction was quenched and excess probe was removed by chloroform methanol precipitation. Precipitated proteome was suspended in 500 µL 6M Urea/25 mM ammonium bicarbonate and allowed to incubate for 15 minutes. 5 µL (1 M DTT) was added and the mixture was heated to 65 °C for 15 minutes. The sample was allowed to cool to rt before 40 µL (0.5 M) iodoacetamide was added and the sample was alkylated for 30 minutes in the dark. 140 µL 10% (wt/vol) SDS was added and the proteome was heated for 5 minutes at 65 °C. The sample was diluted with 6 mL PBS. 100 µL of 50% slurry of Avidin-Agarose from egg white (Sigma-Aldrich) was washed with PBS and added to the proteome sample. The beads were incubated with the proteome > 2h.

The beads were isolated by centrifugation and washed with 0.5% (wt/vol) SDS and PBS (3x). The proteins were digested overnight with sequencing grade trypsin (promega) in 100 µL Pd buffer (100 mM Tris, 100 mM NaCl, 1 mM CaCl₂, 2 % acetonitrile (ACN) and 500 ng trypsin) at 37 °C with vigorous shaking. The pH was adjusted with formic acid to pH 3 and the beads were removed. The peptides were isotopically labeled by on stage tip dimethyl labeling.

### On-stage tip dimethyl labeling

The stage tips were made by inserting C₁₈ material in a 200 µL pipet. The stepwise procedure given in the table below was followed for stage tip desalting and dimethyl labeling. The solutions were eluted by centrifugal force and the constitutions of the reagents are given below.

**Table 2: shows conditions used for isotopically labelling of peptides**

| **Step** | **Solution** | **Centrifugation speed** |
|---|---|---|
| **Conditioning** | Methanol (50 µL) | 2 min 600g |
| **Conditioning** | Stage tip solution B (50 µL) | 2 min 600g |
| **Conditioning** | Stage tip solution A (50 µL) | 2 min 600g |
| **Loading** | Load samples on stage tips | 2.5 min 800g |
| **Washing** | Stage tip solution A (100 µL) | 2.5 min 800g |
| **Dimethyl labeling** | Load 20 µL L or M reagents on stage tip (5X) | 5 min 400g |
| **Washing** | Stage tip solution A (100 µL) | 2.5 min 800g |
| **Elution** | Stage tip solution B (100 µL) | 2.5 min 800g |

**Stage tip solution A:** Stage tip solution A is 0.5% (vol/vol) formic acid (FA) in H₂O. (Freshly prepared solution)
**Stage tip solution B:** Stage tip solution B is 0.5% (vol/vol) FA in 80% (vol/vol) ACN/H₂O. (Freshly prepared solution)

**Table 3: shows Dimethyl labeling reagents used**

| **Light labeling reagent** | **Final concentration** | **Volume** |
|---|---|---|
| **Phosphate buffer (50 mM; pH 7.5)** | | 900 µL |
| **CH₂O (light)** | | 50 µL |
| **NaBH₃CN (0.6 M)** | 0.03 M | 50 µL |
| | | |

| **Medium labeling reagent** | **Final concentration** | **Volume** |
|---|---|---|
| **Phosphate buffer (50 mM; pH 7.5)** | | 900 µL |
| **CD₂O (Medium)** | | 50 µL |
| **NaBH₃CN (0.6 M)** | 0.03 M | 50 µL |

After the final elution step, the desired heavy and light samples were combined and concentrated in a speedvac to remove the ACN. The residue was reconstituted in 95:3:0.1 H₂O/ACN/FA (vol/vol) before LC/MS analysis.

Tryptic peptides were analyzed on a Surveyor nanoLC system (Thermo) hyphenated to a LTQ-Orbitrap mass spectrometer (Thermo). Gold and carbon coated emitters (OD/ID = 360/25 mm tip ID = 5 µm), trap column (OD/ID = 360/ 100 µm packed with 25 mm robust Poros10R2/ 15 mm BioSphere C18 5 µm 120 A) and analytical columns (OD/ID = 360/75 µm packed with 20 cm BioSphere C18 5 mm 120 A) were from Nanoseparations (Nieuwkoop, The Netherlands). The mobile phases (A: 0.1 % FA/H₂O, B: 0.1% FA/ACN) were made with ULC/MS grade solvents (Biosolve). The emitter tip was coupled end-to-end with the analytical column via a 15 mm long TFE Teflon tubing sleeve (OD/ID 0.3 3 1.58 mm, Supelco, USA) and installed in a stainless steel holder mounted in a nano-source base (Upchurch scientific, Idex, USA). General mass spectrometric conditions were as follows: an electrospray voltage of 1.8 kV was applied to the emitter, no sheath and auxiliary gas flow, ion transfer tube temperature 150 °C, capillary voltage 41 V, tube lens voltage 150 V. Internal mass calibration was performed with air-borne protonated polydimethylcyclosiloxane (m/z = 445.12002) and the plasticizer protonated dioctyl phthalate ions (m/z = 391.28429) as lock mass. For shotgun proteomics analysis, 10 µl of the samples was pressure loaded on the trap column with a 10 µl/min flow for 5 min followed by peptide separation with a gradient of 35 min 5% - 30% B, 15 min 30% - 60% B, 5 min A at a flow of 300 ml/min split to 250 nl/min by the LTQ divert valve. For each data-dependent cycle, one full MS scan (300-2000 m/z) acquired at high mass resolution (60,000 at 400 m/z, AGC target 1 x 106, maximum injection time 1000 ms) in the Orbitrap was followed by three MS/MS fragmentations in the LTQ linear ion trap (AGC target 5 x 103, maximum injection time 120 ms) from the three most abundant ions. MS/MS settings were as follows: collision gas pressure 1.3 mT, normalized collision energy 35%, ion selection threshold of 500 counts, activation q = 0.25 and activation S6 time of 30 ms. Fragmented precursor ions that were measured twice within 10 s were dynamically excluded for 60 s and ions with z < 2 or unassigned were not analyzed. Subsequent data analysis was performed using maxquant software. Acetylation (protein N term) and Oxidation (M) were set as variable modifications. The false discovery rate was set at 1% and the peptides were screened against mouse proteome (Uniprot). Serine hydrolases that were identified in at least two repetitive experiments and for which at least 2 unique peptides were identified were considered as valid quantifiable hits.

This methodology allows for a more accurate quantification avoiding band overlap (as observed with gel-based assay) and allows screening over a broader range of specified serine hydrolases. Equal isotopic labeling and detection of light and medium peptides from proteins targeted by both ABPs (Figure 2G) were validated. Next, to investigate the selectivity of inventive compound 25 in this chemoproteomic assay was set out. Compound 25 (10 µM, 30 min) was able to reduce DAGL-α labeling in mouse membrane proteome by more than 70%, but not of any other serine hydrolase identified (Figure 2E). In addition, the inventors tested compound 25 in biochemical assays using recombinant human MAGL and in a cannabinoid CB₁ receptor radioligand displacement assay. A significant interaction (pKᵢ < 5) with these proteins of the endocannabinoid system could not be found. The CB1 displacement assays were performed as described below.

### Cell culture

CHOK1hCB₁_bgal cells (CHOK1 cell line expressing human cannabinoid receptor 1) were cultured in Ham's F12 Nutrient Mixture supplemented with 10% fetal calf serum, 1 mM glutamine, 50 U/mL penicillin, 50 µg/ml streptomycin, 300 mg/mL hygromycin and 800 µg/mL geneticin in a humidified atmosphere at 37°C and 5% CO₂. Cells were subcultured twice a week at a ratio of 1:20 on 10-cm ø plates by trypsinization. For membrane preparation the cells were subcultured 1:10 and transferred to large 15-cm diameter plates.

### Membrane preparation

The cells on thirty 15-cm ø plates were detached from the bottom by scraping them into 5 mL phosphate-buffered saline (PBS), collected in 12 mL Falcon tubes and centrifuged for 5 minutes at 200 x *g* (3,000 rpm). The pellets were resuspended in ice-cold 50 mM Tris-HCl buffer and 5 mM MgCl₂ (pH 7.4). An Ultra Thurrax homogenizer (Heidolph Instrucments, Schwabach, Germany) was used to homogenize the cell suspension. The membranes and the cytosolic fractions were separated by centrifugation at 100,000 x *g* (31,000 rpm) in a Beckman Optima LE-80 K ultracentrifuge (Beckman Coulter Inc., Fullerton, CA) at 4°C for 20 minutes. The pellet was resuspended in 10 mL of Tris-HCl buffer and 5 mM MgCl₂ (pH 7.4) and the homogenization and centrifugation steps were repeated. Finally, the membrane pellet was resuspended in 10 mL 50 mM Tris-HCL buffer and 5 mM MgCl₂ (pH 7.4) and aliquots of 250 µL were stored at -80°C. Membrane protein concentrations were measured using the BCA method.

### [³H]CP55940 Displacement Assays

[³H]CP55940 displacement assays were used for high throughput screening of cold ligands and the determination of their affinity (Kᵢ) and IC₅₀ values. Membrane aliquots containing 5 µg (CHOK1hCB₁_bgal) of membrane protein were incubated in a total volume of 100 µL of assay buffer (50 mM Tris-HCl buffer (pH 7.4), 5 mM MgCl₂ and 0.1% BSA) at 30 °C for 1 hour, in presence of 3.5 nM [³H]CP55940 (CHOK1hCB₁_bgal). Different concentrations of competing ligand were used for determination of affinity (Kᵢ) and IC₅₀ values. Nonspecific binding was determined in the presence of 10 µM AM630 (6-lodopravadoline; a selective inverse agonist for the cannabinoid receptor CB2). Incubations were terminated by rapid vacuum filtration to separate the bound and free radioligand through Whatman GF/C filters (Whatman International, Maidstone, UK), precoated with 0.25% (v/v) polyethylenimine, using a Brandel Harvester (Brandel, Gaithersburg, MD). Filters were subsequently washed three times with ice-cold assay buffer and 3.5 mL of the Emulsifier Safe scintillation fluid (Perkin Elmer, Groningen, The Netherlands) was added to each filter. After 2 hours, the filter-bound radioactivity was determined by scintillation spectrometry using a Tri-Carb 2900 TR liquid scintillation counter (Perkin Elmer, Boston, MA).

MAGL assay was performed as described below. Standard assays were performed under similar conditions as for the glycerol detection besed DAGL-α activity assay , but at a final protein concentration of 1.5 µg/mL (0.3 µg MAGL-overexpressing membranes per well) and with 2-arachidonoylglycerol (2-AG) as the substrate. 2-AG was directly added from a stock solution in acetonitrile and no Triton X-100 was added. Fluorescence was measured in 5 min intervals for 60 min. Final assay concentrations: 0.2 U/mL GK, GPO and HRP, 0.125 mM ATP, 10 µM Amplifu™Red, 25 µM 2-AG, 5% DMSO, 0.5% ACN in a total volume of 200 µL.

Together these results indicate that the inventive compounds are highly selective DAGL-inhibitor.

### Example 7: Reduction of 2-AG levels in cells

To test the cellular activity of inventive compound 25, the inventor used Neuro2A cells, which is a mouse neuroblastoma cell line known to express both DAGL-α and DAGL-β. In a targeted lipidomics experiment the effect of inventive compound 25 on the cellular levels of 2-AG and anandamide was determined.

### Lipid analysis Neuro2A cells.

Neuro2A cells were cultured as described above. Before inhibitor treatment, the culture medium was removed and the cells were washed with warm (37 °C) serum free medium (3x). The cells were treated with vehicle (DMSO) or inhibitor for 1h at 37 °C in serum free medium. After 1h of incubation medium was removed and the cells were washed with cold (4°C) PBS (3x) and subsequently suspended in PBS and pelleted. The PBS was removed and the cell pellet was flash frozen and and stored at -80 ° until endocannabinoid extraction.

Cell pellets were suspended in 100 µL 0.5% NaCl (aq.), and 510 µL 0.2% formic acid (vol/vol) in ACN containing internal standards (d₈-anandamide (6 pmol) and d₈-2-arachidonoyl glycerol (60 pmol)). The mixture was vortexed and loaded on a captiva ND lipid column (A5300635, Agilent technologies). Positive pressure was applied and the flow trough was collected. The elution step was subsequently repeated with 500 µL and 250 µL 0.2% formic acid (vol/vol) in ACN/H₂O 95:5 (vol/vol). The combined elutes were concentrated in a speedvac and reconstituted in H₂O/MeOH 1:1 (vol/vol) for LC/MS analysis.

### LC-Chip-MS/MS analysis

Endocannabinoids were measured using a chip-based nano liquid chromatography (LC) system coupled to a triple quadrupole mass spectrometer. The HPLC-Chip is inserted into the HPLC-Chip-MS interface, which mounts directly on the MS source. It includes a miniature camera for spray visualization, the loading mechanism for chip positioning, the microvalve for flow switching, and fluid connection ports for the nanoLC and microwell- plate autosampler.

Chromatographic conditions were achieved on a 1100 series LC-chip system (Agilent technologies, Walbron, Germany) consisting of a nanoflow pump, a capillary pump, a micro well plate auto sampler with thermostat and a LC-Chip/MS interface (chipcube). The chromatographic separations were performed on an ultra high capacity chip including a 360 nL enrichment column and a 150 mm x 75 µm analytical column, both packed with a Polaris-HR-chip 3µm C18 phase (Agilent Technologies). The mobile phase was composed of 10mM formic acid/Water (A) and ACN (B). The analytical gradient was performed in two steps: first, the 8ul sample was loaded on the enrichment column during an isocratic enrichment phase using the capillary pump delivering a mobile phase in isocratic mode composed of 40%B at a flow rate of 2 µL/min. The column was flushed with two wash cycles for 8min at 100%B to remove unretained components. Then, after the valve switches, a gradient starts at 45%B that linearly increases up to 80% B in 12min at a flow rate of 400nL/min. The column was then rinsed with 95%B for 2 minutes before returning to 45% B. The column was reequilibrated for 5min prior to the next injection. The total analysis time was 20min for each run. During the analysis, the needle was washed with ACN/H2O (1/1, v/v) commanded by an injection program.

A 6440 Triple quadrupole equipped with a nonoESI source operating in positive mode (Agilent technologies, Walborn, Germany) was used for MS detection. Capillary voltage was set to 1800V. The drying gas was set at a flow rate of 4L/min and the source temperature was maintained at 365C. Quantification was obtained using static MRM (multiple reaction monitoring) mode of the transistions at m/z 379.3 → 287.2 for 2AG, 348.3 → 62.2 for AEA and 387.3 → 294.2 for 2AGd8, 356.3 → 62.2 for AEAd8 (internal standards). Mass hunter workstation (Agilent technologies) was used for instrument control. Raw MS data were processes using mass hunter quantitative analysis work station (Agilent technologies). AEA and 2AG was quantified by using a matrix matched internal standard calibration curve. A concentration-dependent reduction of 2-AG was found in Neuro2A cells, whereas anandamide levels were unaffected (Figure 3C, D).

To test the activity of inventive compound 25 in a human cell line, human PC3 cells (prostate cancer cell line) were used. The protocol for lipodomics in PC3 cells is given below. Before inhibitor treatment, the culture medium was removed and the cells were washed with warm (37 °C) serum free medium (3x). The cells were treated with vehicle (DMSO) or inhibitor for 4h at 37 °C in serum free medium. After 4h of incubation medium was removed and the cells were washed with cold (4°C) DPBS (3x) and subsequently suspended in DPBS and pelleted. The cell pellet metabolome was extracted and by MeOH/CHCl₃ extraction and analysed by LCMS/MS of a triple quad mass spectometer (agilent). The concentration of the lipids was determined by comparison with spiked deuterated standards of the lipd species. Inventive compound 25 reduced 2-AG levels, but no change in substrate levels of DAGL was detected, which may suggest that DAG species are rapidly converted into other membrane constituents, such as phosphatidic acid by DAG-kinases. Interestingly, arachidonic acid levels were reduced by inventive compound 25 treatment of PC3 cells. This may indicate that downstream metabolic pathways of 2-AG signaling are also affected and that the biosynthesis of 2-AG is the rate-limiting step. This is in line with a previously reported reduction of arachidonic acid levels in DAGL-α knock-out mice and by KT-109 in mouse liver.

### Example 8: Reduction of synaptic transmission in hippocampus

To demonstrate a physiological effect of the inhibition of 2-AG biosynthesis, the inventors focused their attention to the hippocampal slice preparation in which depolarization-induced suppression of inhibition (DSI) is thought to be mediated via 2-AG-induced activation of presynaptic cannabinoid CB₁ receptors. Experimental evidence, which supports a role of DAGL-α in short-term synaptic plasticity, has been obtained through the use of genetically engineered mice that constitutively lack DAGL-α. DSI was absent in these DAGL-α knock-out mice, but not in their DAGL-β knock-out counter parts.

The cannabinoid CB₁ receptor-dependent synaptic plasticity was investigated with the inventive compound being highly selective, reversible DAGL inhibitor. Horizontal slices (300 µm) of the hippocampus were obtained from male C57BL/6 mice (Harlan, the Netherlands) aged 14-19 days postnatal. Experiments were approved by the animal welfare committee of the University of Amsterdam. Animals were killed by decapitation, their brains quickly removed and placed in oxygenated (95% O₂ - 5% CO₂) ice cold (4 °C) adapted artificial cerebrospinal fluid (aACSF), containing in mM: 120 choline chloride, 3.5 KCI, 0.5 CaCl₂, 6 MgSO₄, 1.25 NaH₂PO₄, 25 D-glucose, 25 NaHCO₃). Slices were cut in aACSF on a vibratome (VT1200S, Leica, Germany) and placed for 30 min in regular ACSF (containing in mM: 120 NaCl, 3.5 KCI, 25 NaHCO₃, 25 D-glucose, 2.5 CaCl₂, 1.3 MgSO₄, 1.25 NaH₂PO₄) at 32 °C. Prior to recording, slices were kept at room temperature for at least one hour. Whole-cell voltage clamp recordings were made at 32 °C from the soma of CA1 pyramidal neurons (holding potential V_{H} = - 80 mV). Recording pipettes were pulled from borosilicate glass (Science Products, Germany) and had a resistance of 2-3 MΩ when filled with pipette solution, containing in mM: 110 KGluconate, 30 KCI, 0.5 EGTA, 10 4-(2-hydroxyethyl)-1-piperazineethanesulfonic acid (HEPES), 4 Mg-ATP, 0.5 Na-GTP. Inhibitory postsynaptic currents (IPSCs) were evoked with a glass stimulus electrode (filled with ACSF) positioned in the stratum radiatum, close to the recorded neuron. With 200-µs biphasic stimulus pulses of half-maximal intensity the IPSCs were evoked every 5 sec. 20 µM 6-cyano-7-nitroquinoxaline-2,3-dione (CNQX) and 10 µM (2*R*)-amino-5-phosphonovaleric acid (AP5) were added to the ACSF to block glutamatergic synaptic currents and 5 µM carbachol to enhance inhibitory input. Recordings were made using an Axopatch 200B (Molecular Devices, USA) and in-house software running under Matlab (MathWorks, USA). Signals were low-pass filtered at 5 kHz and sampled at 10 kHz. Series resistance ranged from 5-15 MΩ and was compensated to ∼65%. Voltage signals were corrected for liquid junction potential.

Stock solutions (10 mM) of LEI105 were made in dimethylsulfoxide (DMSO), which was diluted to the final concentration (10 µM) in ACSF. Recordings were made from CA1 neurons in slices that were pre-incubated with 10 µM LEI105 for at least 30 min; during the experiment this concentration was continuously present. Control experiments were performed on neurons recorded in slices pre-incubated with and in the continuous presence of 0.1 % DMSO.

For each CA1 neuron IPSCs were evoked with 5 sec intervals during a 2-min baseline period, prior to the stimulus evoking depolarization-induced suppression of inhibition (DSI). The maximum amplitude of each IPSC was determined and the mean value of the IPCS amplitudes over this period was calculated and used to normalize the evoked synaptic response. DSI was induced by depolarizing the neuron from -80 to 0 mV for 5 sec. The normalized IPSC current amplitudes as a function of time were averaged and used for comparison between both treatments (Fig. 4B; control, n=18 neurons; LEI105, n=15 neurons). In control conditions the DSI protocol induced a short-lasting reduction of the IPSC amplitude, which returned to baseline level with a single exponential time course. This recovery phase of the DSI response was fitted with a single exponential function and the time constant value obtained from this fit (τ = 34 sec) also correctly fitted the decay measured in the neurons treated with LEI105. DSI was then quantified as the relative reduction in the mean IPSC amplitude during the first 15 sec after DSI induction (Fig. 4C). DSI amplitudes were compared with Student's t-test, using p<0.05 to indicate a significant difference.

A clear suppression of DSI by 10 µM bath application of compound 25 (LEI105) was found in the mouse hippocampus (Figure 4). Of note, at 10 µM inventive compound 25 was still selective in the experimental set up and did not displace ³H-CP55940 from the CB₁ receptor, a result that excludes direct antagonism by inventive compound 25 of the CB₁ receptor.

### Syntheses of compounds

The compounds of the invention can be synthesized according to the general scheme 1, as previously published (Baggelaar, M. P. et al. Angew. Chem. Int. Ed. 2013, 52, 12081-12085.). Alcohol **A** can be oxidized (i) and subsequently nitrilated (ii) to cyanohydrin **B.** Upon Pinner reaction (iii), the corresponding immidate (Pinner salt) can be cyclized to the required oxazole containing heterocycle using aminoalcohol C (iv). Lastly DMP oxidation (v) provides the active α-keto heterocycle D.

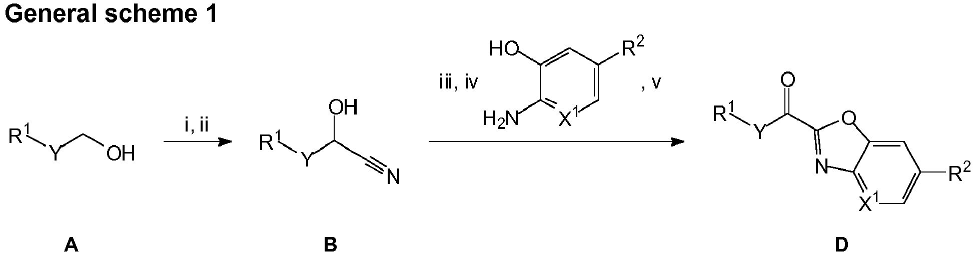

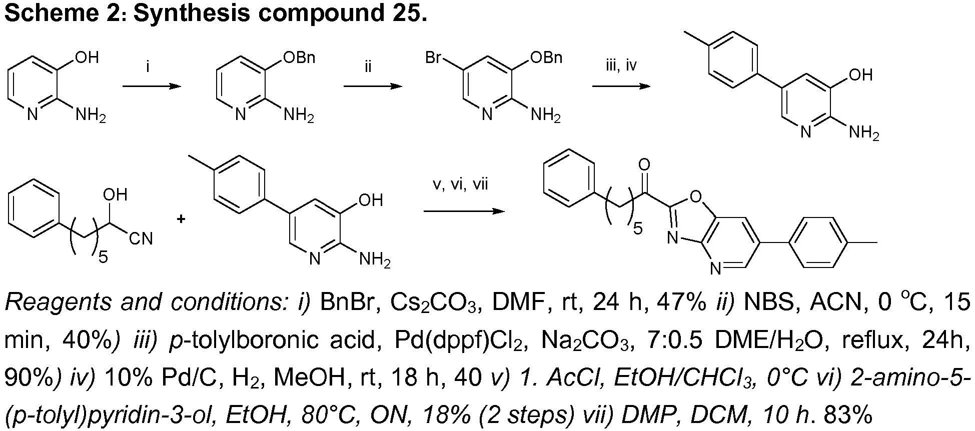

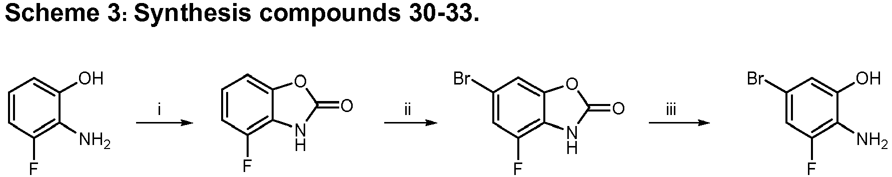

**General procedure for the synthesis of 30 - 33:** Pd(PPh₃)₄ and CsF in H₂O (15 M) was added to a solution of 1-(6-bromo-4-fluorobenzo-2-oxazolyl)-6-phenylhexan-1-one and arylboronic acid in DME (0.02 M). The reaction mixture was brought to reflux and stirred under argon atmosphere until completion as indicated on TLC. The reaction mixture was cooled to rt and diluted with Et₂O. The organic layer was washed with H₂O after which the water layer was extracted with Et₂O (2x). The combined organic layers were washed with brine, dried over anhydrous MgSO₄, filtered and concentrated under reduced pressure. The resulting residue was purified by silica gel column chromatography.

*Reagents and conditions: i) CDI, THF, reflux, 2 h, 100%; ii) NBS, MeCN, rt,* 24 *h, 96%; iii) 3 M NaOH, reflux,* 2 *h, 97%.*

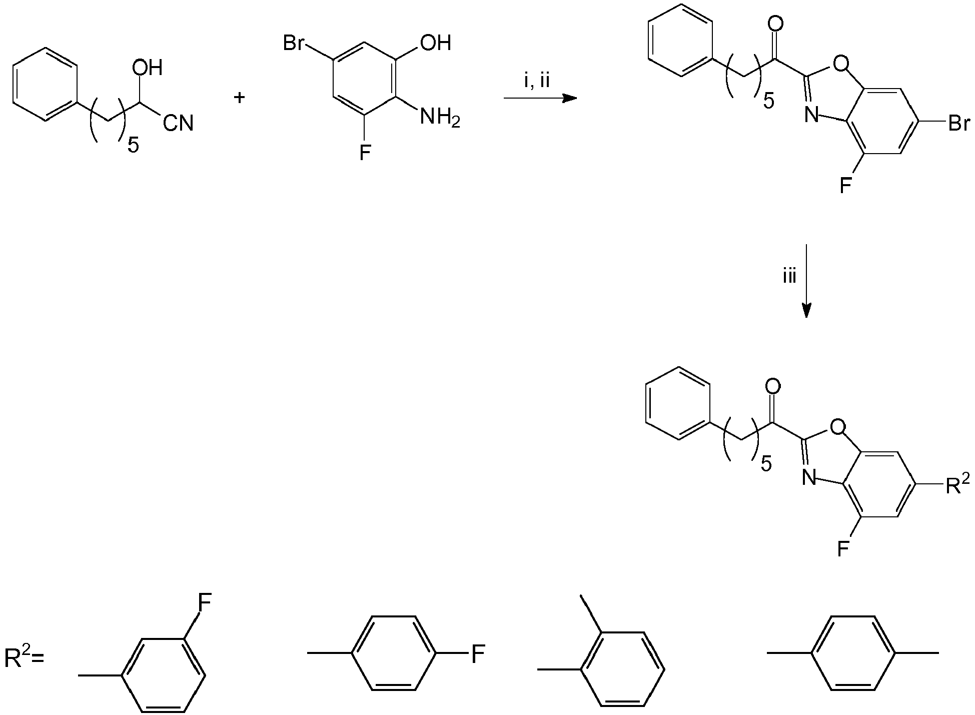

*Reagents and conditions: i) 1: AcCl, 1:1 CHCl₃*/*dry EtOH, 0 °C, 2.5 h, 2: 2-Amino-5-bromo-3-fluorophenol, dry EtOH, reflux, 16 h, 40% (2 steps); ii) DMP, dry DCM, rt, 1.5 h, 84%. iii) Corresponding boronic acid, Pd(PPh₃)₄, CsF, DME, H₂O, 85 °C*, *29-84%.*

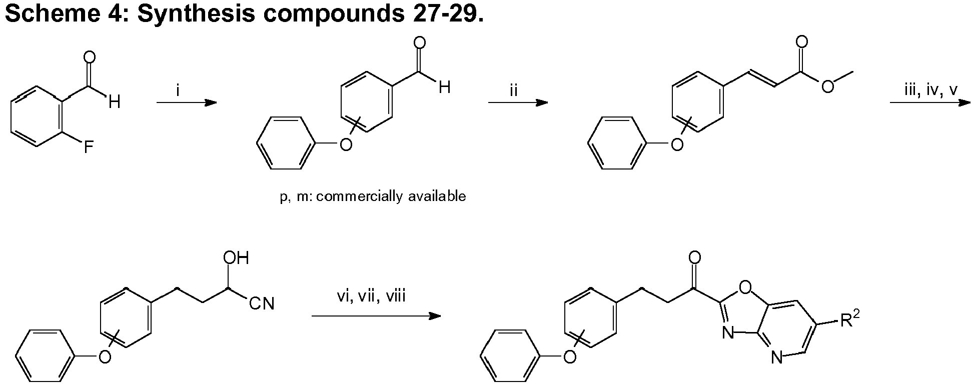

*Reagents and conditions:i) Phenol, K₂CO₃, reflux, THF, then 2-fluorobenzaldehyde, reflux 12 h. 50% (ortho) ii) Methyl 2-(diethoxyphosphoryl)acetate, n-BuLi, THF, -78°C, then phenoxybenzaldehyde. -78°C to rt, 10 h. 68-100% iii) LAH, THF, 0°C, 2h. 22-98% iv) DMP, DCM, rt, 4 h. v) KCN, THF*/*H₂O, rt, 12 h. 51-70% (2 steps) vi) 1. AcCl, EtOH*/*CHCl₃, 0°C vii) corresponding aminohydroxypyridine, EtOH, 80°C, ON. 16-21% (2 steps) viii) DMP, DCM, rt, 10 h. 50-61%*

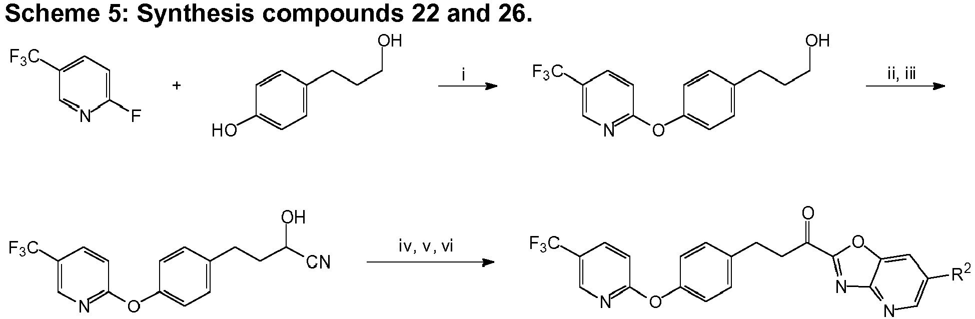

*Reagents and conditions: i) K₂CO₃, reflux, THF, 24 h. 84% ii) DMP, DCM, rt, 4 h. 76% iii) KCN, THF*/*H₂O, rt, 10 h. 64% iv) 1. AcCl, EtOH*/*CHCl₃, 0°C v) corresponding aminohydroxypyridine, EtOH, 80°C, ON.,* 22% *(R² = p-tolyl, 2 steps) vi) DMP, DCM, rt, 3-5 h.* 78% *(R²* = *p-tolyl).*

### Synthesis of reference compound 1

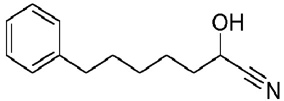

### 2-Hydroxy-7-phenylheptanenitrile

The title compound was synthesized from commercially available 6-phenylhexan-1-ol (1.70 g, 9.51 mmol) to yield 2-hydroxy-7-phenylheptanenitrile (1.67 g, 8.22 mmol, 86% over 2 steps) using previously reported procedures. Spectroscopic data are in agreement with those previously reported.¹

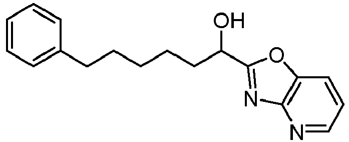

### 1-(Oxazolo[4,5-b]pyridin-2-yl)-6-phenylhexan-1-ol (1a)

AcCl (1.4 mL, 19 mmol) was added dropwise to a dry round bottom flask containing a solution of dry EtOH (1.4 mL, 17 mmol) and dry CHCl₃ (1.4 mL) at 0°C under argon. The reaction mixture was stirred for 30 minutes after which a solution of 2-hydroxy-7-phenylheptanenitrile (117 mg, 0.58 mmol) in dried CHCl₃ (1.0 mL) was added dropwise at 0°C under argon. The reaction mixture was stirred for 2 h, slowly warmed up to rt and concentrated at 25°C *in vacuo.* The crude mixture was coevaporated with toluene (3x5 mL) untill the white solid immidate was obtained. The solid was dissolved in dry EtOH (1.0 mL) and was added under argon to a sealed and dried microwave tube containing a prestirred solution (80°C for 30 minutes, then to rt) of commercially available 2-amino-3-hydroxypyridine (68.8 mg, 0.63 mmol) with pyridine (50 µL, 0.63 mmol) in dry EtOH (4.0 mL). The reaction mixture was heated to reflux (80°C) for 8 h. The reaction mixture was concentrated *in vacuo* and purified by flash chromatography to yield 1-(oxazolo[4,5-*b*]pyridin-2-yl)-6-phenylhexan-1-ol (34 mg, 0.58 mmol, 20%). Spectroscopic data are in agreement with those previously reported.¹

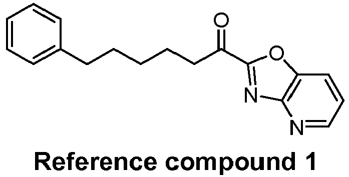

### 1-(Oxazolo[4,5-b]pyridin-2-yl)-6-phenylhexan-1-one (1)

The title compound was synthesized from 1-(oxazolo[4,5-*b*]pyridin-2-yl)-6-phenylhexan-1-ol **(1a)** as previously reported.¹

### Example 9: Synthesis of Compound 25

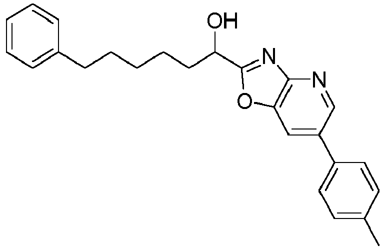

### 1-(6-(p-Tolyl)oxazolo[4,5-b]pyridin-2-yl)-6-phenylhexan-1-ol (25a)

The title compound was synthesized from 2-hydroxy-7-phenylheptanenitrile (**1a**,16 mg, 0.57 mmol) and 2-amino-5-(*p*-tolyl)pyridin-3-ol (77 mg, 0.38 mmol) according to the procedures described for compound **1a.** This yielded 1-(6-(p-tolyl)oxazolo[4,5-*b*]pyridin-2-yl)-6-phenyl-hexan-l-ol (32 mg, 0.082 mmol, 22 %) as a brown solid. ¹H NMR (400 MHz, MeOD) δ 8.69 (d, *J* = 1.8 Hz, 1 H), 8.20 (d, *J* = 1.9 Hz, 1 H), 7.55 (d, *J* = 7.9 Hz, 2H), 7.29 (d, *J* = 7.8 Hz, 2H), 7.23 - 7.17 (m, 2H), 7.13 - 7.08 (m, 3H), 4.96 - 4.90 (m, 1 H), 2.57 (t, *J* = 7.7 Hz, 2H), 2.37 (s, 3H), 1.98 (p, 2H), 1.66 - 1.53 (m, 2H), 1.48 - 1.35 (m, 4H). ¹³C NMR (101 MHz, MeOD) δ 173.16, 154.99, 145.94, 145.00, 143.77, 139.61, 136.36, 135.57, 130.97(2C), 129.38(2C), 129.24(2C), 128.44(2C), 126.63, 118.55, 68.80, 36.76, 36.14, 32.60, 29.91, 26.05, 21.17. IR: 3351.4 (br), 3030.3, 2923.0, 2859.6, 1478.6, 1378.5, 1265.5, 1103.1, 813.3, 740.2, 694.8. HRMS (ESI+) m/z: calculated for C₂₅H₂₇N₂O₂ (M + H⁺) 387.2067; found 387.2072.

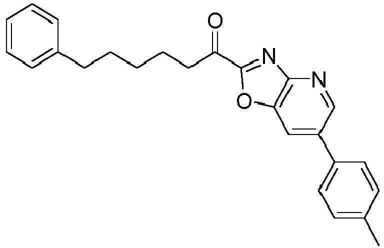

### 1-(6-(p-Tolyl)oxazolo[4,5-b]pyridin-2-yl)-6-phenylhexan-1-one (25)

Dess-Martin periodinane (35 mg, 0.075 mmol) was added to a solution of **25a** (21 mg, 0.05 mmol) in CH₂Cl₂ (2 mL) and the reaction mixture was stirred under argon atmosphere overnight. The reaction mixture was quenched with saturated NaHCO₃ (aq). The layers were separated and the organic layer was extracted with CH₂Cl₂. The combined organic layers were washed with brine, dried on MgSO₄, filtered and concentrated under reduced pressure. The residue was purified by flash chromatography over silica gel using toluene/ethyl acetate (90:10) with 1% Et₃N. Yielding 1-(6-(*p*-tolyl)oxazolo[4,5-*b*]pyridin-2-yl)-6-phenylhexan-1-one (14 mg, 0.036 mmol, 73%) as a white solid. ¹H NMR (400 MHz, CDCl₃) δ 8.97 (d, *J* = 2.0 Hz, 1H), 8.09 (d, *J* = 2.0 Hz, 1 H), 7.54 (d, *J* = 8.1 Hz, 2H), 7.34 (d, *J* = 8.0 Hz, 2H), 7.31 - 7.22 (m, 2H), 7.22 - 7.13 (m, 3H), 3.29 (t, *J* = 7.4 Hz, 2H), 2.64 (d, *J* = 8.0 Hz, 2H), 2.44 (s, 3H), 1.93 - 1.81 (m, 2H), 1.71 (dt, *J* = 15.4, 7.6 Hz, 2H), 1.55 - 1.44 (m, 2H). ¹³C NMR (101 MHz, CDCl₃) δ 190.20, 158.58, 152.99, 148.15, 144.05, 142.37, 138.98, 137.48, 133.95, 130.09 (2C), 128.37 (2C), 128.26 (2C), 127.49 (2C), 125.67, 117.68, 39.69, 35.69, 31.15, 28.69, 23.71, 21.20. IR: 3029.9, 2921.6, 2857.0, 1705.9, 1529.6, 1371.5, 1232.4, 1000.0, 819.7, 749.7, 697.2. HRMS (ESI+) m/z: calculated for C₂₅H₂₅N₂O₂ (M + H⁺) 385.1911; found 385.1914. LCMS purity > 95%, retention time: 7.23, method: CH₃CN/H₂O 50-90%.

### Example 10: Synthesis of Compound 26

### 2-Phenoxybenzaldehyde (26a)

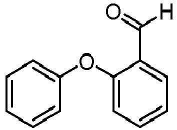

K₂CO₃ (22 g, 159 mmol) was added to a stirred solution of phenol (7.60 g, 81 mmol) in THF (100 mL) at reflux. After 1 h 2-fluorobenzaldehyde (8.49 mL, 101 mmol) was added and the reaction mixture was refluxed for an additional 24 h. Upon completion the mixture was cooled down to rt, concentrated *in vacuo,* saturated NaHCO₃ (100 mL) was added and product was extracted using EtOAc (3 x 80 mL). The combined organic layers were washed with brine, dried, *concentrated in vacuo* and purified by flash chromatography to yield 2-phenoxybenzaldehyde (8.05 g, 40,6 mmol, 50%). ¹H NMR (CDCl₃, 400 MHz): δ 10.52 (s, 1 H), 7.94 (dd, *J* = 7.8, 1.8 Hz, 1 H), 7.54 - 7.48 (m, 1 H), 7.43 - 7.36 (m, 2H), 7.22 - 7.15 (m, 2H), 7.10 - 7.04 (m, 2H), 6.90 (d, *J* = 8.4, 1 H). Spectroscopic data are in agreement with those reported in literature⁶.

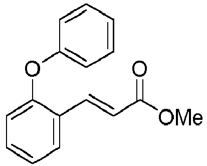

### Methyl 3-(2-phenoxyphenyl)acrylate (26b)

n-BuLi (30.6 ml, 76 mmol) was added dropwise over 10 minutes to a cooled solution (-80 °C) of methyl 2-(dimethoxyphosphoryl)acetate (11.04 mL, 76 mmol) in THF (100 mL) and the resulting mixture was stirred for 1 h. Then 2-phenoxybenzaldehyde (26a, 7.57 g, 38.2 mmol) was added, the reaction mixture was slowly warmed up to rt and stirred overnight. Upon completion the mixture was concentrated *in vacuo,* saturated NaHCO₃ (80 mL) was added and product was extracted using EtOAc (3 x 50 mL). The combined organic layers were washed with brine, dried, concentrated *in vacuo* and purified by flash chromatography to yield methyl 3-(2-phenoxyphenyl)acrylate (6.6 g, 26.0 mmol, 68%). ¹H NMR (CDCl₃, 400 MHz): δ 8.03 (d, *J* = 16.2 Hz, 1 H), 7.62 (dd, *J* = 7.8, 1.7 Hz, 1 H), 7.39 -6.81 (m, 8H), 6.57 (d, *J* = 16.2, 1 H), 3.79 (s, 3H).

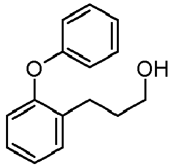

### 3-(2-Phenoxyphenyl)propan-1-ol (26c)

LAH (22,71 ml, 54,5 mmol) was added to a stirred and cooled solution (0 °C) of methyl 3-(2-phenoxyphenyl)acrylate (**26b,** 6.6 g, 26,0 mmol) in THF (100 mL) over 10 minutes. The reaction mixture was stirred for 2 hours and upon completion the mixture was concentrated *in vacuo,* saturated NaHCO₃ (80 mL) was added and product was extracted using EtOAc (3 x 50 mL). The combined organic layers were washed with brine, dried, concentrated *in vacuo* and purified by flash chromatography to yield 3-(2-phenoxyphenyl)propan-1-ol (1.3 g, 5.69 mmol, 22%). ¹H NMR (CDCl₃, 400 MHz): δ 7.34 - 7.26 (m, 3H), 7.17 (td, *J* = 7.7, 1.8 Hz, 1 H), 7.11 - 7.04 (m, 2H), 6.96 - 6.91 (m, 2H), 6.88 (dd, *J* = 8.1, 1.3 Hz, 1 H), 3.63 (t, *J* = 6.3 Hz, 2H), 2.73 (t, *J* = 7.2 Hz, 2H), 1.94 - 1.82 (m, 2H), 1.76 (bs, 1 H). ¹³C APT NMR (CDCl₃, 101 MHz): δ 157.82, 154.69, 133.25, 130.87, 129.87(2C), 127.56, 124.13, 122.89, 119.56, 117.89(2C), 62.21, 33.20, 26.31.

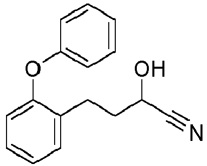

### 2-Hydroxy-4-(2-phenoxyphenyl)butanenitrile (26d)

The title compound was synthesized from 3-(2-phenoxyphenyl)propan-1-ol (**26c,** 1.3 g, 5.69 mmol) according to the previously reported 2 step procedure (see general synthesis). This yielded 2-hydroxy-4-(2-phenoxyphenyl)butanenitrile (727.9 mg, 2.87 mmol, 50%, 2 steps). ¹H NMR (CDCl₃, 400 MHz): δ 7.36 - 7.30 (m, 2H), 7.27 (dd, *J =* 7.5, 1.8 Hz, 1 H), 7.21 (td, *J* = 7.8, 1.8 Hz, 1 H), 7.12 - 7.07 (m, 2H), 6.96 - 6.93 (m, 2H), 6.88 (dd, *J* = 8.2, 1.2 Hz, 1 H), 4.42 (q, *J* = 6.3 Hz, 1 H), 2.87 (t, *J* = 7.4 Hz, 2H), 2.28 - 2.07 (m, 2H). ¹³C APT NMR (CDCl₃, 101 MHz): δ 157.32, 154.88, 131.03, 130.89, 130.00(2C), 128.35, 124.22, 123.32, 119.84, 119.41, 118.13(2C), 60.67, 35.67, 25.61.

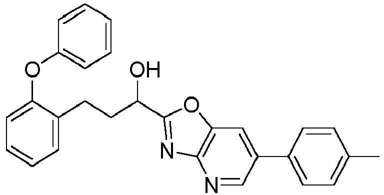

### 3-(2-Phenoxyphenyl)-1-(6-(p-tolyl)oxazolo[4,5-b]pyridin-2-yl)propan-1-ol (26e)

The title compound was synthesized from 2-hydroxy-4-(2-phenoxyphenyl)butanenitrile (**26d,** 144.6 mg, 0.571 mmol) and 2-amino-5-(*p*-tolyl)pyridin-3-ol (84.6 mg, 0.422 mmol) according to the procedures described for compound **1a.** This yielded 3-(2-phenoxyphenyl)-1-(6-(*p*-tolyl)oxazolo[4,5-*b*]pyridin-2-yl)propan-1-ol (35.9 mg, 0.082 mmol, 19%). ¹H NMR (CDCl₃, 400 MHz): δ 8.74 (d, *J* = 2.0 Hz, 1 H), 7.85 (d, *J* = 2.0 Hz, 1H), 7.47 (d, *J* = 7.9 Hz, 2H), 7.33 - 7.27 (m, 5H), 7.17 - 7.11 (m, 1 H), 7.07 - 7.01 (m, 2H), 6.92 (d, *J* = 8.0 Hz, 2H), 6.85 (d, *J* = 8.0 Hz, 1 H), 5.02 (dd, *J* = 7.9, 4.8 Hz, 1 H), 3.49 (bs, 1 H), 2.90 (t, *J* = 7.6 Hz, 2H), 2.43 (s, 3H), 2.40 - 2.28 (m, 2H). ¹³C APT NMR (CDCl₃, 101 MHz): δ 170.93, 170.90, 157.63, 154.80, 153.94, 145.65, 143.55, 138.42, 134.67, 132.18, 131.12, 130.08(2C), 129.85(2C), 127.84, 127.50(2C), 124.06, 122.94, 119.44, 118.00(2C), 116.79, 67.61, 35.70, 25.81, 21.30.

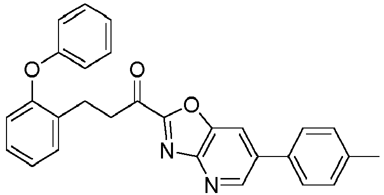

### 3-(2-phenoxyphenyl)-1-(6-(p-tolyl)oxazolo[4,5-b]pyridin-2-yl)propan-1-one (26)

The title compound was synthesized from 3-(2-phenoxyphenyl)-1-(6-(p-tolyl)oxazolo[4,5-*b*]pyridin-2-yl)propan-1-ol (**26e,** 24.1 mg, 0.055 mmol) according to the procedures described for compound **1.** This yielded 3-(2-phenoxyphenyl)-1-(6-(p-tolyl)oxazolo[4,5-b]pyridin-2-yl)propan-1-one (13.7 mg, 0.032 mmol, 57%). HRMS (ESI+) m/z: calculated for C₂₈H₂₃N₂O₃ ([M + H]), 435.1703; found, 435.1699. ¹H NMR (CDCl₃, 400 MHz): δ 8.96 (d, *J* = 2.0 Hz, 1 H), 8.05 (d, *J* = 2.0 Hz, 1 H), 7.56 - 7.49 (m, 2H), 7.38 - 7.27 (m, 5H), 7.21 - 7.14 (m, 1 H), 7.10 - 7.02 (m, 2H), 6.99 - 6.93 (m, 2H), 6.89 - 6.83 (m, 1 H), 3.65 (t, *J* = 7.4 Hz, 2H), 3.20 (t, *J* = 7.4 Hz, 2H), 2.44 (s, 3H). ¹³C APT NMR (CDCl₃, 101 MHz): δ 189.42, 158.62, 157.38, 155.05, 153.11, 148.24, 144.14, 139.12, 137.57, 134.10, 131.31, 130.92(2C), 130.24(2C), 129.87, 128.11, 127.64(2C), 123.89, 123.10, 119.03, 118.34(2C), 117.79, 40.23, 24.89, 21.35. Purity of >95% as determined by LC/MS.

### Example 11: Synthesis of Compound 27

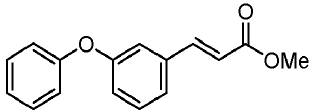

### Methyl 3-(3-phenoxyphenyl)acrylate (27a)

The title compound was synthesized from commercially available 3-phenoxybenzaldehyde (3.0 ml, 17,36 mmol) according to the procedures described for compound **26b.** This yielded methyl 3-(3-phenoxyphenyl)acrylate (4,53 g, 17,81 mmol, 103%). ¹H NMR (CDCl₃, 400 MHz): δ 7.60 (d, *J* = 16.0 Hz, 1 H), 7.30 - 7.22 (m, 2H), 7.20 (d, *J* = 7.9 Hz, 1 H), 7.17 - 7.10 (m, 2H), 7.08 - 7.01 (m, 1 H), 6.99 - 6.90 (m, 3H), 6.36 (d, *J* = 16.0 Hz, 1 H), 3.68 (s, 3H). ¹³C APT NMR (CDCl₃, 101 MHz): δ 166.34, 157.37, 156.33, 143.48, 135.83, 129.72, 129.47(2C), 123.21, 122.53, 119.95, 118.65(2C), 118.21, 117.51, 51.01.

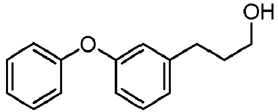

### 3-(3-Phenoxyphenyl)propan-1-ol (27b)

The title compound was synthesized from methyl 3-(3-phenoxyphenyl)acrylate (**27a,** 4.50 g, 17.70 mmol) according to the procedures described for compound **26c.** This yielded 3-(3-phenoxyphenyl)propan-1-ol (3.15 g, 13.80 mmol, 78%). ¹H NMR (CDCl₃, 400 MHz): δ 7.36 - 7.28 (m, 2H), 7.28 - 7.21 (m, 1 H), 7.12 - 7.06 (m, 1 H), 7.03 - 6.97 (m, 2H), 6.96 - 6.92 (m, 1 H), 6.89 - 6.80 (m, 2H), 3.67 (t, *J* = 6.4 Hz, 2H), 2.68 (t, *J =* 6.8 Hz, 2H), 1.92 - 1.83 (m, 2H), 1.56 (bs, 1 H). ¹³C APT NMR (CDCl₃, 101 MHz): δ 157.43, 157.40, 144.06, 129.85(2C), 129.75, 123.51, 123.28, 119.04, 118.96(2C), 116.47, 62.33, 34.17, 32.09.

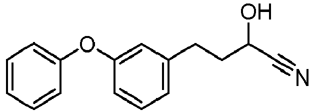

### 2-Hydroxy-4-(3-phenoxyphenyl)butanenitrile (27c)

The title compound was synthesized from 3-(3-phenoxyphenyl)propan-1-ol (**27b,** 3.1 g, 13.58 mmol) according to the previously desscribed general synthesis scheme. This yielded 2-hydroxy-4-(3-phenoxyphenyl)butanenitrile (2.10 g, 8.29 mmol, 61% over 2 steps). ¹H NMR (CDCl₃, 400 MHz): δ 7.37 - 7.31 (m, 2H), 7.29 - 7.23 (m, 1H), 7.14 - 7.09 (m, 1 H), 7.02 - 6.98 (m, 2H), 6.94 (dt, *J* = 7.6, 1.3 Hz, 1 H), 6.88 - 6.84 (m, 2H), 4.43 (t, *J* = 6.3 Hz, 1 H), 3.03 (bs, 1 H), 2.88 - 2.72 (m, 2H), 2.24 - 2.06 (m, 2H). ¹³C APT NMR (CDCl₃, 101 MHz): δ 157.72, 157.09, 141.78, 130.11, 129.92(2C), 123.53, 123.40, 119.87, 119.08(2C), 118.90, 116.96, 60.42, 36.54, 30.63.

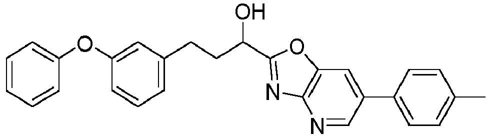

### 3-(3-Phenoxyphenyl)-1-(6-(p-tolyl)oxazolo[4,5-b]pyridin-2-yl)propan-1-ol (27d)

The title compound was synthesized from 2-hydroxy-4-(3-phenoxyphenyl)butanenitrile (27c, 184.1 mg, 0.727 mmol) and 2-amino-5-(*p*-tolyl)pyridin-3-ol (91.3 mg, 0.456 mmol) according to the procedures described for compound 1a. This yielded 3-(3-phenoxyphenyl)-1-(6-(*p*-tolyl)oxazolo[4,5-*b*]pyridin-2-yl)propan-1-ol (41.9 mg, 0.096 mmol, 21 %). ¹H NMR (CDCl₃, 400 MHz): δ 8.70 (d, *J =* 1.9 Hz, 1 H), 7.89 (d, *J =* 1.9 Hz, 1 H), 7.44 (d, *J* = 7.9 Hz, 2H), 7.34 - 7.24 (m, 4H), 7.20 (t, *J* = 7.9 Hz, 1 H), 7.07 (t, *J =* 7.5 Hz, 1 H), 7.00 - 6.90 (m, 4H), 6.79 (dd, *J* = 8.0, 2.4 Hz, 1 H), 5.07 (dd, *J* = 7.7, 5.0 Hz, 1 H), 4.36 (bs, 1 H), 2.90 - 2.81 (m, 2H), 2.41 (s, 3H), 2.38 - 2.28 (m, 2H). ¹³C APT NMR (CDCl₃, 101 MHz): δ 171.27, 157.38, 157.26, 153.75, 145.55, 143.53, 143.05, 138.44, 134.79, 134.49, 130.06(2C), 129.82(2C), 129.78, 127.48(2C), 123.65, 123.26, 119.16, 118.92(2C), 116.91, 116.58, 67.34, 36.74, 31.09, 21.28.

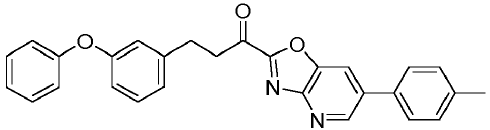

### 3-(3-Phenoxyphenyl)-1-(6-(p-tolyl)oxazolo[4,5-b]pyridin-2-yl)propan-1-one (27)

The title compound was synthesized from 3-(3-phenoxyphenyl)-1-(6-(p-tolyl)oxazolo[4,5-*b*]pyridin-2-yl)propan-1-ol (**27d,** 24.1 mg, 0.055 mmol) according to the procedures described for compound **1.** This yielded 3-(3-phenoxyphenyl)-1-(6-(p-tolyl)oxazolo[4,5-*b*]pyridin-2-yl)propan-1-one (13.7 mg, 0.032 mmol, 57%). HRMS (ESI+) m/z: calculated for C₂₈H₂₃N₂O₃ ([M + H]), 435.1703; found, 435.1701. ¹H NMR (CDCl₃, 400 MHz): δ 8.97 (d, *J* = 2.0 Hz, 1 H), 8.09 (d, *J* = 2.0 Hz, 1 H), 7.58 - 7.49 (m, 2H), 7.38 - 7.30 (m, 4H), 7.29 - 7.22 (m, 1 H), 7.13 - 7.06 (m, 1 H), 7.05 - 6.98 (m, 3H), 6.94 (t, *J* = 2.0 Hz, 1 H), 6.85 (ddd, *J* = 8.3, 2.6, 1.0 Hz, 1 H), 3.62 (t, *J* = 7.6 Hz, 2H), 3.14 (t, *J* = 7.6 Hz, 2H), 2.44 (s, 3H). ¹³C APT NMR (CDCl₃, 101 MHz): 189.11, 158.54, 157.58, 157.19, 153.03, 148.39, 144.24, 142.17, 139.18, 137.74, 134.04, 130.26(2C), 130.00, 129.87(2C), 127.65(2C), 123.41, 123.38, 119.07(2C), 118.98, 117.85, 116.91, 41.17, 29.58, 21.36. Purity of >95% as determined by LC/MS.

### Example 12: Synthesis of Compound 28

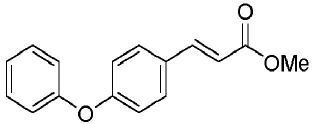

### Methyl 3-(4-phenoxyphenyl)acrylate (28a)

The title compound was synthesized from commercially available 4-phenoxybenzaldehyde (0.88 mL, 5.05 mmol) according to the procedures described for compound **26b.** This yielded methyl 3-(4-phenoxyphenyl)acrylate (1.14 g, 4.50 mmol, 89%). ¹H NMR (CDCl₃, 400 MHz): δ 7.65 (d, *J* = 16.0 Hz, 1 H), 7.48 - 7.42 (m, 2H), 7.37 - 7.31 (m, 2H), 7.17 - 7.10 (m, 1 H), 7.05 - 6.99 (m, 2H), 6.98 - 6.91 (m, 2H), 6.33 (d, *J* = 16.0 Hz, 1 H), 3.77 (s, 3H). ¹³C APT NMR (CDCl₃, 101 MHz): δ 167.45, 159.46, 156.06, 144.05, 129.90(2C), 129.73(2C), 129.08, 124.08, 119.64(2C), 118.33(2C), 116.39, 51.56.

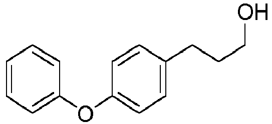

### 3-(4-Phenoxyphenyl)propan-1-ol (28b)

Ccatalytic Pd/C was added to a solution of methyl 3-(4-phenoxyphenyl)acrylate (**28a,** 1.14 g, 4.48 mmol) in MeOH (50 mL) and the mixture was stirred under H₂ (g) for 140 h. Upon completion the mixture was concentrated *in vacuo* and purified by flash chromatography to yield methyl 3-(4-phenoxyphenyl)propanoate. This was directly converted to the title compound according to the procedures described for compound **26c.** This yielded 3-(4-phenoxyphenyl)propan-1-ol (0.545 g, 2.39 mmol, 53% over 2 steps). ¹H NMR (CDCl₃, 400 MHz): δ 7.31 - 7.23 (m, 2H), 7.14 - 7.09 (m, 2H), 7.03 (tt, *J* = 7.3, 1.1 Hz, 1 H), 6.99 - 6.93 (m, 2H), 6.93 - 6.87 (m, 2H), 3.62 (t, *J* = 6.5 Hz, 2H), 2.84 (bs, 1 H), 2.64 (t, *J* = 7.5 Hz, 2H), 1.90 - 1.77 (m, 2H). ¹³C APT NMR (CDCl₃, 101 MHz): δ 157.54, 155.09, 136.84, 129.67(2C), 129.61(2C), 122.94, 119.02(2C), 118.48(2C), 61.87, 34.24, 31.29.

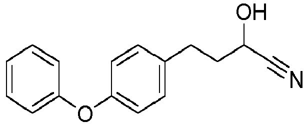

### 2-Hydroxy-4-(4-phenoxyphenyl)butanenitrile (28c)

The title compound was synthesized from 3-(4-phenoxyphenyl)propan-1-ol (**28b,** 0.544 g, 2.39 mmol) according to the previously desscribed general synthesis scheme. This yielded 2-hydroxy-4-(4-phenoxyphenyl)butanenitrile (0.563 g, 2.22 mmol, 93% over 2 steps). ¹H NMR (CDCl₃, 400 MHz): δ 7.33 - 7.25 (m, 2H), 7.16 - 7.10 (m, 2H), 7.09-7.04 (m, 1 H), 7.00 - 6.95 (m, 2H), 6.95 - 6.89 (m, 2H), 4.40 (t, *J* = 6.8 Hz, 1 H), 3.82 (bs, 1 H), 2.86 - 2.71 (m, 2H), 2.22 - 2.01 (m, 2H). ¹³C APT NMR (CDCl₃, 101 MHz): δ 157.26, 155.73, 134.52, 129.78(2C), 129.75(2C), 123.25, 120.07, 119.18(2C), 118.71 (2C), 60.20, 36.59, 29.91.

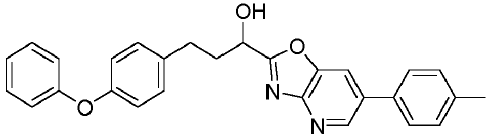

### 3-(4-Phenoxyphenyl)-1-(6-(p-tolyl)oxazolo[4,5-b]pyridin-2-yl)propan-1-ol (28d)

The title compound was synthesized from 2-hydroxy-4-(4-phenoxyphenyl)butanenitrile (**28c,** 126.9 mg, 0.501 mmol) and 2-amino-5-(*p*-tolyl)pyridin-3-ol (83.0 mg, 0.415 mmol) according to the procedures described for compound **1a.** This yielded 3-(4-phenoxyphenyl)-1-(6-(*p*-tolyl)oxazolo[4,5-*b*]pyridin-2-yl)propan-1-ol (28.4 mg, 0.065 mmol, 16%). ¹H NMR (CDCl₃, 400 MHz): δ 8.73 (d, *J =* 1.9 Hz, 1 H), 7.91 (d, *J* = 2.0 Hz, 1 H), 7.49 - 7.42 (m, 2H), 7.34 - 7.27 (m, 4H), 7.22 - 7.16 (m, 2H), 7.11 - 7.03 (m, 1 H), 6.99 - 6.93 (m, 2H), 6.93 - 6.87 (m, 2H), 5.08 (dd, *J* = 7.8, 5.0 Hz, 1 H), 4.14 (bs, 1 H), 2.88 (t, *J* = 7.7 Hz, 2H), 2.42 (s, 3H), 2.40 - 2.28 (m, 2H). ¹³C APT NMR (CDCl₃, 101 MHz): δ 171.19, 157.53, 155.49, 153.85, 145.65, 143.55, 138.46, 135.81, 134.79, 134.54, 130.08(2C), 129.94(2C), 129.79(2C), 127.50(2C), 123.12, 119.13(2C), 118.70(2C), 116.85, 67.35, 37.03, 30.45, 21.29.

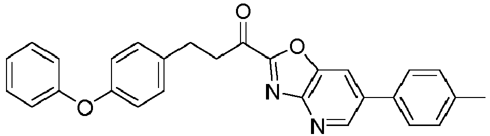

### 3-(4-phenoxyphenyl)-1-(6-(p-tolyl)oxazolo[4,5-b]pyridin-2-yl)propan-1-one (28)

The title compound was synthesized from 3-(4-phenoxyphenyl)-1-(6-(p-tolyl)oxazolo[4,5-*b*]pyridin-2-yl)propan-1-ol (**28d,** 21.3 mg, 0.049 mmol) according to the procedures described for compound **1.** This yielded 3-(4-phenoxyphenyl)-1-(6-(p-tolyl)oxazolo[4,5-b]pyridin-2-yl)propan-1-one (12.9 mg, 0.030 mmol, 61%). HRMS (ESI+) m/z: calculated for C₂₈H₂₃N₂O₃ ([M + H]), 435.1703; found, 435.1700. ¹H NMR (CDCl₃, 400 MHz): 9.00 - 8.95 (m, 1 H), 8.11 - 8.06 (m, 1 H), 7.57 - 7.51 (m, 2H), 7.36 - 7.28 (m, 4H), 7.28 - 7.22 (m, 2H), 7.11 - 7.05 (m, 1 H), 7.01 - 6.92 (m, 4H), 3.63 (t, *J* = 7.5 Hz, 2H), 3.15 (t, *J* = 7.5 Hz, 2H), 2.44 (s, 3H). ¹³C APT NMR (CDCl₃, 101 MHz): δ 189.30, 158.58, 157.50, 155.76, 153.05, 148.38, 144.24, 139.17, 137.74, 135.05, 134.04, 130.25(2C), 129.88(2C), 129.82(2C), 127.64(2C), 123.19, 119.26(2C), 118.77(2C), 117.85, 41.52, 29.07, 21.36. Purity of >95% as determined by LC/MS.

### Example 13: Synthesis of Compound 29

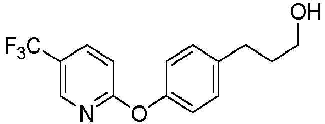

### 3-(4-((5-(Trifluoromethyl)pyridin-2-yl)oxy)phenyl)propan-1-ol (29a)

The title compound was synthesized from 4-(3-hydroxypropyl)phenol (1.311 g, 8.61 mmol), 2-fluoro-5-(trifluoromethyl)pyridine (1.49 g, 9,03 mmol) and K₂CO₃ (1.2 g, 8.68 mmol) according to the procedures described for compound **26a.** This yielded 3-(4-((5-(trifluoromethyl)pyridin-2-yl)oxy)phenyl)propan-1-ol (2.15 g, 7.24 mmol, 84%). ¹H NMR (CDCl₃, 400 MHz): δ 8.47 - 8.41 (m, 1 H), 7.89 (dd, *J* = 8.7, 2.5 Hz, 1 H), 7.29 - 7.23 (m, 2H), 7.09 - 7.04 (m, 2H), 7.02 - 6.96 (m, 1 H), 3.71 (t, *J* = 6.4 Hz, 2H), 2.78 - 2.70 (m, 2H), 1.96 - 1.86 (m, 2H), 1.58 (bs, 1 H). ¹³C APT NMR (CDCl₃, 101 MHz): δ 166.09, 151.30, 145.61 (q, *J* = 4.3 Hz), 139.19, 136.77 (q, *J* = 3.1 Hz), 129.89(2C), 123.82 (q, *J* = 272.4 Hz), 121.48 (q, *J* = 33.3 Hz), 121.46(2C), 111.36, 62.30, 34.25, 31.61.

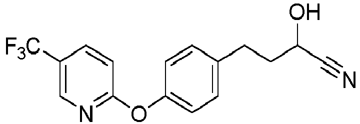

### 2-Hydroxy-4-(4-((5-(trifluoromethyl)pyridin-2-yl)oxy)phenyl)butanenitrile (29b)

The title compound was synthesized from 3-(4-((5-(trifluoromethyl)pyridin-2-yl)oxy)phenyl)propan-1-ol (1.38 g, 4.64 mmol) according to the previously desscribed general synthesis scheme. This yielded 2-hydroxy-4-(4-((5-(trifluoromethyl)pyridin-2-yl)oxy)phenyl)butanenitrile (1.20 g, 3.72 mmol, 69%). ¹H NMR (CDCl₃, 400 MHz): δ 8.47 - 8.40 (m, 1H), 7.91 (dd, *J* = 8.7, 2.5 Hz, 1 H), 7.27 (dd, *J* = 6.7, 1.8 Hz, 2H), 7.13 - 7.05 (m, 2H), 7.03 - 6.98 (m, 1 H), 4.45 (t, *J* = 6.7 Hz, 1 H), 2.95 - 2.79 (m, 2H), 2.27 - 2.11 (m, 2H). ¹³C APT NMR (CDCl₃, 101 MHz): δ 165.91, 154.50, 151.77, 145.51 (q, *J =* 4.3 Hz), 136.99 (q, *J =* 3.4 Hz), 130.02(2C), 123.74 (q, *J* = 272.6 Hz), 121.91(2C), 121.74 (q, *J =* 33.5 Hz), 119.93, 111.47, 60.38, 36.62, 30.18.

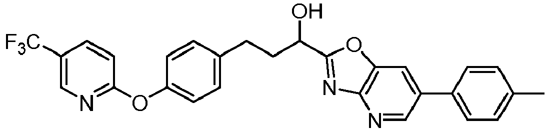

### 1-(6-(p-Tolyl)oxazolo[4,5-b]pyridin-2-yl)-3-(4-((5-(trifluoromethyl)pyridin-2-yl)oxy)phenyl)propan-1-ol (29c)

The title compound was synthesized from 2-hydroxy-4-(4-((5-(trifluoromethyl)pyridin-2-yl)oxy)phenyl)butanenitrile (0.167 g, 0.518 mmol) and 2-amino-5-(*p*-tolyl)pyridin-3-ol (214 mg, 1.069 mmol) according to the procedures described for compound **1a.** This yielded 1-(6-(p-tolyl)oxazolo[4,5-b]pyridin-2-yl)-3-(4-((5-(trifluoromethyl)pyridin-2-yl)oxy)phenyl)propan-1-ol (57.3 mg, 0.113 mmol, 22%). ¹H NMR (CDCl₃, 400 MHz): δ 8.84 - 8.66 (m, 1 H), 8.50 - 8.36 (m, 1 H), 7.94 - 7.90 (m, 1 H), 7.86 (dd, *J* = 8.7, 2.6 Hz, 1 H), 7.46 (d, *J* = 7.8 Hz, 2H), 7.36 - 7.23 (m, 4H), 7.11 - 7.00 (m, 2H), 6.96 (d, *J* = 8.6 Hz, 1 H), 5.16 - 5.08 (m, 1H), 4.41 (bs, 1H), 2.92 (t, J = 7.6 Hz, 2H), 2.42 (m, 5H). ¹³C APT NMR (CDCl₃, 101 MHz): δ 171.16, 165.98, 153.87, 151.47, 145.63, 145.56 (q, *J =* 4.0 Hz), 143.57, 138.46, 138.19, 136.75 (q, *J* = 3.0 Hz), 134.81, 134.54, 130.06(4C), 127.48(2C), 123.80 (q, *J* = 272.7 Hz), 121.50 (q, *J* = 34.3 Hz), 121.49(2C), 116.86, 111.34, 67.35, 36.83, 30.62, 21.26.

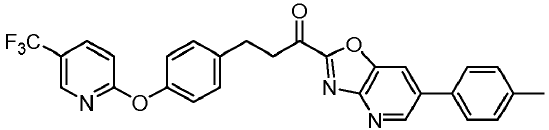

### 1-(6-(p-Tolyl)oxazolo[4,5-b]pyridin-2-yl)-3-(4-((5-(trifluoromethyl)pyridin-2-yl)oxy)phenyl)propan-1-one (29)

The title compound was synthesized from 1-(6-(*p*-tolyl)oxazolo[4,5-*b*]pyridin-2-yl)-3-(4-((5-(trifluoromethyl)pyridin-2-yl)oxy)phenyl)propan-1-ol (38.5 mg, 0.076 mmol) according to the procedures described for compound 1. This yielded 1-(6-(p-tolyl)oxazolo[4,5-b]pyridin-2-yl)-3-(4-((5-(trifluoromethyl)pyridin-2-yl)oxy)phenyl)propan-1-one (30 mg, 0.060 mmol, 78%). HRMS (ESI+) m/z: calculated for C₂₈H₂₁F₃N₃O₃ ([M + H]), 504.1530; found, 504.1527. ¹H NMR (CDCl₃, 400 MHz): δ 8.98 (d, *J* = 2.0 Hz, 1 H), 8.46 - 8.41 (m, 1 H), 8.10 (d, *J =* 2.0 Hz, 1 H), 7.89 (dd, *J* = 8.7, 2.6 Hz, 1 H), 7.56 - 7.51 (m, 2H), 7.35 (t, *J* = 8.5 Hz, 4H), 7.12 - 7.07 (m, 2H), 6.99 (d, *J* = 8.6 Hz, 1 H), 3.67 (t, *J* = 7.5 Hz, 2H), 3.20 (t, *J* = 7.5 Hz, 2H), 2.44 (s, 3H). ¹³C APT NMR (CDCl₃, 101 MHz): δ 189.16, 166.00, 158.59, 153.07, 151.76, 148.43, 145.63 (q, *J* = 4.3 Hz), 144.28, 139.20, 137.79, 137.40, 136,78 (q, *J* = 3.1 Hz), 134.06, 130.27(2C), 130.03(2C), 127.66(2C), 121.73(2C), 117.85, 111.41, 41.31, 29.16, 21.35. Two carbon signals (quatonary quartets of **-CF₃** and **-C-**CF₃) not observed. Purity of >95% as determined by LC/MS.

### Example 14: Synthesis of Compound 30

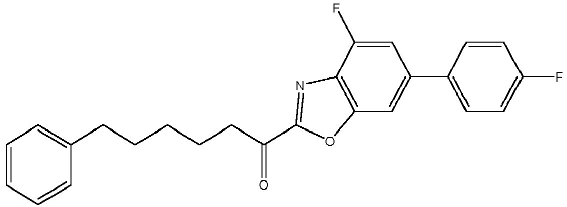

***1-(4-fluoro-6-(4-fluorophenyl)benzo*[*d*]*oxazol-2-yl)-6-phenylhexan-1-one:*** 1-(6-bromo-4-fluorobenzo-2-oxazolyl)-6-phenylhexan-1-one (1 eq., 33 mg, 0.085 mmol), 4-fluorophenylboronic acid (5 eq., 59.2 mg, 0.42 mmol), Pd(PPh₃)₄ (9.8 mg, 10 mol%), and CsF (8 eq., 102.8 mg, 0.68 mmol) were used as starting substances. Using the general procedure described above, *1-(4-fluoro-6-(4-fluorophenyl)benzo[d]oxazol-2-yl)-6-phenylhexan-1-one* (compound 30) was obtained as a white solid (27.6 mg, 81%) after purification by silica gel column chromatography (EtOAc/pentane, 2%): *R_{F}* = 0.45 (5% EtOAc/pentane); ¹H NMR (400 MHz, CDCl₃): δ = 7.62 - 7.52 (m, 3H), 7.35 (d, *J =* 10.5 Hz, 1 H), 7.32 - 7.23 (m, 2H), 7.23 - 7.12 (m, 5H), 3.24 (t, *J* = 7.4 Hz, 2H), 2.64 (t, *J=* 7.7 Hz, 2H), 1.85 (p, *J* = 7.5 Hz, 2H), 1.70 (p, *J* = 7.7 Hz, 2H,), 1.47 (qd, *J* = 10.3, 9.3, 4.4 Hz, 2H). HR-MS (ESI+): *m*/*z =* calc. for C₂₅H₂₂F₂NO₂ [M + H⁺] 406.1613. Found 406.1610; Δ = 0.74 ppm. LC-MS purity > 93%, retention time: 5.46, method TFA 70-90%.

### Example 15: Synthesis of Compound 31

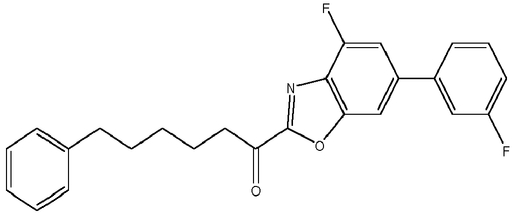

***1-(4-fluoro-6-(2-fluorophenyl)benzo*[*d*]*oxazol-2-yl)-6-phenylhexan-1-one:*** 1-(6-bromo-4-fluorobenzo-2-oxazolyl)-6-phenylhexan-1-one (1 eq., 33.9 mg, 0.087 mmol), 2-fluorophenylboronic acid (5 eq., 60.7 mg, 0.43 mmol), Pd(PPh₃)₄ (10 mg, 10 mol%), and CsF (8 eq., 105.4 mg, 8 mmol) were used as starting substances. Using the general procedure described above, *1-(4-fluoro-6-(2-fluorophenyl)benzo*[*d*]*oxazol-2-yl)-6-phenylhexan-1-one* (compound 31) was obtained as a white solid (10.1 mg, 29%) after purification by silica gel column chromatography (EtOAc/pentane, 2%): *R_{F}* = 0.75 (5% EtOAc/pentane); ¹H NMR (400 MHz, CDCl₃): δ = 7.65 (t, *J* = 1.3 Hz, 1 H), 7.47 (td, *J* = 7.7, 1.8 Hz, 1 H), 7.44 - 7.36 (m, 2H), 7.30 - 7.24 (m, 3H), 7.24 - 7.15 (m, 4H), 3.26 (t, *J* = 7.4 Hz, 2H), 2.64 (t, *J* = 7.7 Hz, 2H), 1.86 (p, *J* = 7.5 Hz, 2H), 1.71 (p, *J* = 7.6 Hz, 2H), 1.54 - 1.43 (m, 2H). HR-MS (ESI+): *m*/*z* = calc. for C₂₅H₂₂F₂NO₂ [M + H⁺] 406.1613. Found 406.1605; Δ = 1.97 ppm. LC-MS purity > 81%, retention time: 5.9, method TFA 70-90%.

### Example 16: Synthesis of Compound 32

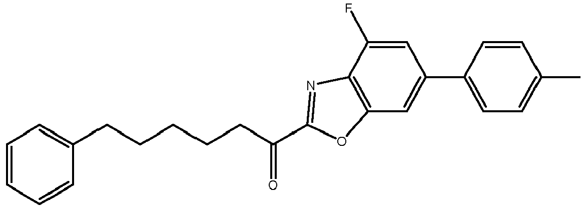

***1-(4-fluoro-6-(p-tolyl)benzo*[*d*]*oxazol-2-yl)-6-phenylhexan-1-one:*** 1-(6-bromo-4-fluorobenzo-2-oxazolyl)-6-phenylhexan-1-one (1 eq., 33.5 mg, 0.086 mmol), 4-tolylboronic acid (4 eq., 46.7 mg, 0.34 mmol), Pd(PPh₃)₄ (9.9 mg, 10 mol%), and CsF (8 eq., 104.4 mg, 8 mmol) were used as starting substances. Using the general procedure described above, *1-(4-fluoro-6-(p-tolyl)benzo[d]oxazol-2-yl)-6-phenylhexan-1-one* (compound 32) was obtained as an off-white solid (20.9 mg, 61%) after purification by silica gel column chromatography (Et₂O/PetEt, 5%): *R_{F}* = 0.55 (10% Et₂O/PetEt); ¹H NMR (400 MHz, CDCl₃): δ = 7.61 (d, *J* = 1.3 Hz, 1 H), 7.50 (d, *J* = 8.0 Hz, 2H), 7.39 (d, *J =* 10.8 Hz, 1 H), 7.33 - 7.23 (m, 4H), 7.21 - 7.13 (m, 3H), 3.24 (t, *J =* 7.4 Hz, 2H), 2.64 (t, *J* = 7.7 Hz, 2H), 2.42 (s, 3H), 1.85 (p, *J* = 7.5 Hz, 2H), 1.70 (p, *J =* 7.6 Hz, 2H), 1.54 - 1.41 (m, 2H). HR-MS (ESI+): *m*/*z* = calc. for C₂₆H₂₅FNO₂ [M + H⁺] 402.1864. Found 402.1862; Δ = 0.50 ppm. LC-MS purity > 99%, retention time: 5.88, method TFA 70-90%.

### Example 17: Synthesis of Compound 33

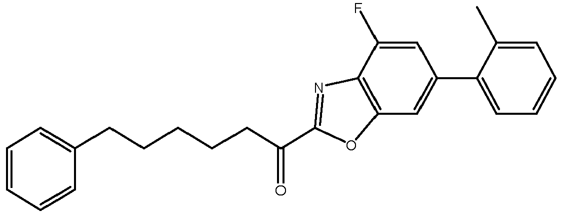

***1-(4-fluoro-6-(o-tolyl)benzo*[*d*]*oxazol-2-yl)-6-phenylhexan-1-one:*** 1-(6-bromo-4-fluorobenzo-2-oxazolyl)-6-phenylhexan-1-one (1 eq., 34.2 mg, 0.088 mmol), 2-tolylboronic acid (4 eq., 47.7 mg, 0.35 mmol), Pd(PPh₃)₄ (10.1 mg, 10 mol%), and CsF (8 eq., 106.5 mg, 8 mmol) were used as starting substances. Using the general procedure described above, *1-(4-fluoro-6-(o-tolyl)benzo[d]oxazol-2-yl)-6-phenylhexan-1-one* (compound 33) was obtained as an white solid (21.1 mg, 60%) after purification by silica gel column chromatography (Et₂O/PetEt, 5%): *R_{F}* = 0.62 (10% Et₂O/PetEt); ¹H NMR (400 MHz, CDCl₃): δ = 7.41 (t, *J* = 1.0 Hz, 1 H), 7.35 - 7.21 (m, 6H), 7.21 - 7.11 (m, 4H), 3.26 (t, *J* = 7.4 Hz, 2H), 2.64 (t, *J* = 7.7 Hz, 2H), 2.28 (s, 3H), 1.86 (p, *J* = 7.5 Hz, 2H), 1.71 (p, *J =* 7.5 Hz, 2H), 1.54 - 1.41 (m, 2H). HR-MS (ESI+): *m*/*z =* calc. for C₂₆H₂₅FNO₂ [M + H⁺] 402.1864. Found 402.1855; Δ = 2.24 ppm. LC-MS purity > 99%, retention time: 5.64, method TFA 70-90%.

### Example 18: Synthesis of Compound 34

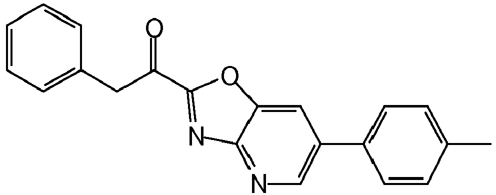

### 2-phenyl-1-(6-(p-tolyl)oxazolo[4,5-b]pyridin-2-yl)ethan-1-one:

The title compound was synthesized from yielded 2-phenyl-1-(6-(p-tolyl)oxazolo[4,5-*b*]pyridin-2-yl)ethan-1-ol according to the procedures described for compound 1. This yielded 2-phenyl-1-(6-(*p*-tolyl)oxazolo[4,5-*b*]pyridin-2-yl)ethan-1-one. HRMS (ESI+): *m*/*z =* 328.1212 [M + H⁺]

### Example 19: Synthesis of Compound 35

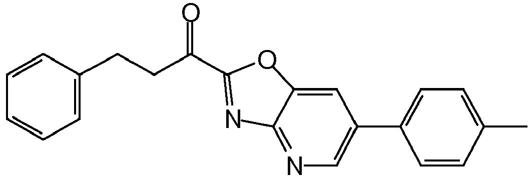

### 3-phenyl-1-(6-(p-tolyl)oxazolo[4,5-b]pyridin-2-yl)propan-1-one:

The title compound was synthesized from 3-phenyl-1-(6-(*p*-tolyl)oxazolo[4,5-*b*]pyridin-2-yl)propan-1-ol according to the procedures described for compound **1.** This yielded 3-phenyl-1-(6-(*p*-tolyl)oxazolo[4,5-*b*]pyridin-2-yl)propan-1-one. HRMS (ESI+): *m*/*z =* 342.1368 [M + H⁺]

### Example 20: Synthesis of Compound 36

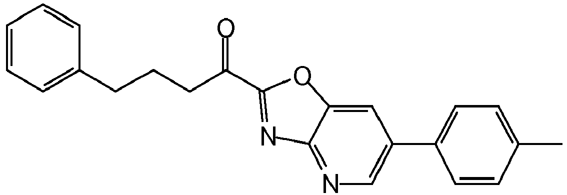

### 4-phenyl-1-(6-(p-tolyl)oxazolo[4,5-b]pyridin-2-yl)butan-1-one:

The title compound was synthesized from 4-phenyl-1-(6-(*p*-tolyl)oxazolo[4,5-*b*]pyridin-2-yl)butan-1-ol according to the procedures described for compound 1. This yielded 4-phenyl-1-(6-(*p*-tolyl)oxazolo[4,5-*b*]pyridin-2-yl)butan-1-one. HRMS (ESI+): *m*/*z =* 356.1525 [M + H⁺]

### Example 21: Synthesis of Compound 37

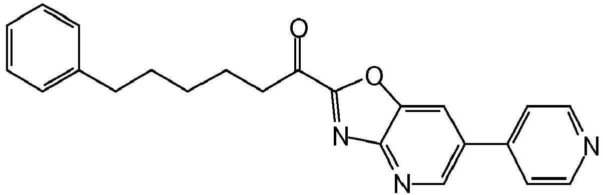

### 6-phenyl-1-(6-(pyridin-4-yl)oxazolo[4,5-b]pyridin-2-yl)hexan-1-one:

The title compound was synthesized from 6-phenyl-1-(6-(pyridin-4-yl)oxazolo[4,5-*b*]pyridin-2-yl)hexan-1-ol according to the procedures described for compound **1.** This yielded 6-phenyl-1-(6-(pyridin-4-yl)oxazolo[4,5-*b*]pyridin-2-yl)hexan-1-one. HRMS (ESI+): *m*/*z* = 371.1634 [M + H⁺]

### Example 22: Synthesis of Compound 38

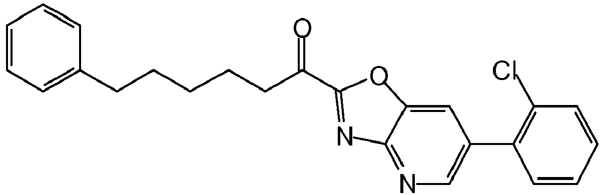

### 1-(6-(2-chlorophenyl)oxazolo[4,5-b]pyridin-2-yl)-6-phenylhexan-1-one:

The title compound was synthesized from 1-(6-(2-chlorophenyl)oxazolo[4,5-*b*]pyridin-2-yl)-6-phenylhexan-1-ol according to the procedures described for compound 1. This yielded 1-(6-(2-chlorophenyl)oxazolo[4,5-*b*]pyridin-2-yl)-6-phenylhexan-1-one.

HRMS (ESI+): *m*/*z* = 404.1292 [M + H⁺]

### Example 23: Synthesis of Compound 39

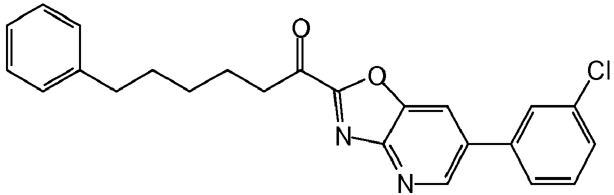

### 1-(6-(3-chlorophenyl)oxazolo[4,5-b]pyridin-2-yl)-6-phenylhexan-1-one:

The title compound was synthesized from 1-(6-(3-chlorophenyl)oxazolo[4,5-*b*]pyridin-2-yl)-6-phenylhexan-1-ol according to the procedures described for compound 1. This yielded 1-(6-(3-chlorophenyl)oxazolo[4,5-*b*]pyridin-2-yl)-6-phenylhexan-1-one. HRMS (ESI+): *m*/*z =* 404.1292 [M + H⁺]

### Example 24: Synthesis of Compound 40

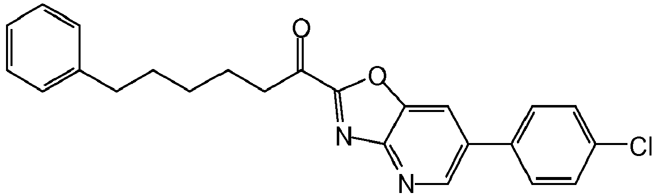

### 1-(6-(4-chlorophenyl)oxazolo[4,5-b]pyridin-2-yl)-6-phenylhexan-1-one:

The title compound was synthesized from 1-(6-(4-chlorophenyl)oxazolo[4,5-*b*]pyridin-2-yl)-6-phenylhexan-1-ol according to the procedures described for compound **1.** This yielded 1-(6-(4-chlorophenyl)oxazolo[4,5-*b*]pyridin-2-yl)-6-phenylhexan-1-one. HRMS (ESI+): *m*/*z =* 404.1292 [M + H⁺]

### Example 25: Synthesis of Compound 41

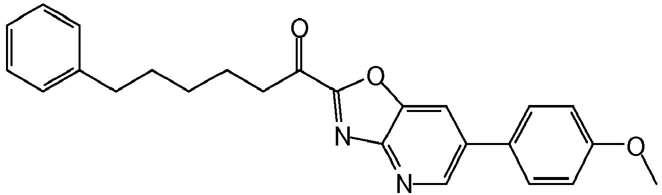

### 1-(6-(4-methoxyphenyl)oxazolo[4,5-b]pyridin-2-yl)-6-phenylhexan-1-one:

The title compound was synthesized from 1-(6-(4-methoxyphenyl)oxazolo[4,5-*b*]pyridin-2-yl)-6-phenylhexan-1-ol according to the procedures described for compound **1.** This yielded 1-(6-(4-methoxyphenyl)oxazolo[4,5-*b*]pyridin-2-yl)-6-phenylhexan-1-one. HRMS (ESI+): *m*/*z* =400.1787 [M + H⁺]

### Example 26: Synthesis of Compound 42

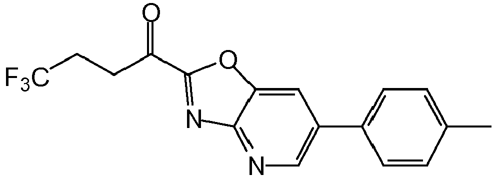

### 4,4,4-trifluoro-1-(6-(p-tolyl)oxazolo[4,5-b]pyridin-2-yl)butan-1-one:

The title compound was synthesized from 4,4,4-trifluoro-1-(6-(*p*-tolyl)oxazolo[4,5-*b*]pyridin-2-yl)butan-1-ol according to the procedures described for compound **1.** This yielded 4,4,4-trifluoro-1-(6-(*p*-tolyl)oxazolo[4,5-*b*]pyridin-2-yl)butan-1-one. HRMS (ESI+): *m*/*z* = 334.0929 [M + H⁺]

### Example 27: Synthesis of Compound 43

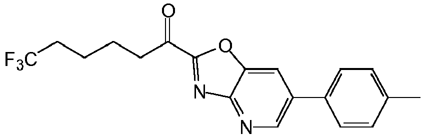

### 6,6,6-trifluoro-1-(6-(p-tolyl)oxazolo[4,5-b]pyridin-2-yl)hexan-1-one:

The title compound was synthesized from 6,6,6-trifluoro-1-(6-(*p*-tolyl)oxazolo[4,5-*b*]pyridin-2-yl)hexan-1-ol according to the procedures described for compound **1.** This yielded 6,6,6-trifluoro-1-(6-(*p*-tolyl)oxazolo[4,5-*b*]pyridin-2-yl)hexan-1-one. HRMS (ESI+): *m*/*z =* 362.1242 [M + H⁺]

### Example 28: Synthesis of Compound 44

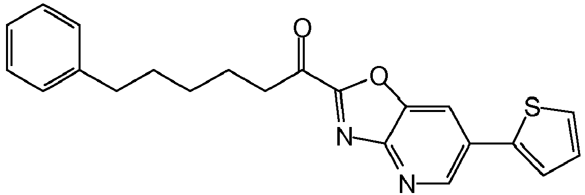

### 6-phenyl-1-(6-(thiophen-2-yl)oxazolo[4,5-b]pyridin-2-yl)hexan-1-one:

The title compound was synthesized from 6-phenyl-1-(6-(thiophen-2-yl)oxazolo[4,5-*b*]pyridin-2-yl)hexan-1-ol according to the procedures described for compound **1.** This yielded 6-phenyl-1-(6-(thiophen-2-yl)oxazolo[4,5-*b*]pyridin-2-yl)hexan-1-one. HRMS (ESI+): *m*/*z =* 376.1246 [M + H⁺]

### Example 29: Synthesis of Compound 45

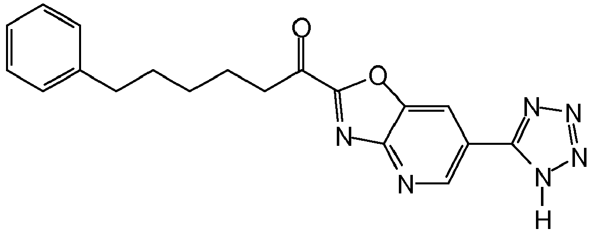

### 1-(6-(1H-tetrazol-5-yl)oxazolo[4,5-b]pyridin-2-yl)-6-phenylhexan-1-one:

The title compound was synthesized from 1-(6-(1*H*-tetrazol-5-yl)oxazolo[4,5-*b*]pyridin-2-yl)-6-phenylhexan-1-ol according to the procedures described for compound 1. This yielded 1-(6-(1*H*-tetrazol-5-yl)oxazolo[4,5-*b*]pyridin-2-yl)-6-phenylhexan-1-one. HRMS (ESI+): *m*/*z =* 362.1491 [M + H⁺]

### Example 30: Synthesis of Compound 46

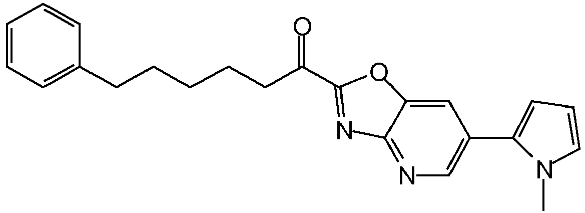

### 1-(6-(1-methyl-1H-pyrrol-2-yl)oxazolo[4,5-b]pyridin-2-yl)-6-phenylhexan-1-one:

The title compound was synthesized from 1-(6-(1-methyl-1*H*-pyrrol-2-yl)oxazolo[4,5-*b*]pyridin-2-yl)-6-phenylhexan-1-ol according to the procedures described for compound 1. This yielded 1-(6-(1-methyl-1H-pyrrol-2-yl)oxazolo[4,5-*b*]pyridin-2-yl)-6-phenylhexan-1-one. HRMS (ESI+): m/z= 373.1790 [M + H⁺]

### Example 31: Synthesis of Compound 47

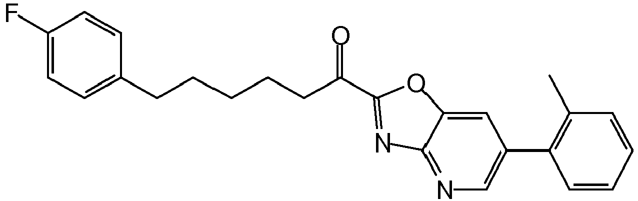

### 6-(4-fluorophenyl)-1-(6-(o-tolyl)oxazolo[4,5-b]pyridin-2-yl)hexan-1-one:

The title compound was synthesized from 6-(4-fluorophenyl)-1-(6-(*o*-tolyl)oxazolo[4,5-*b*]pyridin-2-yl)hexan-1-ol according to the procedures described for compound **1.** This yielded 6-(4-fluorophenyl)-1-(6-(*o*-tolyl)oxazolo[4,5-*b*]pyridin-2-yl)hexan-1-one. HRMS (ESI+): *m*/*z =* 402.1744 [M + H⁺]

### Example 32: Synthesis of Compound 48

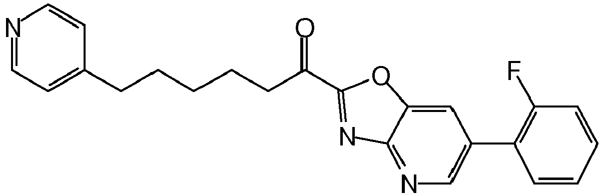

### 1-(6-(2-fluorophenyl)oxazolo[4,5-b]pyridin-2-yl)-6-(pyridin-4-yl)hexan-1-one

The title compound was synthesized from 1-(6-(2-fluorophenyl)oxazolo[4,5-*b*]pyridin-2-yl)-6-(pyridin-4-yl)hexan-1-ol according to the procedures described for compound **1.** This yielded 1-(6-(2-fluorophenyl)oxazolo[4,5-*b*]pyridin-2-yl)-6-(pyridin-4-yl)hexan-1-one. HRMS (ESI+): *m*/*z =* 389.1540 [M + H⁺]

### Example 33: Synthesis of Compound 49

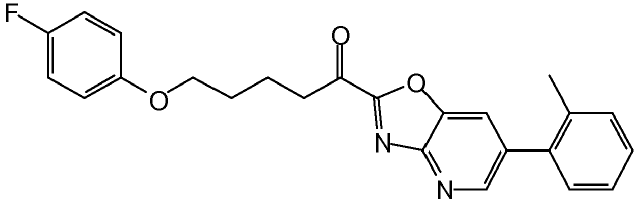

### 5-(4-fluorophenoxy)-1-(6-(o-tolyl)oxazolo[4,5-b]pyridin-2-yl)pentan-1-one:

The title compound was synthesized from 5-(4-fluorophenoxy)-1-(6-(*o*-tolyl)oxazolo[4,5-*b*]pyridin-2-yl)pentan-1-ol according to the procedures described for compound **1.** This yielded 5-(4-fluorophenoxy)-1-(6-(*o*-tolyl)oxazolo[4,5-*b*]pyridin-2-yl)pentan-1-one. HRMS (ESI+): *m*/*z* = 404.1536 [M + H⁺]

### Example 34: Synthesis of Compound 50

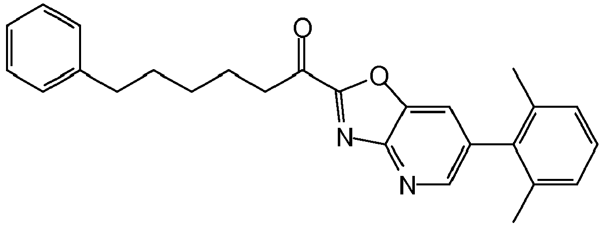

### 1-(6-(2,6-dimethylphenyl)oxazolo[4,5-b]pyridin-2-yl)-6-phenylhexan-1-one:

The title compound was synthesized from 1-(6-(2,6-dimethylphenyl)oxazolo[4,5-*b*]pyridin-2-yl)-6-phenylhexan-1-ol according to the procedures described for compound **1.** This yielded 1-(6-(2,6-dimethylphenyl)oxazolo[4,5-b]pyridin-2-yl)-6-phenylhexan-1-one. HRMS (ESI+): *m*/*z =* 398.1994 [M + H⁺]

### Example 35: Synthesis of Compound 51

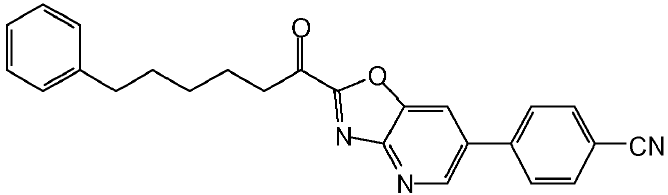

### 4-(2-(6-phenylhexanoyl)oxazolo[4,5-b]pyridin-6-yl)benzonitrile:

The title compound was synthesized from 4-[2-(1-hydroxy-6-phenylhexyl)[1,3]oxazolo[4,5-*b*]pyridin-6-yl]benzonitrile according to the procedures described for compound 1. This yielded 4-(2-(6-phenylhexanoyl)oxazolo[4,5-*b*]pyridin-6-yl)benzonitrile. HRMS (ESI+): m/z = 395.1634 [M + H⁺]

### Example 36: Synthesis of Compound 52

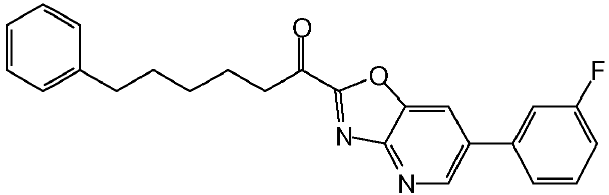

### 1-(6-(3-fluorophenyl)oxazolo[4,5-b]pyridin-2-yl)-6-phenylhexan-1-one:

The title compound was synthesized from 1-(6-(3-fluorophenyl)oxazolo[4,5-*b*]pyridin-2-yl)-6-phenylhexan-1-ol according to the procedures described for compound **1.** This yielded 1-(6-(3-fluorophenyl)oxazolo[4,5-*b*]pyridin-2-yl)-6-phenylhexan-1-one. HRMS (ESI+): *m*/*z =* 388.1587 [M + H⁺]

### Example 37: Synthesis of Compound 53

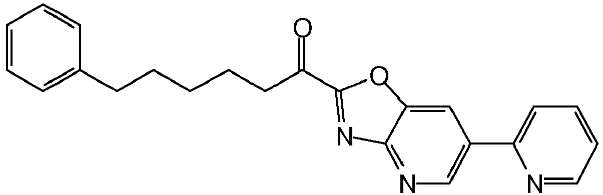

### 6-phenyl-1-(6-(pyridin-2-yl)oxazolo[4,5-b]pyridin-2-yl)hexan-1-one:

The title compound was synthesized from 6-phenyl-1-(6-(pyridin-2-yl)oxazolo[4,5-*b*]pyridin-2-yl)hexan-1-ol according to the procedures described for compound **1.** This yielded 6-phenyl-1-(6-(pyridin-2-yl)oxazolo[4,5-*b*]pyridin-2-yl)hexan-1-one. HRMS (ESI+): *m*/*z* = 371.1634 [M + H⁺]

### Example 38: Synthesis of Compound 54

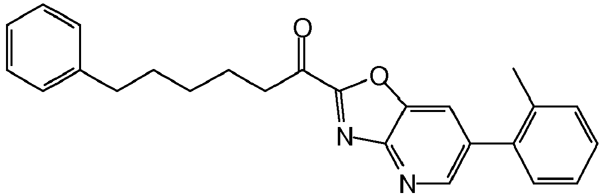

### 6-phenyl-1-(6-(o-tolyl)oxazolo[4,5-b]pyridin-2-yl)hexan-1-one:

The title compound was synthesized from 6-phenyl-1-(6-(*o*-tolyl)oxazolo[4,5-*b*]pyridin-2-yl)hexan-1-ol according to the procedures described for compound **1.** This yielded 6-phenyl-1-(6-(*o*-tolyl)oxazolo[4,5-*b*]pyridin-2-yl)hexan-1-one. HRMS (ESI+): *m*/*z =* 384.1838 [M + H⁺]

### Example 39: Synthesis of Compound 55

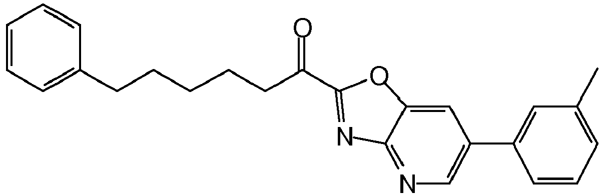

### 6-phenyl-1-(6-(m-tolyl)oxazolo[4,5-b]pyridin-2-yl)hexan-1-one:

The title compound was synthesized from 6-phenyl-1-(6-(*m*-tolyl)oxazolo[4,5-*b*]pyridin-2-yl)hexan-1-ol according to the procedures described for compound **1.** This yielded 6-phenyl-1-(6-(*m*-tolyl)oxazolo[4,5-*b*]pyridin-2-yl)hexan-1-one. HRMS (ESI+): *m*/*z =* 384.1838 [M + H⁺]

### Example 40: Synthesis of Compound 56

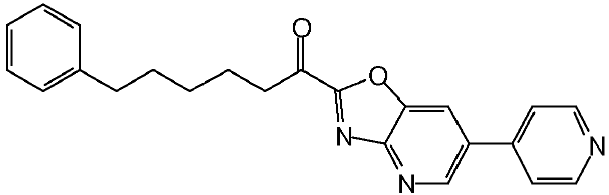

### 6-phenyl-1-(6-(pyridin-4-yl)oxazolo[4,5-b]pyridin-2-yl)hexan-1-one:

The title compound was synthesized from 6-phenyl-1-(6-(pyridin-4-yl)oxazolo[4,5-*b*]pyridin-2-yl)hexan-1-ol according to the procedures described for compound **1.** This yielded 6-phenyl-1-(6-(pyridin-4-yl)oxazolo[4,5-*b*]pyridin-2-yl)hexan-1-one. HRMS (ESI+): *m*/*z* = 371.1634 [M + H⁺]

### Example 41: Synthesis of Compound 57

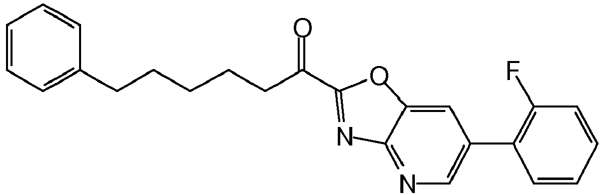

### 1-(6-(2-fluorophenyl)oxazolo[4,5-b]pyridin-2-yl)-6-phenylhexan-1-one:

The title compound was synthesized from 1-(6-(2-fluorophenyl)oxazolo[4,5-*b*]pyridin-2-yl)-6-phenylhexan-1-ol according to the procedures described for compound **1.** This yielded 1-(6-(2-fluorophenyl)oxazolo[4,5-*b*]pyridin-2-yl)-6-phenylhexan-1-one. HRMS (ESI+): *m*/*z =* 388.1587 [M + H⁺]

### Example 42: Synthesis of Compound 58

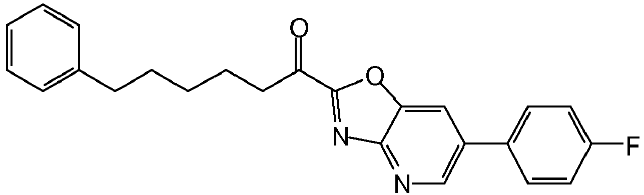

### 1-(6-(4-fluorophenyl)oxazolo[4,5-b]pyridin-2-yl)-6-phenylhexan-1-one:

The title compound was synthesized from 1-(6-(4-fluorophenyl)oxazolo[4,5-*b*]pyridin-2-yl)-6-phenylhexan-1-ol according to the procedures described for compound **1.** This yielded 1-(6-(4-fluorophenyl)oxazolo[4,5-*b*]pyridin-2-yl)-6-phenylhexan-1-one. HRMS (ESI+): *m*/*z =* 388.1587 [M + H⁺]

## Claims

1. Compound having the general formula (I) 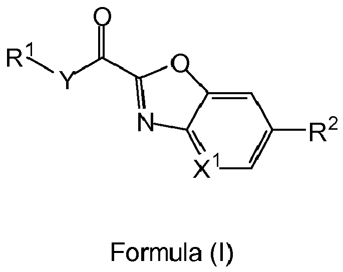 wherein
X¹ is -CH-, -CF- or -N-,
Y represents one of the following moieties:
- CH₂-, -C₂H₄-, -C₃H₆-, -C₄H₈-, -C₅H₁₀-, -C₆H₁₂-, -C₇H₁₄-, -C₈H₁₆-, -C₉H₁₈-, -C₁₀H₂₀-, -CH(CH₃)-, -CH(CH₃)CH-, -CH₂CH(CH₃)-, -CH(CH₃)-C₂H₄-, -CH₂-CH(CH₃)CH₂-, -CH₂-CH₂CH(CH₃)-, -CH(CH₃)-C₃H₆-, -CH₂-CH(CH₃)-C₂H₄-, -CH₂-CH₂-CH(CH₃)CH₂-, -CH₂-CH₂-CH₂CH(CH₃)-, -CH(CH₃)-C₄H₈-, -CH₂-CH(CH₃)-C₃H₆-, -CH₂-CH₂-CH(CH₃)-C₂H₄-, -CH₂-CH₂-CH₂-CH(CH₃)-CH₃-, -CH₂-CH₂-CH₂-CH₂-CH(CH₃)-, -CH₂O-, -C₂H₄O-, -C₃H₆O-, -C₄H₈O-, -C₅H₁₀O-, -C₆H₁₂O-, -C₇H₁₄O-, -C₈H₁₆O-, -C₉H₁₈O- -C₁₀H₂₀O-, -CH₂NH-, -C₂H₄NH-, -C₃H₆NH-, -C₄H₈NH-, -C₅H₁₀NH- -C₆H₁₂NH-, -C₇H₁₄NH-, -C₈H₁₆NH-, -C₉H₁₈NH-, -C₁₀H₂₀NH-, -CH₂OCH₂-, -C₂H₄OCH₂-, -C₃H₆OCH₂-, -C₄H₈OCH₂- -C₅H₁₀OCH₂-, -C₆H₁₂OCH₂-, -C₇H₁₄OCH₂-, -C₈H₁₆OCH₂-, -C₉H₁₈OCH₂-, -C₁₀H₂₀OCH₂-, -CH₂NHCH₂-, -C₂H₄NHCH₂-, -C₃H₆NHCH₂-, -C₄H₈NHCH₂- -C₅H₁₀NHCH₂-, -C₆H₁₂NHCH₂-, -C₇H₁₄NHCH₂-, -C₈H₁₆NHCH₂-, -C₉H₁₈NHCH₂-, -C₁₀H₂₀NHCH₂-, -CH=CH-, -CH=CH-CH₂-, -CH₂-CH=CH-, -CH=CH-C₂H₄-, -CH₂-CH=CH-CH₂-, -C₂H₄-CH=CH-, -CH=CH-C₃H₆-, -CH₂-CH=CH-C₂H₄-, -C₂H₄-CH=CH-CH₂-, -C₂H₄-CH₂-CH=CH-, -CH=CH-C₄H₈-, -CH₂-CH=CH-C₃H₆-, -C₂H₄-CH=CH-C₂H₄-, -C₃H₆-CH=CH-CH₂-, -C₄H₈-CH=CH- -CH=CH-CH=CH-, -CH=CH-CH=CH-CH₂-, -CH₂-CH=CH-CH=CH-, -CH=CH-CH₂-CH=CH-, -C≡C-, -C≡C-CH₂-, -CH₂-C≡C-, -C≡C-C₂H₄-, -CH₂-C≡C-CH₂-, -C₂H₄-C≡C-, -C₂H₄-C≡C-, -C≡C-C₃H₆-, -CH₂-C≡C-C₂H₄-, -C₂H₄-C≡C-CH₂-, -C₃H₆-C≡C-, -C≡C-C₄H₈-, -CH₂-C≡C-C₃H₆-, -C₂H₄-C≡C-C₂H₄-, -C₃H₆-C≡C-CH₂-, -C≡C-C≡C-, -C≡C-C≡C-CH₂-, -C≡C-CH₂-C≡C-, -CH₂-C≡C-C≡C-, -C₂H₄-C≡C-C≡C-, -CH₂-C≡C-CH₂-C≡C-, -C≡C-C₂H₄-C≡C-, -CH₂-C≡C-C≡C-CH₂-, -C≡C-CH₂-C≡C-CH₂-, or -C≡C-C≡C-C₂H₄-;
R¹ represents: -CF₃, -CHF₂, -CH₂F, -CF₂CF₃, -CHFCF₃, -CF₂CHF₂, -CHFCHF₂, -CF₂CH₂F, -CHFCH₂F, -CHFCH₃, -CF₂CH₃, 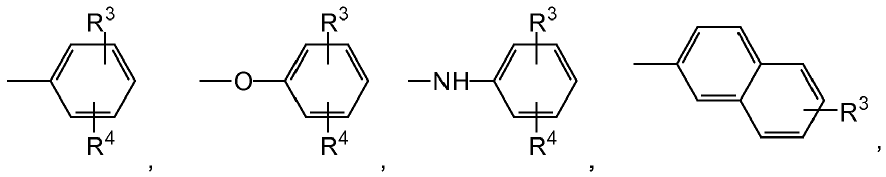 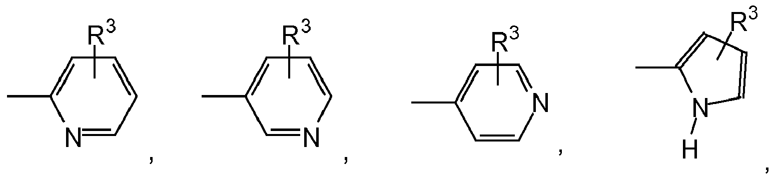 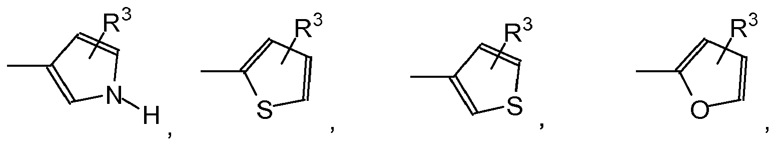 or 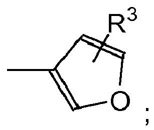
R² represents one of the following moieties: -CH(CH₃)₂, -CH(C₂H₅)₂, -CH₂CH(CH₃)₂, -C(CH₃)₃, -CH₂C(CH₃)₃, -CHPh₂, -CPh₃, -CH₂CPh₃, 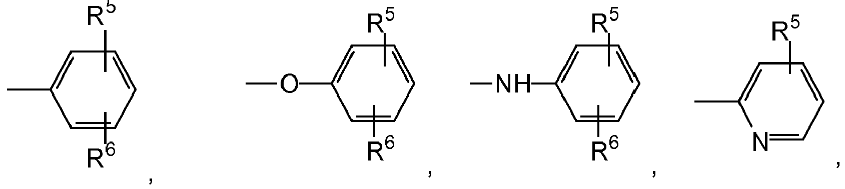 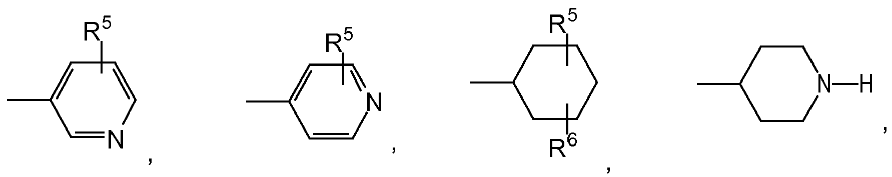 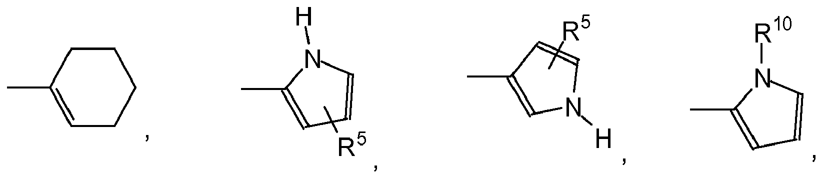 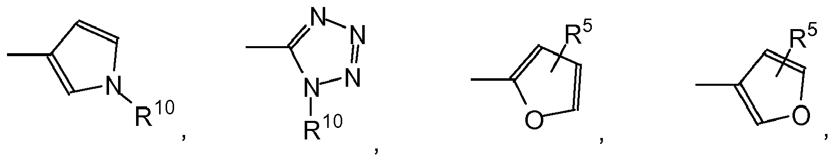 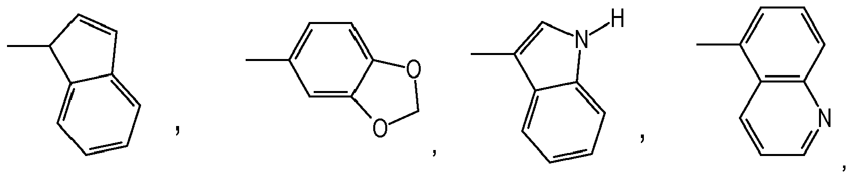 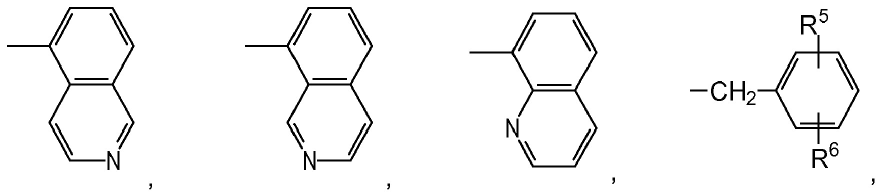 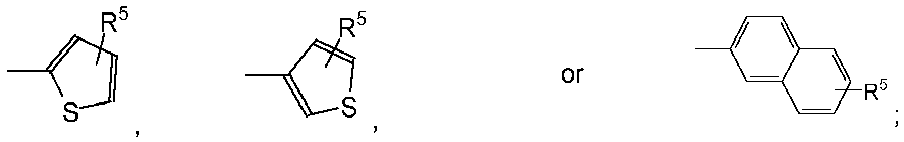
R³ and R⁴ are independently of each other selected from: -R⁸, -R⁹, -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SO₂CH₃, -SO₂C₂H₅, -SO₂CH(CH₃)₂, -SO₂C(CH₃)₃, -SO₃H, -SO₃CH₃, -SO₃C₂H₅, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -NH-CO-NH₂, -NH-CO-NHCH₃, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, 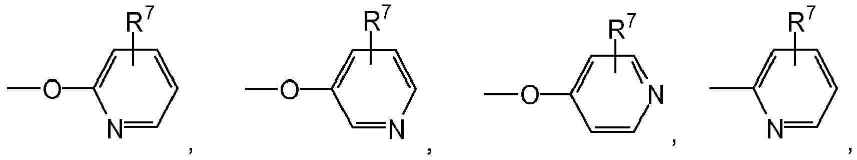 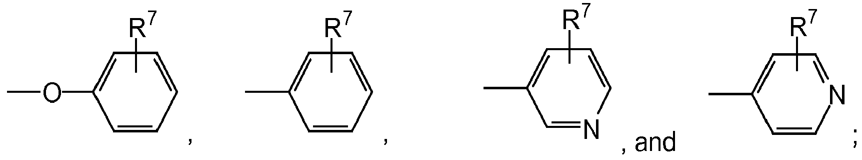
R⁵ and R⁶ are independently of each other selected from:
-H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -N₃, -CN, -COC₂H₅, -COC₃H₇, -COOH, -COOCH₃, -COOC₂H₅, -COOC₃H₇, -CONH₂, -CONHCH₃, -NH₂, -NHCH₃, -NHC₂H₅, -NHC₃H₇, -N(C₂H₅)₂, -SO₂CH₃, -SO₂C₂H₅, -SO₂C₃H₇, -SO₃H, -SO₃CH₃, -CH₂Cl, -CH₂Br, -CH₂I, -CH₂F, -CHF₂, -CF₃, -CF₂CF₃, -CH₂CH₂F, -CH₂CHF₂, -CH₂CF₃, -CF₂CF₂CF₃, -CH₂CH₂CF₃, -CF=CF₂, -CH=CF₂, -CF=CFCF₃, -CH=CHCF₃, -CF₂CF=CF₂, -CH₂CH=CF₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -CH=CH₂, -CH₂-C(CH₃)₂-CH=CH₂, -C(CH₃)₂-CH₂-CH=CH₂, -CH=C(CH₃)-CH(CH₃)₂, -Ph, -CHPh₂, -CPh₃, -CH=CH-Ph, -CH₂-Ph, -C₂H₄-Ph, -OCH₂-Ph, and -OC₂H₄-Ph;
R⁷, R⁸ and R⁹ are independently of each other selected from:
- H, -F, -Cl, -Br, -I, -CH₂F, -CHF₂, -CF₃, -CF₂CF₃, -CH₂CH₂F, -CH₂CHF₂, -CH₂CF₃, -CF₂CF₂CF₃, -CH₂CH₂CF₃, -CF=CF₂, -CH=CF₂, -CF=CFCF₃, -CH=CHCF₃, -CF₂CF=CF₂, -CH₂CH=CF₂, -OH, -OCH₃, -OC₂H₅, -OC₃H₇, -OC₃H₇, -OC₄H₉, -NH₂, -NHCH₃, -NHC₂H₅, -NHC₃H₇, -NH(CH(CH₃)₂, -NHC₄H₉, -CH₂F, -CHF₂, -CF₃, -CH₂Cl, -CH₂Br, -CH₂I, -CH₂-CH₂Cl, -CH₂-CH₂Br, -CH₂-CH₂I, -CH₃, -C₂H₅, -C₃H₇, -C₄H₉, -C₅H₁₁, -C₆H₁₃, -C₇H₁₅, -C₈H₁₇, -C₉H₁₉, -C₁₀H₂₁, -CH(CH₃)₂, -CH(CH₃)-C₂H₅, -CH₂-CH(CH₃)CH₃, -CH(CH₃)-C₃H₇, -CH₂-CH(CH₃)-C₂H₅, -CH₂-CH₂-CH(CH₃)₂, -CH(CH₃)-C₄H₉, -CH₂-CH(CH₃)-C₃H₇, -CH₂-CH₂-CH(CH₃)-C₂H₅, -CH₂-CH₂-CH₂-CH(CH₃)-CH₃, -CH₂-CH₂-CH₂-CH₂-CH(CH₃)₂, -CH=CH₂, -CH=CH-CH₃, -CH₂-CH=CH₂, -CH=CH-C₂H₅, -CH₂-CH=CH-CH₃, -C₂H₄-CH=CH₂, -CH=CH-C₃H₇, -CH₂-CH=CH-C₂H₅, -C₂H₄-CH=CH-CH₃, C₂H₄-CH₂-CH=CH₂, -CH=CH-C₄H₉, -CH₂-CH=CH-C₃H₇, -C₂H₄-CH=CH-C₂H₅, -C₃H₆-CH=CH-CH₃, -C₄H₈-CH=CH₂, -CH=CH-CH=CH₂, -CH=CH-CH=CH-CH₃, -CH₂-CH=CH-CH=CH₂, -CH=CH-CH₂-CH=CH₂, -C≡C-CH₃, -CH₂-C≡CH, -C≡C-C₂H₅, -CH₂-C≡C-CH₃, -C₂H₄-C≡CH, -C₂H₄-C≡CH, -C≡C-C₃H₇, -CH₂-C≡C-C₂H₅, -C₂H₄-C≡C-CH₃, -C₃H₆-C≡CH, -C≡C-C₄H₉, -CH₂-C≡C-C₃H₇, -C₂H₄-C≡C-C₂H₅, -C₃H₆-C≡C-CH₃, -C=C-C=CH, -C≡C-C≡C-CH₃, -C≡C-CH₂-C≡CH, -CH₂-C≡C-C≡CH, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -Ph, -CH₂-Ph, -CH₂-CH₂-Ph, -CH=CH-Ph, -CPh₃, -OCH₂-Ph, and -OC₂H₄-Ph;
R¹⁰ represents one of the following groups: -H, -CH₃, -CH₂CH₃, -CH(CH₃)₂, -CH₂CH₂CH₃, or -CH₂CH₂CH₂CH₃;
and enantiomers, stereoisomeric forms, mixtures of enantiomers, diastereomers, mixtures of diastereomers, prodrugs, hydrates, solvates, acid salt forms, tautomers, and racemates of the above mentioned compounds and pharmaceutically acceptable salts thereof.

2. A compound according to claim 1, wherein X¹ represents -N- or -CF-.

3. A compound according to any one of claims 1 or 2, wherein the linker Y represents -(CH₂)ₙ-,
and wherein n is an integer number selected from: 0, 1, 2, 3, 4, 5, 6, 7, 8, 9, and 10.

4. The compound according to any one of claims 1 to 3, wherein the compound is selected from the group of compounds consisting of:
1-(6-(*p*-tolyl)oxazolo[4,5-*b*]pyridin-2-yl)-6-phenylhexan-1-one
3-(2-phenoxyphenyl)-1-(6-(*p*-tolyl)oxazolo[4,5-*b*]pyridin-2-yl)propan-1-one,
3-(3-Phenoxyphenyl)-1-(6-(*p*-tolyl)oxazolo[4,5-*b*]pyridin-2-yl)propan-1-one,
3-(4-phenoxyphenyl)-1-(6-(*p*-tolyl)oxazolo[4,5-*b*]pyridin-2-yl)propan-1-one,
1-(6-(p-Tolyl)oxazolo[4,5-b]pyridin-2-yl)-3-(4-((5-(trifluoromethyl)pyridin-2-yl)oxy)phenyl)propan-1-one,
1-(4-fluoro-6-(4-fluorophenyl)benzo[d]oxazol-2-yl)-6-phenylhexan-1-one,
1-(4-fluoro-6-(2-fluorophenyl)benzo[d]oxazol-2-yl)-6-phenylhexan-1-one,
1-(4-fluoro-6-(*p*-tolyl)benzo[*d*]oxazol-2-yl)-6-phenylhexan-1-one, and
1-(4-fluoro-6-(o-tolyl)benzo[d]oxazol-2-yl)-6-phenylhexan-1-one,
2-phenyl-1-(6-(*p*-tolyl)oxazolo[4,5-*b*]pyridin-2-yl)ethan-1-one,
3-phenyl-1-(6-(*p*-tolyl)oxazolo[4,5-*b*]pyridin-2-yl)propan-1-one,
4-phenyl-1-(6-(*p*-tolyl)oxazolo[4,5-*b*]pyridin-2-yl)butan-1-one,
6-phenyl-1-(6-(pyridin-4-yl)oxazolo[4,5-*b*]pyridin-2-yl)hexan-1-one,
1-(6-(2-chlorophenyl)oxazolo[4,5-*b*]pyridin-2-yl)-6-phenylhexan-1-one,
1-(6-(3-chlorophenyl)oxazolo[4,5-*b*]pyridin-2-yl)-6-phenylhexan-1-one,
1-(6-(4-chlorophenyl)oxazolo[4,5-*b*]pyridin-2-yl)-6-phenylhexan-1-one,
1-(6-(4-methoxyphenyl)oxazolo[4,5-*b*]pyridin-2-yl)-6-phenylhexan-1-one,
4,4,4-trifluoro-1-(6-(*p*-tolyl)oxazolo[4,5-*b*]pyridin-2-yl)butan-1-one,
6,6,6-trifluoro-1-(6-(*p*-tolyl)oxazolo[4,5-*b*]pyridin-2-yl)hexan-1-one,
6-phenyl-1-(6-(thiophen-2-yl)oxazolo[4,5-*b*]pyridin-2-yl)hexan-1-one,
1-(6-(1*H*-tetrazol-5-yl)oxazolo[4,5-*b*]pyridin-2-yl)-6-phenylhexan-1-one,
1-(6-(1-methyl-1*H*-pyrrol-2-yl)oxazolo[4,5-*b*]pyridin-2-yl)-6-phenylhexan-1-one,
6-(4-fluorophenyl)-1-(6-(*o*-tolyl)oxazolo[4,5-*b*]pyridin-2-yl)hexan-1-one,
1-(6-(2-fluorophenyl)oxazolo[4,5-*b*]pyridin-2-yl)-6-(pyridin-4-yl)hexan-1-one,
5-(4-fluorophenoxy)-1-(6-(*o*-tolyl)oxazolo[4,5-*b*]pyridin-2-yl)pentan-1-one,
1-(6-(2,6-dimethylphenyl)oxazolo[4,5-*b*]pyridin-2-yl)-6-phenylhexan-1-one,
4-(2-(6-phenylhexanoyl)oxazolo[4,5-*b*]pyridin-6-yl)benzonitrile,
1-(6-(3-fluorophenyl)oxazolo[4,5-*b*]pyridin-2-yl)-6-phenylhexan-1-one,
6-phenyl-1-(6-(pyridin-2-yl)oxazolo[4,5-*b*]pyridin-2-yl)hexan-1-one,
6-phenyl-1-(6-(*o*-tolyl)oxazolo[4,5-*b*]pyridin-2-yl)hexan-1-one,
6-phenyl-1-(6-(*m*-tolyl)oxazolo[4,5-*b*]pyridin-2-yl)hexan-1-one,
6-phenyl-1-(6-(pyridin-4-yl)oxazolo[4,5-*b*]pyridin-2-yl)hexan-1-one,
1-(6-(2-fluorophenyl)oxazolo[4,5-*b*]pyridin-2-yl)-6-phenylhexan-1-one, and
1-(6-(4-fluorophenyl)oxazolo[4,5-*b*]pyridin-2-yl)-6-phenylhexan-1-one.

5. A compound according to any one of claims 1 to 4 as selective inhibitors of diacylglycerol lipase.

6. A compound according to any one of claims 1 to 5 for use as pharmaceutically active agent.

7. A compound of claim 6 for use as suitable pharmaceutically active agent for the treatment of disorders associated with, accompanied by and/or caused by pathologically 2-arachidonoylglycerol levels.

8. A compound of claim 6 or 7 for use in the treatment and/or prevention of neurodegenerative diseases, inflammatory disease and impaired energy balance.

9. A compound of claim 8, wherein the neurodegenerative diseases are selected from the following group consisting of: amyotrophic lateral sclerosis, Parkinson's disease, Huntington's disease, spinal muscular-atrophy, progressive supranuclear palsy, Wilson's disease, multi-system atrophy, Shy-Drager syndrome, Alzheimer's disease, spinocerebellar ataxia, glaucoma, Pick's disease, Lewy-body disease, Hallervorden-Spatz disease, Creutzfeld-Jakob-disease, Machado-Joseph disease, Friedreich ataxia, non-Friedreich ataxias, Gilles de la Tourette syndrome, familial tremors, olivopontocerebellar degenerations, paraneoplasticcerebral syndromes, hereditary spastic paraplegias, hereditary optic neuropathy, degenerative retinal diseases, retinitis pigmentosa, dry and humid macular degeneration, Stargardt disease, and Keams- Sayre syndrome.

10. A compound of claim 8, wherein the inflammatory disease is mediated by arachidonic acid metabolites

11. A compound of claim 6 or 7, for use in the treatment and/or prevention of neurodegenerative diseases, inflammatory disease and impaired energy balance, wherein the compound prevents the formation of prostaglandin ester formation.

12. A compound of claim 9, wherein the impaired energy balance is obesity.

13. A compound of claim 8 for use in the treatment of drug abuse.

14. A compound of claim 13, wherein the drug is selected from the group consisting of Δ⁹-tetrahydrocannabinol, ethanol, nicotine, cocaine and opiates.

15. Pharmaceutical composition comprising at least one compound according to any one of claims 1 to 5 as an active ingredient, together with at least one pharmaceutically acceptable carrier, excipient and/or diluent.
